# FASCICULE DE BREVET EUROPEEN

(11) **EP 1 200 419 B1**
(45) Date de publication et mention de la délivrance du brevet: **14.04.2004**
(21) Numéro de dépôt: 00951647.7
(22) Date de dépôt: 11.07.2000
(51) Int. Cl.: C07D 277/42, C07D 417/12, A61K 31/426, A61K 31/427, A61P 25/00

(54) **DERIVES D'AMINOTHIAZOLE ET LEUR UTILISATION COMME LIGANDS DES RECEPTEURS CRF**
AMINOTHIAZOLDERIVATE UND IHRE VERWENDUNG ALS CRF-REZEPTOR-LIGANDEN
AMINOTHIAZOLE DERIVATIVES AND THEIR USE AS CRF RECEPTOR LIGANDS

(30) Priorité: 15.07.1999 FR 9909144
(43) Date de publication de la demande: 02.05.2002
(73) Titulaire: SANOFI-SYNTHELABO, 75013 Paris (FR)
(72) Inventeur: FONTAINE, Evelyne, F-31320 Castanet-Tolosan (FR); GESLIN, Michel, F-31270 Villeneuve Tolosane (FR); GULLY, Danielle, F-31600 Muret (FR); PRADINES, Antoine, F-31120 Roquettes (FR); ROGER, Pierre, F-78423 Montigny-le-Bretonneux (FR)
(74) Mandataire: Thouret-Lemaitre, Elisabeth
(86) Numéro de dépôt international: PCT/FR2000/001995
(87) Numéro de publication internationale: WO 2001/005776

(56) Documents cités:
- EP-A- 0 576 350
- EP-A- 0 659 747
- WO-A-97/00868
- WO-A-98/15543

## Description

La présente invention a pour objet de nouveaux dérivés amino ramifiés du thiazole, un procédé pour leur préparation et les compositions pharmaceutiques les contenant. Ces nouveaux dérivés du thiazole sont pourvus d'activité antagoniste du CRF (corticotropin releasing factor) et peuvent donc constituer des principes actifs de compositions pharmaceutiques.

Le facteur de libération de l'hormone corticotrope (CRF) est un peptide dont la séquence de 41 acides aminés, a été caractérisée par Vale W. *et al*. en 1981 (Science, 1981, *213*, 1394-1397). Le CRF est le principal facteur endogène impliqué dans la régulation de l'axe hypothalamo-hypophyso-surrénalien (libération de l'hormone adrénocorticotrope : ACTH) et ses pathologies, ainsi que dans les syndromes dépressifs qui en découlent. Le CRF provoque également la sécrétion de β-endorphine, de β-lipotropine et de corticostérone. Le CRF est donc le régulateur physiologique de la sécrétion de l'hormone adrénocorticotrope (ACTH) et plus généralement des peptides dérivés de la propiomélanocortine (POMC). Outre sa localisation hypothalamique, le CRF est largement distribué dans le système nerveux central, mais également dans des tissus extra-neuronaux tels que les glandes surrénales et les testicules. La présence de CRF a été également mise en évidence au cours de processus inflammatoires.

De nombreuses expérimentations animales ont montré que l'administration centrale de CRF provoque des effets anxiogènes variés tels que la modification du comportement en général : par exemple néophobie, réduction de la réceptivité sexuelle, diminution de la consommation alimentaire et du sommeil lent chez le rat. L'injection intracérébroventriculaire de CRF augmente également l'excitation des neurones noradrénergiques du locus coeruleus qui est souvent associée chez l'animal, à un état d'anxiété. Chez le rat, l'administration centrale ou périphérique du CRF ou des peptides apparentés (par exemple urocortine, sauvagine) induit outre des effets centraux tels que l'augmentation de l'éveil et de la réactivité émotionnelle face à l'environnement, des modifications de la vidange gastrique, de la sécrétion acide, du transit intestinal et de l'excrétion fécale ainsi que des effets tensionnels. Le CRF est impliqué également dans la régulation complexe des réponses inflammatoires, d'une part avec un rôle pro-inflammatoire dans certains modèles animaux, d'autre part en tant qu'inhibiteur des effets induits par l'augmentation de la perméabilité vasculaire consécutive de l'inflammation.

L'utilisation d'un antagoniste peptidique, l'alpha-hélical CRF(9-41) (α-CRF) ou d'anticorps spécifiques (Rivier J. *et al*., Science, 1984, *224*, 889-891) a permis de confirmer le rôle de ce peptide dans l'ensemble de ces effets. Ces expériences ont également confirmé le rôle important du CRF chez l'homme dans l'intégration des réponses complexes observées lors d'un stress physiologique, psychologique ou immunologique tant sur le plan neuroendocrinien, viscéral que comportemental (Morley J.E. *et al*, Endocrine Review, 1987, *8*, 3, 256-287 ; Smith M.A. *et al*., Horm. Res., 1989, *31*, 66-71). En outre, des données cliniques militent en faveur de l'implication effective du CRF dans les nombreux désordres découlant d'un état de stress (Gulley L.R. *et al*., J. Clin. Psychiatry, 1993, *54*, 1, (suppl.), 16-19) par exemple :
- l'existence du test au CRF (administration i.v.) chez l'homme a permis de démontrer la modification de la réponse en ACTH chez les patients dépressifs (Breier A. *et al*., Am. J. Psychiatry, 1987, *144*, 1419-1425).
- la découverte d'une hypersécrétion de CRF endogène dans certaines pathologies, par exemple d'un taux de CRF élevé dans le liquide céphalorachidien chez les patients non médiqués, déprimés ou atteints de démence type maladie d'Alzheimer (Nemeroff C.B. *et al*., Science, 1984, *226*, 4680, 1342-1343 ; Regul. Pept., 1989, *25*, 123-130), ou d'une densité de récepteurs au CRF diminuée dans le cortex de victimes de suicide (Nemeroff C.B. *et al.,* Arch. Gen. Psychiatry, 1988, *45*, 577-579).
- le dysfonctionnement des neurones CRF-dépendants est même suggéré dans les pathologies sévères que sont les maladies d'Alzheimer, de Parkinson, la chorée de Huntington et la sclérose latérale amyotrophique (De Souza E.B.,Hospital Practice, 1988, *23*, 59).

L'administration centrale de CRF dans de nombreuses espèces animales, produit des effets comportementaux semblables à ceux obtenus chez l'homme dans les situations de stress. Lorsqu'ils sont répétés dans le temps, ces effets peuvent entraîner des pathologies diverses telles que : fatigue, hypertension, troubles cardiaques et tensionnels, modification de la vidange gastrique, de l'excrétion fécale (colite, colon irritable), modification de la sécrétion acide, hyperglycémie, croissance retardée, anorexie, néophobie, migraines, troubles de la reproduction, immunosuppression (processus inflammatoires, infections multiples et cancers) et désordres neuropsychiatriques variés (dépression, anorexie nerveuse et anxiété).

L'injection par voie intracérébroventriculaire de l'antagoniste peptidique de référence, l'α-CRF prévient les effets obtenus soit par l'administration de CRF exogène, soit par l'utilisation d'agents stressants (éther, contrainte, bruit, choc électrique, sevrage éthanolique, chirurgie) capables par eux-mêmes d'induire une augmentation du taux de CRF endogène. Ces résultats sont confirmés par l'étude de nombreuses molécules peptidiques antagonistes structuralement apparentées au CRF et qui possèdent une durée d'action prolongée par rapport à l'α-CRF (Rivier J. *et al*., J. Med. Chem., 1993, *36*, 2851-2859 ; Menzaghi F. *et al*., J. Pharmacol. Exp. Ther., 1994, *269*, 2, 564-572 ; Hernandez J.F. *et al*., J. Med. Chem., 1993, *36*, 2860-2867).

De tels composés peptidiques antagonistes du CRF sont décrits par exemple dans les brevets US 5,109,111, US 5,132,111, US 5,245,009 et dans les demandes de brevet WO 92/22576 et WO 96/19499.

En outre, des études préliminaires ont montré que des antidépresseurs tricycliques pouvaient moduler le taux de CRF ainsi que le nombre de récepteurs au CRF dans le cerveau (Grigoriadis D.E. *et al.,* Neuropsychopharmacoloy, 1989, *2*, 53-60). De même des anxiolytiques benzodiazépiniques sont capables d'inverser l'effet du CRF (Britton K.T. *et al*., Psychopharmacology, 1988, *94*, 306), sans que le mécanisme d'action de ces substances soit totalement élucidé. Ces résultats confortent si nécessaire le besoin grandissant de molécules antagonistes non-peptidiques des récepteurs du CRF.

Il est important de signaler également, trois conséquences possibles des états de stress chronique que sont l'immunodépression, les troubles de la fertilité, ainsi que l'installation du diabète.

Le CRF exerce de tels effets en interagissant avec des récepteurs membranaires spécifiques qui ont été caractérisés dans l'hypophyse et le cerveau de nombreuses espèces (souris, rat et homme) ainsi que dans le coeur, le muscle squelettique (rat, souris) et dans le myomètre et le placenta au cours de la grossesse.

Un grand nombre de dérivés du 2-aminothiazole est déjà connu. La demande de brevet EP 462 264 décrit des dérivés du 2-aminothiazole, dont l'amine tertiaire en position 2, comporte deux substituants ayant chacun au moins un hétéroatome dont un dérivé d'amine. Ces composés sont des antagonistes du facteur d'activation des plaquettes (PAF-acéther) et trouvent leurs applications dans le traitement de l'asthme, de certains états allergiques ou inflammatoires, de maladies cardiovasculaires, de l'hypertension et de diverses pathologies rénales ou encore comme agents contraceptifs.

La demande GB 2 022 285 décrit des composés possédant une activité régulatrice de la réponse immunitaire et ayant des propriétés anti-inflammatoires. Il s'agit de dérivés du thiazole substitués en position 2 par des groupes amines secondaires.

Certains dérivés du 2-acylaminothiazole ont été décrits dans la demande de brevet EP 432 040. Ces composés sont des antagonistes de la cholécystokinine et de la gastrine.

Des dérivés du 2-amino-4,5-diphénylthiazole ayant des propriétés antiinflammatoires sont aussi connus (demande de brevet JP-01 75 475).

On connaît aussi des dérivés du 2-amino-4-(4-hydroxyphényl)thiazole utiles comme intermédiaires de synthèse pour la préparation des dérivés du 2,2-diarylchroménothiazole (demande de brevet EP 205 069).

Des dérivés du 2-(N-méthyl-N-benzylamino)thiazole sont aussi décrits dans J. Chem. Soc. Perkin, Trans 1, 1984, *2*, 147-153 et dans J. Chem. Soc. Perkin, Trans 1, 1983, *2*, 341-347.

La demande de brevet WO 94/01423 décrit des dérivés du 2-aminothiazole. Ces composés sont utilisés comme insecticides ; ils ne présentent aucune substitution en position 5 de l'hétérocycle.

De même, la demande de brevet WO 96/16650 décrit des composés dérivés de 2-aminothiazole. Ces composés sont utilisés comme antibiotiques.

La demande de brevet EP 283 390, décrit parmi d'autres dérivés du thiazole, des dérivés du 2-(N-alkyl-N-pyridylalkylamino)thiazole dont l'amine en position 2 est substituée par un radical pyridylalkyle non ramifié.

Ces composés possèdent notamment une activité stimulante de la transmission cholinergique centrale. Ils peuvent ainsi être utilisés comme agonistes des récepteurs muscariniques et trouvent leurs applications dans le traitement des troubles de la mémoire et des démences séniles.

Des dérivés du 2-aminothiazole dont l'amine en position 2 est une amine tertiaire ayant un substituant alkyle ou aralkyle ramifié ont été décrits dans EP 576 350, WO 9 815 543 et EP 659 747 comme possédant une affinité pour les récepteurs du CRF.

Le brevet US 5,063,245 décrit des antagonistes du CRF qui permettent de déplacer *in vitro* la liaison du CRF à ses récepteurs spécifiques à une concentration voisine d'une micromole. Depuis de nombreuses demandes de brevets concernant des molécules non-peptidiques ont été publiées, par exemple les demandes WO 94/13643, WO 94/13644, WO 94/13661, WO 94/13676, WO 94/13677, WO 94/10333, WO 95/00640, WO 95/10506, WO 95/13372, WO 95/33727, WO 95/33750, WO 95/34563, EP 691 128 ou EP 729 758.

Il a maintenant été trouvé selon le présente invention que certains dérivés amino ramifiés du thiazole, objet de la présente invention, présentent une excellente affinité *vis à vis* des récepteurs du CRF. De plus, compte tenu de leur structure, ces molécules possèdent une bonne dispersibilité et/ou solubilité dans des solvants ou solutions couramment utilisés en thérapeutique qui leur confère une activité pharmacologique et permettent aussi la préparation aisée de formes galéniques orales et parentérales.

Cela est étonnant et inattendu, puisque les composés de l'invention sont plus actifs *in vivo* que des composés de structure proche en particulier par une inhibition plus significative de la réponse induite par le CRF au niveau de l'axe hypothalamo-hypophyso-surrénalien.

La présente invention a pour objet, les composés, sous forme racémique ou d'énantiomère pur, de formule : 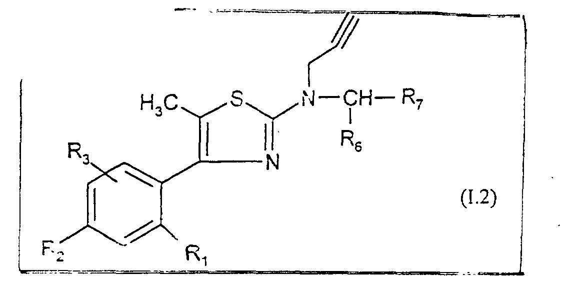 dans laquelle
- R₁ et R₂ identiques ou différents représentent chacun indépendamment un atome d'halogène ; un hydroxy(C₁-C₅)alkyle ; un (C₁-C₅)alkyle ; un aralkyle dans lequel la partie aryle est en (C₆-C₈) et.la partie alkyle en (C₁-C₄) ; un (C₁-C₅)alcoxy ; un groupe trifluorométhyle ; un groupe nitro ; un groupe nitrile ; un groupe -SR dans lequel R représente l'hydrogène, un (C₁-C₅)alkyle ou un aralkyle dans lequel la partie aryle est en (C₆-C₈) et la partie alkyle en (C₁-C₄) ; un groupe -S-CO-R dans lequel R représente un radical (C₁-C₅)alkyle ou un aralkyle dans lequel la partie aryle est en (C₆-C₈) et la partie alkyle est en (C₁-C₄) ; un groupe -COORa dans lequel Ra représente l'hydrogène ou un (C₁-C₅)alkyle ; un groupe -CONRaRb avec Ra et Rb tels que définis ci-dessus pour Ra ; un groupe -NRaRb avec Ra et Rb tels que définis précédemment pour Ra ; un groupe -CONRcRd ou -NRcRd dans lesquels Rc et Rd constituent avec l'atome d'azote auquel ils sont liés un hétérocycle de 5 à 7 chaînons ; ou un groupe -NHCO-NRaRb avec Ra et Rb tels que définis ci-dessus pour Ra ;
- R₃ représente l'hydrogène ou est tel que défini ci-dessus pour R₁ et R₂ ;
- ou bien R₂ constitue avec R₃ lorsque celui-ci substitue le phényle en position 5 un groupe -X-CH₂-X- dans lequel X représente indépendamment un CH₂, un atome d'oxygène ou de soufre ;
- R₆ représente un (C₁-C₆)alkyle ; un (C₁-C₆)alcoxy(C₁-C₃)alkyle ; un (C₃-C₅)cycloalkyle ; un (C₃-C₆)cycloalkyl(C₁-C₆)alkyle ; un (C₁-C₆)alkylthio(C₁-C₃)alkyle ; un (C₁-C₆)alkylsulfoxy(C₁-C₃)alkyle ; un (C₁-C₆)alkylsulfodioxy(C₁-C₃)alkyle ;
- R₇ représente un phényle non substitué, mono, di ou trisubstitué en position 3, 4 ou 5 par un halogène, par un (C₁-C₅)alkyle, par un groupe -O-CH₂-O- sur deux atomes de carbone voisins du phényle, par un -CF₃, -NO₂, -CN, par un groupe -COOR₈, -CONR₈R₉ ou par un groupe -CH₂OR₈ dans lesquels R₈ et R₉ représentent un (C₁-C₃)alkyle, OR₁₀ dans lequel R₁₀ représente un (C₁-C₅)alkyle ; ou bien R₇ représente un groupe pyridyle, thiophène, pyrazolyle, imidazolyle, (C₃-C₅)cycloalkyle
   ou (C₃-C₆)cycloalkyl(C₁-C₆)alkyle ;
leurs sels d'addition, leurs hydrates et/ou leurs solvats.

Dans la présente description les groupes alkyles ou les groupes alcoxy sont linéaires ou ramifiés.

Par atome d'halogène, on entend atome de fluor, de chlore, de brome ou d'iode.

Les hétérocycles définis pour R₇ peuvent éventuellement être substitués par les mêmes substituants que ceux du phényle.

Selon un autre de ses aspects, l'invention concerne les composés, sous forme racémique ou d'énantiomère pur, de formule (I.2) dans laquelle :
- R₁ et R₂ identiques ou différents représentent chacun indépendamment un atome d'halogène; un (C₁-C₅)alkyle ; un (C₁-C₅)alcoxy ;
- R₃ représente l'hydrogène ou est tel que défini ci-dessus pour R₁ et R₂ ;
- R₆ représente un (C₁-C₆)alkyle ; un (C₁-C₆)alcoxy(C₁-C₃)alkyle ; un (C₃-C₅)cycloalkyle ; un (C₃-C₆)cycloalkyl(C₁-C₆)alkyle ;
- R₇ représente un phényle non substitué, mono ou disubstitué en position 3 ou 4 par un halogène, un groupe (C₁-C₅)alkyle, un groupe -CH₂OR₈ dans lequel R₈ représente un (C₁-C₃)alkyle ou par un groupe -O-CH₂-O- en position 3,4 ; ou bien R₇ représente un groupe (C₃-C₅)cycloalkyle ;
leurs sels d'addition, leurs hydrates et/ou leurs solvats.

L'invention concerne également les composés de formule (I.2), sous forme racémique ou d'énantiomère pur, dans lesquelles R₃ est en position 5 du phényle, ainsi que leurs sels d'addition, leurs hydrates et/ou leurs solvats.

Selon un autre de ses aspects, l'invention concerne les composés choisis parmi :
- Chlorhydrate de la [4-(2-chloro-4-méthoxy-5-méthylphényl)-5-méthylthiazol-2-yl]-[(1R)-(1-(3-fluoro-4-méthylphényl)-2-méthoxyéthyl)]prop-2-ynylamine (EXEMPLE 31)
- Chlorhydrate de la [4-(2-chloro-4-méthoxy-5-méthylphényl)-5-méthylthiazol-2-yl]-[(1S)-(1-phénylbutyl)]prop-2-ynylamine (EXEMPLE 33)
- Chlorhydrate de la [4-(2-chloro-4-méthoxy-5-méthylphényl)-5-méthylthiazol-2-yl]-[(1S)-(2-cyclopropyl-1-phényléthyl)]prop-2-ynylamine (EXEMPLE 34)
- Chlorhydrate de la [4-(2-chloro-4-méthoxyphényl)-5-méthylthiazol-2-yl]-[(1S)-(2-cyclopropyl-1-phényléthyl)]prop-2-ynylamine (EXEMPLE 35)
- Chlorhydrate de la [4-(2-chloro-4-méthoxy-5-méthylphényl)-5-méthylthiazol-2-yl]-[(1 S)-(2-cyclopropyl-1-(4-fluorophényl)éthyl)]prop-2-ynylamine (EXEMPLE 36)
- Chlorhydrate de la [4-(2-chloro-4-méthoxy-5-méthylphényl)-5-méthylthiazol-2-yl]-[(1S)-(1-phénylpentyl)]prop-2-ynylamine (EXEMPLE 37)
- Chlorhydrate de la [4-(2-chloro-4-méthoxy-5-méthylphényl)-5-méthylthiazol-2-yl]-[(1 R)-(2-méthoxy-1-(4-méthoxyméthylphényl)éthyl)]prop-2-ynylamine (EXEMPLE 40)
- Chlorhydrate de la [4-(2-chloro-4-méthoxy-5-méthylphényl)-5-méthylthiazol-2-yl]-[(1 S)-(1-(4-méthoxyméthylphényl)pentyl)]prop-2-ynylamine (EXEMPLE 42)
- Chlorhydrate de la [4-(2-chloro-4-méthoxy-5-méthylphényl)-5-méthylthiazol-2-yl]-[(1S)-(1-(4-fluorophényl)pentyl)]prop-2-ynylamine (EXEMPLE 45)
- Chlorhydrate de la [4-(2-chloro-4-méthoxy-5-méthylphényl)-5-méthylthiazol-2-yl]-[(1S)-(cyclopropyl-phényl-méthyl)]prop-2-ynylamine (EXEMPLE 47)
- Chlorhydrate de la [4-(2-chloro-4-méthoxy-5-méthylphényl)-5-méthylthiazol-2-yl]-[(1S)-(1-(3-fluoro-4-méthylphényl)pentyl)]prop-2-ynylamine (EXEMPLE 49)
- Chlorhydrate de la [4-(2-chloro-4-méthoxy-5-méthylphényl)-5-méthylthiazol-2-yl]-[(1S)-(2-cyclopropyl-1-(3-fluoro-4-méthylphényl)éthyl)]prop-2-ynylamine (EXEMPLE 50)
- Chlorhydrate de la [4-(2-chloro-4-méthoxy-5-méthylphényl)-5-méthylthiazol-2-yl]-[(1S)-(1-(4-fluorophényl)butyl)]prop-2-ynylamine (EXEMPLE 51)
- Chlorhydrate de la [4-(2-chloro-4-méthoxy-5-méthylphényl)-5-méthylthiazol-2-yl]-[(1S)-(1-(3-fluoro-4-méthoxyméthylphényl)butyl)]prop-2-ynylamine (EXEMPLE 52)
- Chlorhydrate de la [4-(2-chloro-4-méthoxy-5-méthylphényl)-5-méthylthiazol-2-yl]-[(1 S)-(2-cyclopropyl-1-(4-chlorophényl)éthyl)]prop-2-ynylamine (EXEMPLE 53)
- Chlorhydrate de la [4-(2-chloro-4-méthoxy-5-méthylphényl)-5-méthylthiazol-2-yl]-[(1S)-(2-cyclopropy)-1-(4-méthylphényl)éthyl)]prop-2-ynylamine (EXEMPLE 54)
- Chlorhydrate de la [4-(2-chloro-4-méthoxy-5-méthylphényl)-5-méthylthiazol-2-yl]-[(1S)-(2-cyclobutyl-1-(4-fluorophényl)éthyl)]prop-2-ynylamine (EXEMPLE 55)
- Chlorhydrate de la [4-(2-chloro-4-méthoxy-5-méthylphényl)-5-méthylthiazol-2-yl]-[(1S)-(2-cyclopropyl-1-(4-bromophényl)éthyl)]prop-2-ynylamine (EXEMPLE 56)
- Chlorhydrate de la [4-(2-chloro-4-méthoxy-5-méthylphényl)-5-méthylthiazol-2-yl]-[(1S)-(2-cyclopropyl-1-(3,4-méthylènedioxyphényl)éthyl)]prop-2-ynylamine (EXEMPLE 57)
- Chlorhydrate de la [4-(2-chloro-4-méthoxyphényl)-5-méthylthiazol-2-yl]-[(1 S)-(2-cyclopropyl-1-(3-fluoro-4-méthylphényl)éthyl)]prop-2-ynylamine (EXEMPLE 58)
- Chlorhydrate de la [4-(2,4-diméthoxy-5-méthylphényl)-5-méthylthiazol-2-yl]-[(1S)-(2-cyclopropyl-1-(3-fluoro-4-méthylphényl)éthyl)]prop-2-ynylamine (EXEMPLE 59)
- Chlorhydrate de la [4-(4-méthoxy-2,5-diméthylphényl)-5-méthylthiazol-2-yl]-[(1S)-(2-cyclopropy)-1-(3-fluoro-4-méthylphényl)éthyl)]prop-2-ynylamine (EXEMPLE 60)
- Chlorhydrate de la [4-(2-chloro-4-méthoxy-5-méthylphényl)-5-méthylthiazol-2-yl]-[(1S)-(1-(3,4-méthylènedioxyphényl)butyl)]prop-2-ynylamine (EXEMPLE 61)
ainsi que les bases correspondantes, les autres sels d'addition, leurs solvats et/ou hydrates.

Les composés de l'invention sous forme libre présentent généralement des propriétés de base faible. Toutefois suivant la nature des substituants certains peuvent présenter des propriétés acides.

Les sels des composés de formule (I) avec des acides ou des bases (lorsque cela est possible) pharmaceutiquement acceptables sont les sels préférés, mais ceux qui peuvent permettre d'isoler les composés de formule (I) notamment de les purifier ou d'obtenir des isomères purs, sont aussi objet de l'invention.

Parmi les acides pharmaceutiquement acceptables pour la préparation des sels d'addition aux composés de formule (I), on peut citer les acides chlorhydrique, bromhydrique, phosphorique, fumarique, citrique, oxalique, sulfurique, ascorbique, tartrique, maléique, mandélique, méthanesulfonique, lactobionique, gluconique, glucarique, succinique, sulfonique, hydroxypropane sulfonique.

Parmi les bases pharmaceutiquement acceptables pour la préparation des sels d'addition aux composés de formule (I) lorsque ceux-ci ont des propriétés acides, on peut citer l'hydroxyde de sodium, de potassium ou d'ammonium.

Les composés selon l'invention ainsi que les intermédiaires utiles pour leur préparation sont préparés selon des méthodes bien connues de l'homme de l'art, en particulier selon EP 576 350 et EP 659 747.

Le schéma réactionnel suivant illustre un procédé de préparation pour la synthèse des composés (I) pour lesquels le groupe R⁵ est un groupe propargyle.

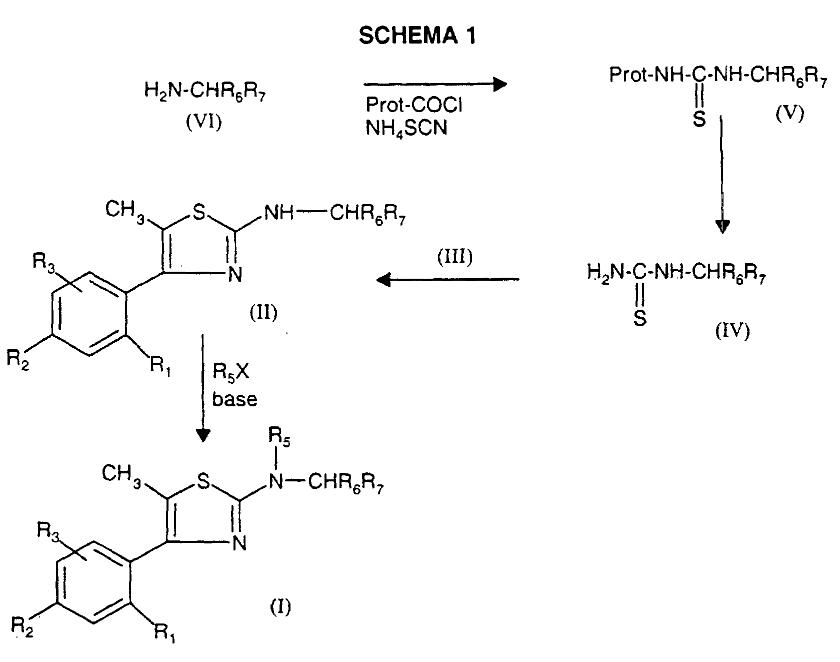

Selon un autre de ses aspects, la présente invention a également pour objet un procédé pour la préparation des composés de formule (I) caractérisé en ce que l'on fait réagir un dérivé alpha-halogéné, de préférence alpha-bromé ou alpha-chloré de formule (III) 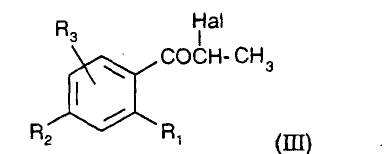 dans laquelle R₁, R₂ et R₃ sont tels que définis pour (I.2) et Hal représente un atome d'halogène de préférence de brome ou de chlore, avec une thiourée de formule : 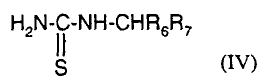 dans laquelle R₆ et R₇ sont tels que définis pour (I) pour obtenir un composé de formule (II) 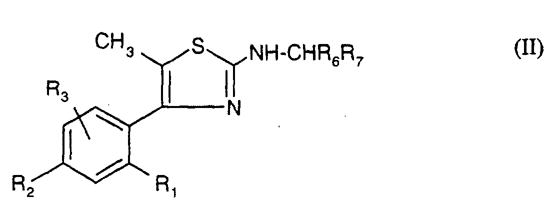 dans laquelle R₁, R₂, R₃, R₆ et R₇ sont tels que définis pour (I) pour le soumettre ensuite à une réaction d'alkylation pour fournir le composé (I).

Les réactions d'alkylation mises en oeuvre dans le procédé ci-dessus sont réalisées dans les conditions habituelles connues de l'homme de l'art par action d'un agent alkylant approprié tel que, par exemple, un halogénure d'alcényle ou d'alcynyle en présence d'une base de préférence l'hydrure de sodium.

Les dérivés de formule (III) peuvent être obtenus à partir des cétones correspondantes non halogénées de formule 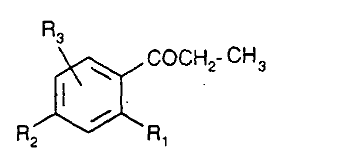 dans laquelle R₁, R₂, et R₃ sont tels que définis pour (I) soit (i) par action du brome dans un solvant organique approprié, tel que l'acide acétique, le tétrachlorure de carbone ou l'éther diéthylique, soit (ii) par action des tribromures d'ammonium quaternaires selon la méthode décrite dans Bull. Chem. Soc. Japan, 1987, 60, 1159-1160 et 2667-2668, soit (iii) encore par action du bromure cuivrique dans un solvant organique, tel qu'un mélange de chloroforme et d'acétate d'éthyle selon J. Org. Chem. 1964, 29, 3451-3461. En variante, les composés de formule (III) peuvent être obtenus par action du bromure de 2-bromopropionyle sur un benzène substitué de formule : 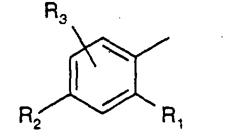 dans laquelle R₁, R₂ et R₃ sont tels que définis pour (I), par une réaction de Friedel et Crafts.

Les cétones mentionnées ci-dessus sont en général des produits connus ou disponibles dans le commerce. Ces composés peuvent être préparés par réaction de Friedel et Crafts, en présence d'un acide de Lewis selon des méthodes bien connues de l'homme de l'art.

Les dérivés de thiourées (IV) sont obtenus à partir des dérivés de thiourées protégées (V) 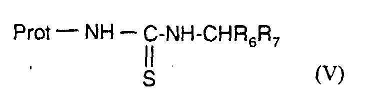 dans lesquels Prot représente un groupe protecteur, par exemple benzoyle ou pivaloyle, R₆ et R₇ étant tels que définis précédemment pour (I), soit par un traitement basique, en utilisant de préférence l'ammoniaque, l'hydroxyde de sodium ou de l'hydrazine à une température allant de la température ambiante au reflux du mélange réactionnel, soit par un traitement acide en utilisant de préférence l'acide chlorhydrique.

Les composés de formule (V) sont préparés en faisant réagir selon des méthodes connues, un isothiocyanate, par exemple un isothiocyanate de benzoyle ou un isothiocyanate de pivaloyle sur les amines correspondantes de formule (VI) :

H₂N-CHR₆R₇ (VI)

dans lesquelles R₆ et R₇ sont tels que définis pour (I).

La préparation des aminothiazoles optiquement actifs, c'est à dire sous forme d'énantiomères purs, est réalisée à partir d'amines primaires optiquement actives selon le SCHEMA 2 suivant par un procédé identique à celui précédemment décrit.

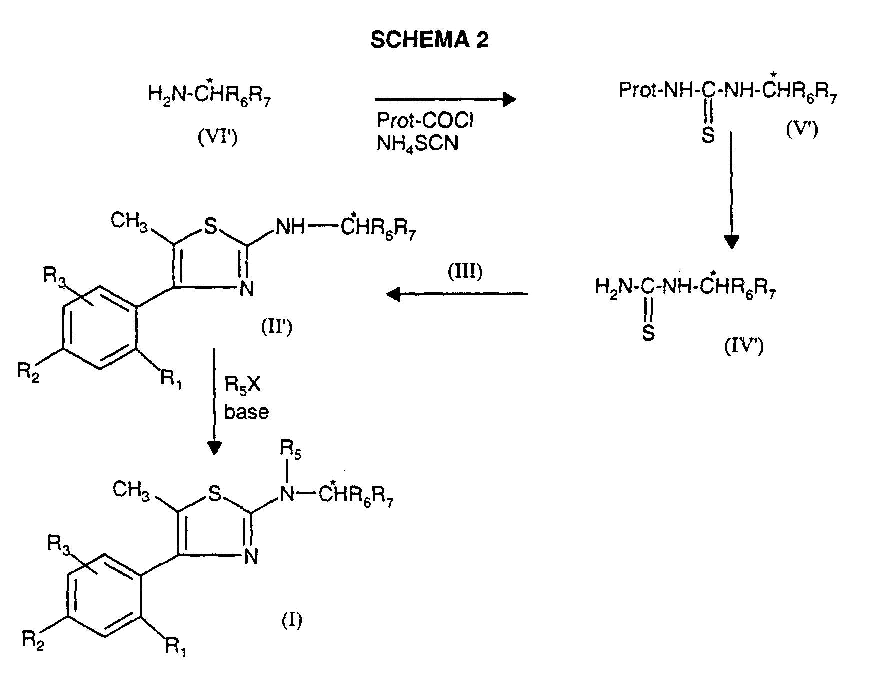

Les composés de formule (I), ci-dessus comprennent également ceux dans lesquels un ou plusieurs atomes d'hydrogène ou de carbone ont été remplacés par leur isotope radioactif par exemple le tritium ou le carbone-14. De tels composés marqués sont utiles dans les travaux de recherche, de métabolisme ou de pharmacocinétique, ou encore dans des essais biochimiques en tant que ligands de récepteur.

Les composés de la présente invention ont fait l'objet d'études biochimiques et pharmacologiques. Ils possèdent des propriétés pharmacologiques fort intéressantes.

Les composés de l'invention déplacent, à des concentrations inférieures à 10 µM, la liaison du CRF ou de peptides apparentés iodés (urotensine, sauvagine) par exemple ¹²⁵I tyrosine CRF, aux récepteurs présents sur membranes de cerveau ou sur des cellules en culture, selon la méthode décrite par E.B. De Souza (J. Neurosci., 1987, 7, 1 , 88-100).

L'activité antagoniste des composés selon l'invention a été démontrée par leur capacité à inhiber certaines activités associées au CRF. En particulier, les composés de formule (I) sont capables d'inhiber la sécrétion d'adrénocorticotropine (ACTH) induite par le CRF. L'étude sur la sécrétion d'ACTH induite par le CRF a été réalisée, *in vivo* chez des rats éveillés, selon une méthode adaptée de C. Rivier *et al*., Endocrinology, 1982, *110*(1), 272-278.

Le CRF est un neuropeptide qui contrôle l'activité de l'axe hypothalamo-hypophyso-surrénalien. Ce facteur est responsable des réponses endocrines et comportementales liées au stress.

En effet, il a été démontré que le CRF peut moduler le comportement comme aussi certaines fonctions du système nerveux autonome (G.F. Koob, F.E. Bloom, Fed. Proc., 1985, *44*, 259 ; M.R. Brown, L.A. Fisher, Fed. Proc., 1985, *44*, 243). Plus particulièrement, le CRF induit la sécrétion de la corticotropine (ACTH), des β-endorphines et autres peptides dérivés de la pro-opiomélanocortine (A. Tazi *et al*., Régul. Peptides, 1987, *18*, 37 ; M.R. Brown *et al*, Regul. Peptides, 1986, *16*, 321 ; C.L. Williams *et al*., Am. J. Physiol., 1987, G 582, 253).

Les composés de l'invention peuvent donc être utiles à la régulation de la sécrétion de ces substances endogènes. Ils trouvent plus spécialement leurs applications en tant que principes actifs des médicaments pour diminuer la réponse au stress (comportement, états émotionnels, troubles gastro-intestinaux et cardiovasculaires, désordres du système immunitaire) et plus généralement dans les pathologies impliquant le CRF, par exemple les désordres psychiatriques, l'anxiété, la dépression, l'anorexie nerveuse, l'épilepsie, les troubles de l'activité sexuelle et de la fertilité, la maladie d'Alzheimer ou autres.

Les composés de l'invention sont très stables et sont donc ainsi particulièrement appropriés pour constituer le principe actif de médicaments.

L'invention s'étend aussi aux compositions pharmaceutiques contenant comme principe actif, un composé de formule (I) ou l'un de ses sels pharmaceutiquement acceptables, éventuellement en association avec un ou plusieurs excipients inertes et appropriés.

Dans chaque unité de dosage le principe actif de formule (I) est présent dans les quantités adaptées aux doses journalières envisagées. Chaque unité de dosage est convenablement ajustée selon le dosage et le type d'administration prévu, par exemple comprimés, gélules et similaires, sachets, ampoules, sirops et similaires, gouttes, patch transdermique ou transmucosal de façon à ce qu'une telle unité de dosage contienne 0,5 à 800 mg de principe actif, de préférence 0,5 à 200 mg.

Les composés selon l'invention peuvent également être utilisés en association avec un autre principe actif utile pour la thérapeutique souhaitée tels que par exemple des anxiolytiques, des antidépresseurs ou des anorexigènes.

Les composés de formule (I) sont peu toxiques ; leur toxicité est compatible avec leur utilisation comme médicament pour le traitement des troubles et des maladies ci-dessus.

Les composés de formule (I), peuvent être formulés dans des compositions pharmaceutiques pour l'administration aux mammifères, y compris l'homme, pour le traitement des maladies susdites.

Les compositions pharmaceutiques ainsi obtenues sont présentées avantageusement sous des formes diverses, telles que par exemple, des solutions injectables ou buvables, dragées, comprimés ou gélules. Les compositions pharmaceutiques contenant comme principe actif au moins un composé de formule (I) ou l'un de ses sels, sont notamment utiles pour le traitement à titre préventif ou curatif, des maladies liées au stress et plus généralement dans le traitement de toutes les pathologies impliquant le CRF, tels que par exemple : la maladie de Cushing, les désordres neuropsychiatriques comme la dépression, l'anxiété, la panique, les désordres compulsifs obsessionnels, les troubles de l'humeur, le stress post-traumatique, les troubles du comportement, l'agressivité, l'anorexie, la boulimie, l'hyperglycémie, le travail prémature, les grossesses à risques, la croissance retardée, les troubles du sommeil, l'épilepsie, et les dépressions de tout types ; la maladie d'Alzheimer, de Parkinson ; la chorée de Huntington ; la sclérose latérale amyotrophique ; les troubles vasculaires, cardiaques et cérébraux ; les troubles de l'activité sexuelle et de la fertilité ; l'immunodépression, l'immunosuppression, les processus inflammatoires, les infections multiples, l'arthrite rhumatoïde, l'ostéo-arthrite, les uvéites, le psoriasis ainsi que le diabète ; les cancers ; les troubles fonctionnels gastro-intestinaux et les inflammations qui en découlent (colon irritable et inflammatoire, diarrhées) ; les troubles de perception de la douleur, les fibromyalgies liées ou non aux troubles du sommeil, la fatigue, la migraine ; les symptômes liés à la dépendance (alcoolique) et au sevrage de drogues.

La posologie peut largement varier en fonction de l'âge, du poids et de l'état de santé du patient, de la nature et de la sévérité de l'affection, ainsi que de la voie d'administration. Cette posologie comprend l'administration par jour d'une ou plusieurs doses d'environ 0,5 mg à 800 mg, de préférence d'environ 0,5 à 200 mg.

Dans les compositions pharmaceutiques de la présente invention pour l'administration orale, sublinguale, sous-cutanée, intramusculaire, intraveineuse, transdermique, transmucosale, locale ou rectale, le principe actif peut être administré sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration transmucosale le principe actif peut être formulé en présence d'un promoteur tel qu'un sel biliaire, d'un polymère hydrophile tel que par exemple l'hydroxypropylcellulose, l'hydroxypropylméthylcellulose, l'hydroxyéthyl-cellulose, l'éthylcellulose, la carboxyméthylcellulose, le dextran, la polyvinylpyrrolidone, les pectines, les amidons, la gelatine, la caséine, les acides. acryliques, les esters acryliques et leurs copolymères, les polymères ou copolymères de vinyle, les alcools vinyliques, les alcoxypolymères, les polymères d'oxyde de polyéthylène, les polyéthers ou leur mélange.

Le principe actif peut être formulé également sous forme de microcapsules, éventuellement avec un ou plusieurs supports ou additifs.

Le principe actif peut être également présenté sous forme de complexe avec une cyclodextrine, par exemple α-, β- ou γ- cyclodextrine, 2-hydroxypropyl-β-cyclodextrine ou méthyl-β-cyclodextrine.

Les EXEMPLES suivants, donnés à titre non limitatif, illustrent l'invention.

Dans les PREPARATIONS sont décrites les méthodes de synthèse des différents intermédiaires permettant d'obtenir les composés de l'invention. Ces intermédiaires sont tous obtenus selon des méthodes bien connues de l'homme de l'art.

Les points de fusion ont été mesurés selon la technique Micro-Köfler et sont exprimés en degré Celsius.

Les spectres de résonance magnétique nucléaire du proton (¹H RMN) ont été effectués dans le CDCl₃ sauf mention contraire, à 200 MHz ou à 300 MHz. Les déplacements chimiques sont exprimés en p.p.m. et les constantes de couplage en Hertz.

Les excès énantiomériques (ee) sont évalués à partir des chromatogrammes obtenus soit par chromatographie HPLC sur phase chirale, soit par chromatographie par fluide supercritique (SFC) chirale.

Les pouvoirs rotaroires des produits optiquement actifs sont caractérisés par leur [α]^{t°}_{D} (les concentrations c des solutions analysées sont exprimées en grammes pour 100 ml)

Les abréviations utilisées ci-après sont les suivantes : s = singulet ; m = multiplet ; d = doublet ; t = triplet ; q = quadruplet.

Les composés de l'invention présentent une analyse centésimale conforme à la théorie.

Les composés de l'invention décrits dans les TABLEAUX 3 et 5 ont également des spectres RMN conformes à leur structure.

### PREPARATION DES α-BROMOCETONES DE FORMULE (III)

### • 2-Bromo-1-(2-chloro-4-méthoxy-5-méthylphényl)propan-1-one Composé III.1

Une solution de 46 g (280 mmol) de 4-chloro-2-méthoxy-toluène dans 150 ml de dichlorométhane est agitée à 0°C et additionnée de 29,4 g (280 mmol) de bromure de 2-bromopropionyle. Le mélange est additionné par portions de 39,2 g (294 mmol) de trichlorure d'aluminium. Il est agité en laissant la température remonter progressivement à l'ambiante. Après agitation pendant 4 heures, le mélange réactionnel est versé lentement sur de la glace. Ce mélange sous agitation est additionné de 50 ml d'acide chlorhydrique 1 N et 1 litre d'eau, puis est extrait par 1,2 litre de *ter*-butyl-méthyl-éther. La phase organique est lavée par de l'eau, une solution aqueuse saturée d'hydrogénocarbonate de sodium, de l'eau, puis une solution saturée en chlorure de sodium. Elle est séchée sur sulfate de sodium anhydre puis évaporée à sec. Le résidu brut est purifié par chromatographie sur gel de silice (solvant: cyclohexane-acétate d'éthyle 50/1 ). On obtient 67g de *composé III-1*. R=82%.
RMN ¹H : 7,44(s, **Ar**, 1 H) ; 6,86(s, **Ar**, 1 H) ; 5,41 (q, J=5,35Hz, C**H**, 1H) ; 3,90(s, OC**H**₃, 3H) ; 2,23(s, C**H**₃, 3H) ; 1,91 (d, J=5,35Hz, C**H**₃, 3H).

Par la même méthode ont été synthétisés :
- **2-Bromo-1-(2-chloro-4-méthoxyphényl)propan-1-one *Composé III.2***
- **2-Bromo-1-(2,4-dichloro-5-méthylphényl)propan-1-one *Composé III.3***
- **2-Bromo-1-(2,4-diméthoxy-5-méthylphényl)propan-1-one *Composé III.4***
- **2-Bromo-1-(4-méthoxy-2,5-diméthylphényl)propan-1-one *Composé III.5***

### PREPARATION DES AMINES RACEMIQUES DE FORMULE (VI)

### Première Méthode

### a) 2-amino-2-(4-fluorophényl)éthanol Composé 1.1

60 ml (60 mmol) d'une solution 1 M d'hydrure d'aluminium-lithium dans le tétrahydrofurane sont agités à reflux puis additionnés par portions de 5 g (29 mmol) de 4-fluoro-DL-α-phénylglycine (FLUKA). Après une agitation à reflux de six heures, le mélange réactionnel est agité à 0°C puis additionné lentement de 2,5 ml d'eau, 2,5 ml d'une solution aqueuse d'hydroxyde de sodium à 15 % puis 7,5 ml d'eau. La suspension obtenue est filtrée sur célite. Le filtrat est concentré et repris par 300 ml de dichlorométhane. La solution est lavée avec une solution saturée en chlorure de sodium, séchée sur sulfate de sodium anhydre puis évaporée à sec. On obtient 3,3 g de produit huileux jaune. R = 73 %.
MS (MH⁺ = 156)
¹H RMN : 7,23-7,33(m, **Ar**, 2H) ; 6,95-7,07(m, **Ar**, 2H) ; 4,08(m, C**H**, 1 H) ; 3,45-3,86(m, C**H**₂O, 2H) ; 2,03(s, N**H**₂ et O**H**, 3H).

### b) 1-(4-fluorophényl)-2-méthoxyéthylamine Composé VI.1

0,94 g (23 mmol) d'hydrure de potassium obtenus par lavage au pentane de 2,2 g d'une suspension huileuse, sont mis en suspension dans 18 ml de tétrahydrofurane et agités à 10°C. Une solution de 3,3 g (21 mmol) de *Composé 1.1*. dans 43 ml de tétrahydrofurane est additionnée lentement. Après agitation pendant seize heures à température ambiante, une solution de 1,3 ml (20,8 mmol) d'iodométhane dans 25 ml de tétrahydrofurane est additionnée en une heure trente minutes. Le mélange réactionnel est agité pendant trois heures à température ambiante puis est versé sur 300 ml d'eau glacée salée. Le mélange est extrait par 500 ml de *ter*-butylméthyloxyde. La phase organique est lavée à l'eau puis avec une solution saturée en chlorure de sodium, séchée sur sulfate de sodium anhydre puis évaporée à sec. On obtient 3,2 g d'amine huileuse. R = 88 %.
¹H RMN : 7,24-7,38(m, **Ar**, 2H) ; 6,93-7,05(m, **Ar**, 2H) , 4,16(m, C**H**, 1H) ; 3,45(dd, C**H**_{**2**}, 1H) ; 3,36(s, OC**H**_{**3**}, 3H) ; 3,29(d, C**H**_{**2**}, 1H) ; 1,70(s, N**H**_{**2**}, 2H).
- De la même façon, on obtient la **2-méthoxy-1-phényléthylamine Composé VI.2**

### Deuxième Méthode

### a) Synthèse de phénylcétones substituées. Composés 3

### Mode opératoire A :

### • 1-(3-fluoro-4-méthylphényl)-2-méthoxyéthan-1-one Composé 3.1

Pour préparer le magnésien on laisse 14 g (583 mmol, 1 éq.) de copeaux de magnésium sous agitation en présence de verre pilé et sous argon pendant une nuit. On les recouvre de 400 ml d'éther diéthylique, puis on ajoute une pointe de spatule d'iode. On ajoute lentement 110 g (582 mmol) de 4-bromo-2-fluorotoluène en solution dans 700 ml d'éther diéthylique de façon à maintenir un léger reflux, puis on chauffe le mélange réactionnel à reflux pendant trois heures. On ajoute 39 ml de méthoxyacétonitrile (610 mmol, 1, 1 éq.) et on laisse réagir pendant deux heures. Une fois la réaction terminée on verse le mélange réactionnel sur 1,5 kg de glace, puis sous agitation on ajoute 300 ml d'acide chlorhydrique concentré. On extrait à l'éther diéthylique, on sèche sur sulfate de sodium et on évapore. On récupère 77 g de *Composé 3.1* que l'on utilise directement dans la seconde étape sans le purifier.

De la même façon, on obtient les composés suivants :
- **1-(4-chloro-3-fluorophényl)-2-méthoxyéthan-1-one *Composé 3.2***
- **1-(4-chlorophényl)-2-méthoxyéthan-1-one *Composé 3.3***
- **3-cyclopropyl-1-(4-fluorophényl)propan-1-one *Composé 3.4***

### Mode opératoire B :

### • 2-méthoxy-1-(4-méthoxyméthylphényl)éthan-1-one Composé 3.5

Une solution de 62 g (308 mmol) de 1-bromo-4-méthoxyméthylphényle dans 600 ml de tétrahydrofurane est agitée à -70°C et additionnée lentement de 200 ml (320 mmol) d'une solution 1,6 M de butyl-lithium. Le mélange réactionnel est agité pendant 30 minutes à -70°C, puis additionné lentement d'une solution de 50 g (380 mmol) de 2,N-diméthoxy-N-méthyl-acétamide. Le mélange réactionnel est agité en laissant la température remonter progressivement à l'ambiante. Après 4 heures sous agitation, il est refroidi à 0°C et additionné lentement d'une solution aqueuse saturée de chlorure d'ammonium. Le mélange est extrait par l'acétate d'éthyle et la phase organique est lavée à l'eau puis avec une solution saturée en chlorure de sodium, séchée sur sulfate de sodium anhydre puis évaporée à sec. Le résidu obtenu est purifié par chromatographie sur gel de silice (solvant : cyclohexane-acétate d'éthyle 9/1 puis 3/1). On obtient 32 g de cétone. R=53 %
RMN ¹H : 7,89(d, J=8,1 Hz, **Ar**, 2H) ; 7,40(d, J=8,1 Hz, **Ar**, 2H) ; 4,66(s, OC**H**_{**2**}, 2H) ; 4,48(s, OC**H**₂, 2H) ; 3,47(s, C**H**₃, 3H) ; 3,38(s, C**H**₃, 3H)

### b) Synthèse des oximes Composés 4

### • 1-(3-fluoro-4-méthylphényl)-2-méthoxyéthan-1-one oxime Composé 4.1

### Mode opératoire A :

On mélange 33 g de chlorhydrate d'hydroxylamine (475 mmol, 1,6 éq.) à 30 ml d'eau et 100 ml d'éthanol. A 0°C on ajoute 54 g (296 mmol) du *Composé 3.1* dilué dans 30 ml d'éthanol. Une fois l'addition terminée, on ajoute 60 g de pastilles d'hydroxyde de sodium (1,5 mol, 5 éq.) préalablement concassées en maintenant la température en dessous de 30°C. On laisse le mélange réactionnel pendant une nuit à température ambiante, puis on se place à 0°C pour neutraliser le mélange réactionnel avec de l'acide chlorhydrique concentré (pH < 7). On extrait ensuite à l'acétate d'éthyle, on lave la phase organique à l'eau et avec une solution saturée de chlorure de sodium. On sèche la phase organique sur sulfate de sodium, on évapore. L'huile ainsi obtenue est chromatographiée sur gel de silice avec pour éluant un mélange acétate d'éthyle cyclohexane 1/9 (v/v). On obtient 26 g d'isomère (Z) et 9 g d'isomère (E), soit un rendement R = 45 % en (Z) et 16 % en (E).

### Mode opératoire B :

On mélange 47 g de chlorhydrate d'hydroxylamine (676 mmol, 1,6 éq.) à 275 ml de pyridine. A 0°C on ajoute 77 g (423 mmol) de *Composé 3.1*. On laisse le mélange réactionnel pendant cinq heures à température ambiante. Une fois la réaction terminée, on évapore la pyridine puis on extrait ensuite au dichlorométhane. On lave la phase organique à l'eau puis avec une solution saturée de chlorure de sodium. On sèche la phase organique sur sulfate de sodium, on évapore et on chromatographie sur gel de silice l'huile ainsi obtenue avec pour éluant un mélange acétate d'éthyle cyclohexane 1/9 (v/v) pour obtenir 42,5 g de composé (Z) et 14 g de composé (E), soit un rendement R = 51 % en Z et 17 % en E.
¹H RMN du composé Z : 11,59(N-OH, s, 1 H) ; 7,20-7,40 (**Ar**, m, 3H) ; 4,51(-O-C**H**_{**2**}-, s, 2H) ; 3,18(OC**H**_{**3**}, s, 3H) ; 2,20 (C**H**_{**3**}-Ph, s, 3H).
¹H RMN du composé E : 11,30(N-O**H**, s, 1 H) ; 7,20-7,50(**Ar**, m, 3H) ; 4,21(-O-C**H**_{**2**}-, s, 2H) ; 3,17(OC**H**_{**3**}, s, 3H) ; 2,22(C**H**_{**3**}-Ph, s, 3H).

De la même façon on obtient les produits suivants par un des deux modes opératoires précités :
- **1-(4-chloro-3-fluorophényl)-2-méthoxyéthan-1-one oxime *Composé 4.2***
- **1-(4-chlorophényl)-2-méthoxyéthan-1-one oxime *Composé 4.3***
- **1-phénylbutan-1-one oxime *Composé 4.4***
- **1-(4-méthoxyméthylphényl)-2-méthoxyéthan-1-one oxime *Composé 4.5***
- **1-(4-méthoxyméthylphényl)butan-1-one oxime *Composé 4.6***
- **dicyclobutylcétone oxime *Composé 4.7***
- **1-phényl-pentan-1-one oxime *Composé 4.8***

### c) Synthèse des amines Composés VI

### • 1-(3-fluoro-4-méthylphényl)-2-méthoxyéthylamine Composé VI.3

A 10 ml d'une solution d'hydrure d'aluminium-lithium 1 M dans le tétrahydrofurane (10 mmol, 8 éq.), on ajoute lentement, à 0°C, une solution de 1 g de *Composé 4.1* (5 mmol) dissout dans 15 ml de tétrahydrofurane. On laisse remonter à température ambiante puis on laisse réagir le mélange réactionnel pendant deux heures et on chauffe à reflux pendant une heure. On refroidit le mélange réactionnel à zéro degré pour additionner 10 ml d'eau. On extrait la phase aqueuse à l'éther diéthylique. Les phases organiques réunies sont extraites par une solution d'acide chlorhydrique 2N.

La phase aqueuse acide obtenue est agitée à 0°C et additionnée d'une solution d'hydroxyde de sodium à 35%. La solution alcaline obtenue est extraite par du dichlorométhane. La phase organique est lavée à l'eau saturée en chlorure de sodium puis séchée sur sulfate de sodium et évaporée à sec. Après filtration sur silice avec pour éluant un mélange dichlorométhane/méthanol 95/5 (v/v) on obtient 0,6 g de *Composé VI.3*. R = 65 %.
¹H RMN : 6,90-7,20(**Ar**, m, 3H) ; 4,14(-C**H**-N, dd, J = 4 et 8,5, 1H) ; 3,47(-C**H**_{**2**}-O, dd, J = 4 et 9, 1H) ; 3,37(OC**H**_{**3**}, s, 3H) ; 3,32(-C**H**_{**2**}-O, dd, 1 H, j = 8,5 et 9) ; 2,24(C**H**_{**3**}-Ph, d, J = 1,8, 3H) ; 1,68(-N**H**_{**2**}, s, 2H).

De la même façon on obtient les composés suivants :
- **1-(4-chloro-3-fluorophényl)-2-méthoxyéthylamine *Composé VI.4***
- **dicyclobutylméthylamine *Composé VI.5***

### Troisième méthode :

### a) Synthèse de O-alkyl-oxime Composés 6

### Mode opératoire A :

### • 1-phénylbutan-1-one O-méthyloxime Composé 6.1

A 66 g (0,40 mole) de 1-phénylbutan-1-one oxime (*Composé 4.4*) dans un mélange de 400 ml de diméthylformamide et de tétrahydrofurane (1:1), sont ajoutés par petites portions 18 g (0,45 mole) d'hydrure de sodium à 55 % dans l'huile, à 0°C et en une heure. Après addition de 31 ml (0,5 mole) d'iodure de méthyle, le mélange réactionnel devient progressivement très épais. Après addition de 50 ml d'éthanol, puis d'eau, le mélange réactionnel est extrait par 4 x 250 ml d'acétate d'éthyle. La phase organique est lavée à l'eau saturée de chlorure de sodium, séchée sur sulfate de sodium, puis évaporée sous vide. On obtient 75 g d'huile jaune pâle, mélange d'isomères géométriques ((Z) 7 % et (E) 93 %). R = 94% (Z+E).

Ces deux isomères peuvent être séparés par chromatographie sur silice en éluant avec un mélange cyclohexane/acétate d'éthyle.
¹H RMN : 7,56-7,93(m, **Ar**, 2H) ; 7,24-7,40(m, **Ar**, 3H) 3,95[s, OC**H**_{**3**}, (E)] ; 3,82[s, OC**H**_{**3**}, (Z)] ; 2,71[m, C**H**_{**2**}, (E)] ; 2,50[m, C**H**_{**2**}, (Z)] ; 1,41-1,64(m, C**H**_{**2**}, 2H) ; 0,84-1,03(m, C**H**_{**3**}, 3H).

De la même façon, on obtient les oximes alkylées suivantes :
- **1-phénylpentan-1-one *O*-méthyloxime *Composé 6.2***
- **1-(4-chlorophényl)-2-méthoxyéthan-1-one *O*-benzyloxime *Composé 6.3***
- **2-méthoxy-1-(4-méthoxyméthylphényl)éthan-1-one *O*-méthyloxime *Composé 6.4***
- **1-(4-méthoxyméthylphényl)butan-1-one *O*-méthyloxime *Composé 6.5***

### Mode opératoire B :

### • Cyclobutyl-(4-fluorophényl)cétone O-benzyloxime Composé 6.6

Une solution de 15 g (84 mmol) de cyclobutyl-(4-fluorophényl)cétone dans 80 ml d'éthanol est agitée à température ambiante et additionnée de 20,2 g (126 mmol) de chlorhydrate de O-benzylhydroxylamine. Le mélange est ensuite additionné par portions de 8,4 g (210mmol) d'hydroxyde de sodium et agité pendant 4 heures à température ambiante. Le mélange est additionné d'eau et extrait par l'acétate d'éthyle. La phase organique est lavée à l'eau jusqu'à neutralité, puis avec de l'eau saturée de chlorure de sodium. Elle est séchée sur sulfate de sodium, puis évaporée à sec . On obtient 28,4 g d'un mélange d'isomères (E 58%, Z 42%)
¹H RMN (DMSO D₆) : 7,15-7,48(m, **Ar**, 9H) ; 5,07[s, OC**H**_{**2**}, (E)] ; 5,01[s, OC**H**_{**2**}, (Z)] ; 3,68-3,82[m, C**H** cyclobutyle, (E)] ; 3,36-3,52[m, **CH** cyclobutyle, (Z)] ; 1,58-2,30(m, C**H**_{**2**} cyclobutyle, 6H).

La même méthode est utilisées pour obtenir le composé suivant :
- **3-cyclopropyl-1-(4-fluorophényl)propan-1-one *O*-benzyloxime *Composé 6.7***

### b) Synthèse des amines Composés VI

### • 1-phénylbutylamine Composé VI.6

A 85 ml d'une solution 1 M d'hydrure d'aluminium lithium dans le tétrahydrofurane, est ajoutée sous argon et goutte à goutte, une solution de 14,2 g (0,085 mole) de 1-phénylbutan-1-one *O*-méthyloxime (*Composé 6. 1*) dans 85 ml de tétrahydrofurane. A la fin de l'addition, le mélange réactionnel est chauffé pendant une heure trente à reflux. Après une nuit à température ambiante sont ajoutés successivement 3,5 ml d'H₂O, puis 3,5 ml d'hydroxyde de sodium 15 % puis 10,5 ml H₂O. Le précipité est filtré et lavé à l'éther diéthylique. Le filtrat tétrahydrofurane/éther diéthylique est lavé à l'eau, puis est extrait trois fois avec une solution d'acide chlorhydrique 1 N. Les phases aqueuses acides sont réunies puis alcalinées à 0°C par l'hydroxyde de sodium 35 %. Après extractions avec du dichlorométhane, lavages à l'eau, séchage sur sulfate de sodium puis évaporation sous vide, on obtient 9,3 g d'huile, R = 73 %.
¹H RMN : 7,11-7,36(m, **Ar**, 5H) ; 3,81-3,95(m, C**H**, 1H) ; 1,73(s, N**H**₂, 2H) ; 1,60-1,70(m, C**H**₂, 2H) ; 1,15-1,36(m, C**H**₂, 2H) ; 0,95-0,98(m, C**H**₃, 3H).

De la même façon, on obtient les amines suivantes :
- **1-phénylpentylamine *Composé VI.7***
- **1-(4-chlorophényl)-2-méthoxyéthylamine *Composé VI.8***
- **2-méthoxy-1-(4-méthoxyméthylphényl)éthylamine *Composé VI.9***
- **1-(4-méthoxyméthylphényl)butylamine *Composé VI.10***
- **Cyclobutyl-(4-fluorophényl)méthylamine *Composé VI.11***
- **3-cyclopropyl-1-(4-fluorophényl)propylamine *Composé VI.12***

### Quatrième méthode :

### • 1-(4-fluorophényl)pentylamine Composé VI.13

Une solution de 1,21 g (10 mmol) de 4-fluorobenzonitrile dans 10 ml de tétrahydrofurane est agitée à 0°C et additionnée goutte à goutte de 10 ml (10 mmol) d'une solution 1M de borane-tétrahydrofurane. Le mélange est agité pendant une heure trente minutes à température ambiante puis transféré lentement sur 18,8 ml d'une solution 1,6 M de butyllithium dans l'hexane préalablement refroidie et agitée à - 78°C. Le mélange réactionnel est agité pendant deux heures à -78°C puis hydrolysé à cette température par 10 ml d'acide chlorhydrique 2N. La phase organique est extraite par de l'acide chlorhydrique 2N et la phase aqueuse acide obtenue est neutralisée à 0°C par addition lente d'hydroxyde de sodium à 35 % puis extraite par l'acétate d'éthyle. La phase organique est lavée à l'eau puis avec une solution saturée en chlorure de sodium, séchée sur sulfate de sodium anhydre puis évaporée à sec. On obtient 0,95 g d'amine huileuse. R = 53 %.
¹H RMN : 7,21-7,31(m, **Ar**, 2H) ; 6,93-7,05(m, **Ar**, 2H) ; 4,13(t, C**H**, 1H) ; 1,59-1,75(m, C**H**_{**2**}, 2H) ; 1,49(s, N**H**_{**2**}, 2H) ; 1,24-1,33(m, C**H**_{**2**}-C**H**_{**2**}, 4H) ; 0,85(t, C**H**_{**3**}, 3H).

De la même façon, on obtient la :
- **1-(3-fluoro-4-méthylphényl)pentylamine *Composé VI.14***

### Cinquième méthode :

### • 1-(4-fluorophényl)butylamine Composé VI.15

A une suspension de 2,4 g (100 mmol) de magnésium dans 30 ml d'éther diéthylique sont ajoutés un cristal d'iode puis 17,4 g (100 mmol) de 4-bromofluorobenzène (dilué dans 70 ml d'éther diéthylique) de façon à créer un léger reflux. Le mélange réactionnel est chauffé à reflux pendant une heure puis refroidi à température ambiante et additionné de 5,75 g (85 mmol) de butyronitrile dilué dans 30 ml d'éther diéthylique. Le mélange réactionnel est chauffé à reflux pendant deux heures puis refroidi et filtré sur coton de verre. Le filtrat est agité à température ambiante et additionné lentement de 100 ml (100 mmol) d'une solution 1M d'hydrure d'aluminium lithium dans le tétrahydrofurane. Le mélange réactionnel est agité à reflux pendant dix huit heures puis est refroidi à 0°C et additionné successivement de 3,8 ml d'eau, 3,8 ml d'hydroxyde de sodium à 15 %, puis 11,4 ml d'eau. Le mélange est filtré sur célite et le filtrat est évaporé à sec. Le résidu obtenu est filtré sur silice, éluant dichlorométhane/méthanol 98/2 (v/v). On obtient 6,3 g de produit huileux. R = 37%.
¹H RMN : 7,22-7,36(m, **Ar**, 2H) ; 6,92-7,05(m, **Ar**, 2H) ; 3,87(t, C**H**, 1H) ; 1,45-1,65(m, C**H**_{**2**}, 2H) ; 1,12-1,40(m, C**H**_{**2**}, 2H) ; 0,88(t, C**H**_{**3**}, 3H).

### PREPARATION DES THIOUREES RACEMIQUES, Composés IV

### • N[1-(4-fluorophényl)-2-méthoxyéthyl]thiourée Composé IV.1

A^{^{·}} une solution de 4,5 g (58 mmol) d'isothiocyanate d'ammonium dans 115 ml d'acétone agitée à 0°C sont additionnés 6,5 ml (56,6 mmol) de chlorure de benzoyle. Après trente minutes, 8,6 g (56 mmol) de *Composé VI.1* en solution dans 100 ml d'acétone sont ajoutés lentement. Le mélange réactionnel est agité pendant deux heures à température ambiante puis concentré sous pression réduite. La suspension est reprise par 200 ml de *ter*-butylméthyloxyde et 200 ml d'eau. La phase organique est lavée à l'eau, puis avec une solution saturée en chlorure de sodium, séchée sur sulfate de sodium anhydre puis évaporée à sec. Le résidu d'évaporation est dissout dans 180 ml d'éthanol et la solution obtenue est additionnée de 5,85 ml (116 mmol) d'hydrazine monohydratée. Après agitation pendant seize heures à température ambiante, la réaction n'étant pas complète, 1,7 ml d'hydrazine sont rajoutés. Après agitation pendant vingt quatre heures à température ambiante, le mélange réactionnel est évaporé. Le résidu d'évaporation est dissout par 500 ml d'acétate d'éthyle et la phase organique est lavée à l'eau, puis avec une solution saturée en chlorure de sodium, séchée sur sulfate de sodium anhydre et évaporée à sec. Le résidu d'évaporation est chromatographié sur une colonne de silice, éluant : cyclohexane/acétate d'éthyle 1/1, (v:v). On obtient 8,5 g (40 mmol) de produit solide blanc. R = 69 %. F = 154°C.
¹H RMN (DMSO-D₆) : 8,10(d, N**H**, 1H) ; 7,28-7,32(m, **Ar**, 2H) ; 7,08-7,17(m, **Ar** et N**H**_{**2**}, 4H) ; 5,45(m, C**H**, 1H) ; 3,54-3,62(m, CH-C**H**_{**2**}, 2H) ; 3,24(s, OC**H**_{**3**}, 3H).

De la même façon, on obtient les thiourées suivantes décrites dans le TABLEAU 1 :

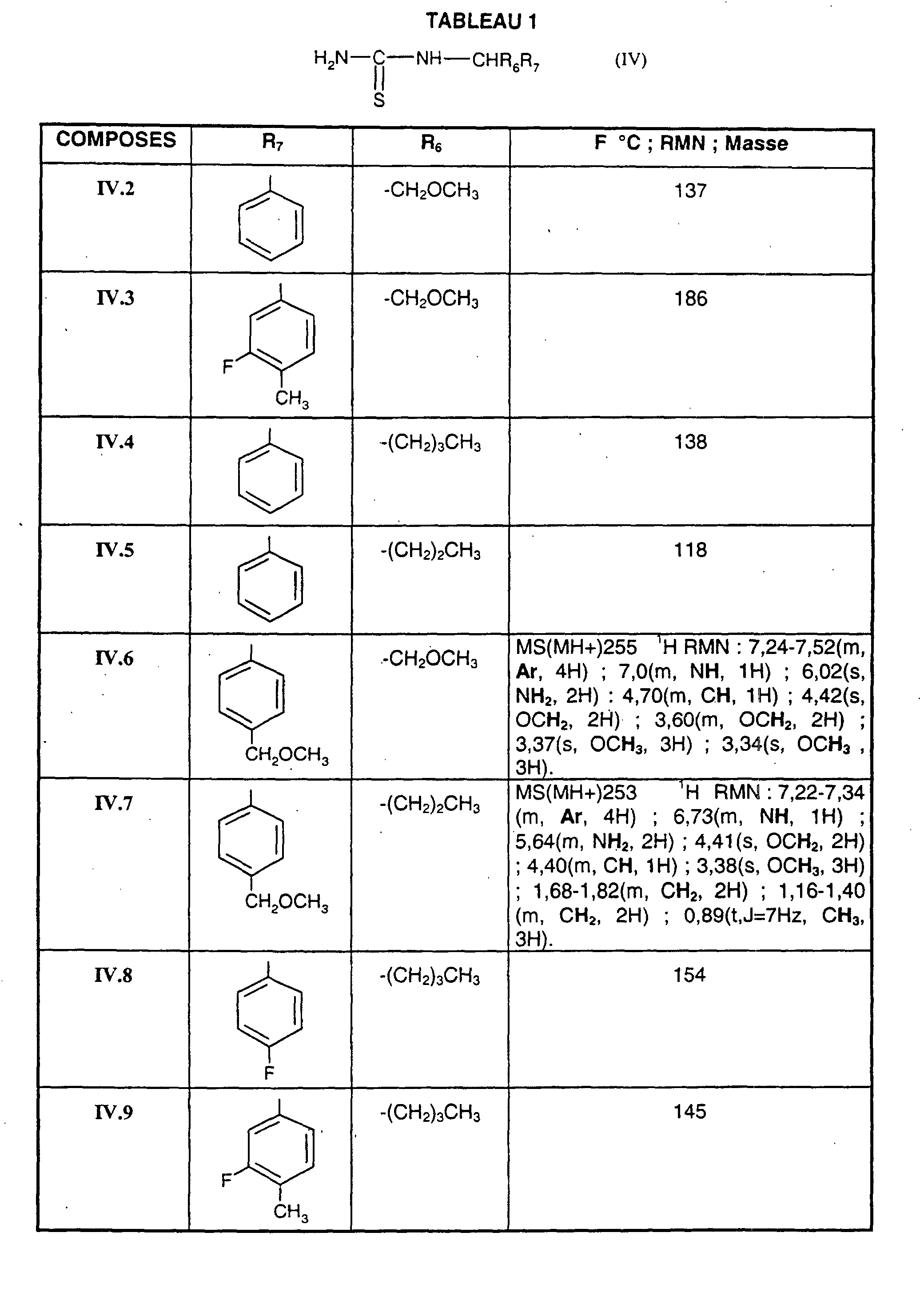

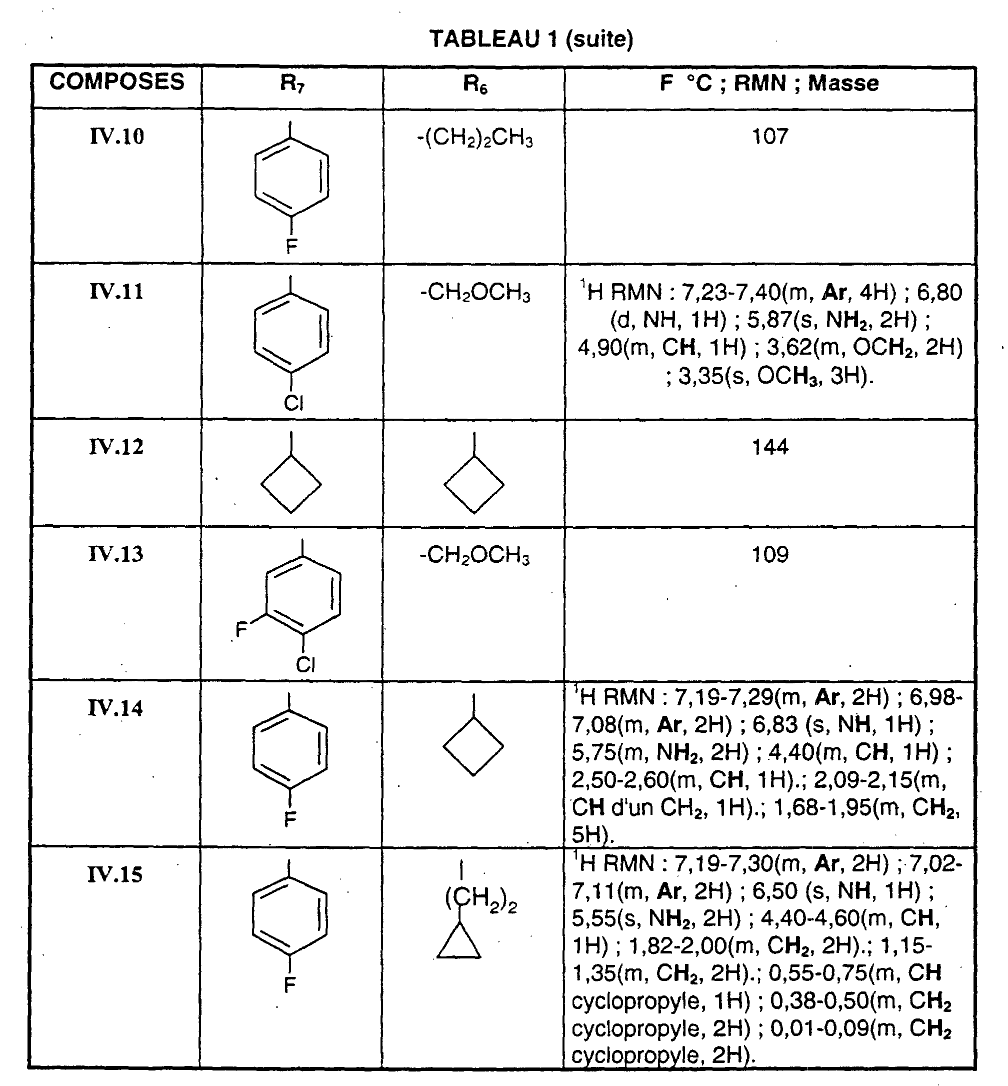

### PREPARATION DES THIAZOLES NH Composés II

### • [4-(2-chloro-4-méthoxy-5-méthylphényl)-5-méthylthiazol-2-yl]-[1-(4-méthoxyméthylphényl)butyl]-amine Composé II.1

A 1,4 g (5,54 mmol) de 1-(4-méthoxyméthylphényl)butylthiourée *(Composé IV.7*) dans 60 ml d'éthanol sont ajoutés 1,92 g (6 mmol) de 2-bromo-1-(2-chloro-4-méthoxy-5-méthylphényl)propan-1-one *(Composé III.1)* et 1,5 ml de triéthylamine. Le mélange réactionnel est agité à 85°C pendant trois heures puis est concentré sous pression réduite. Le résidu est repris par 100 ml de dichlorométhane et 50 ml d'eau. La phase organique est lavée à l'eau saturée de chlorure de sodium, séchée sur sulfate de sodium puis évaporée à sec sous vide. L'extrait brut est purifié par chromatographie sur colonne de gel de silice, éluant : cyclohexane/acétate d'éthyle 9/1 (v/v). On obtient 2,35 g d'aminothiazole. R = 96 %.
MS(MH⁺) = 445
¹H RMN : 7,26-7,36(m, **Ar**, 4H) ; 7,10(s, **Ar**, 1H) ; 6,83(s, **Ar**, 1H) ; 5,44-5,47(m, N**H**, 1H) ; 4,43(s, OC**H**_{**2**}, 2H) ; 4,17-4,33(m, C**H**, 1H) ; 3,81 (s, OC**H**_{**3**}, 3H) ; 3,39(s, OC**H**_{**3**}, 3H) ; 2,14(s, C**H**_{**3**}, 3H) ; 2,05(s, C**H**_{**3**}, 3H) ; 1,63-1,88(m, C**H**_{**2**}, 2H) ; 1,23-1,48(m, C**H**_{**2**}, 2H) ; 0,90(t, C**H**_{**3**}, 3H).

De la même façon ont été préparés les produits suivants décrits dans le TABLEAU 2 :

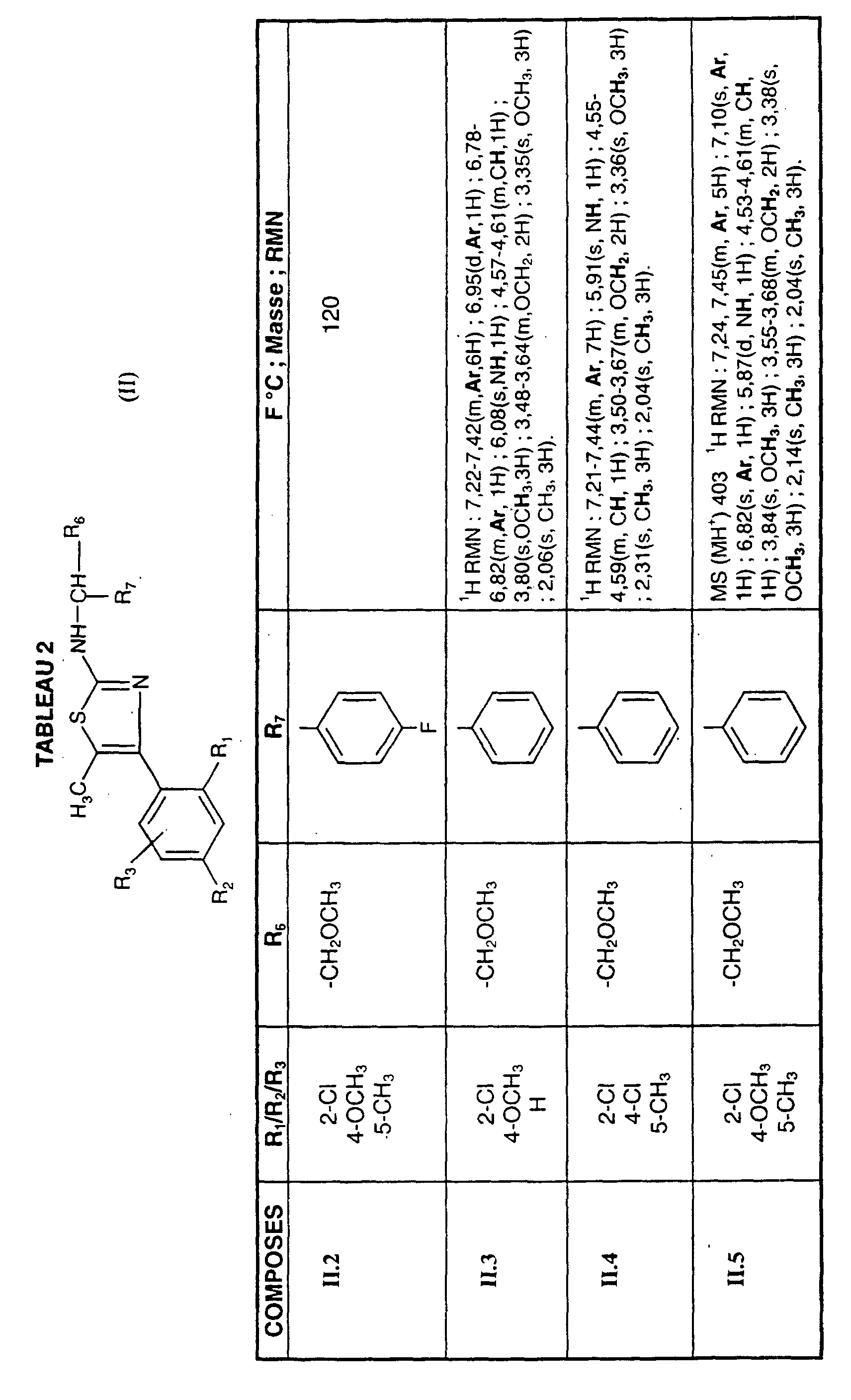

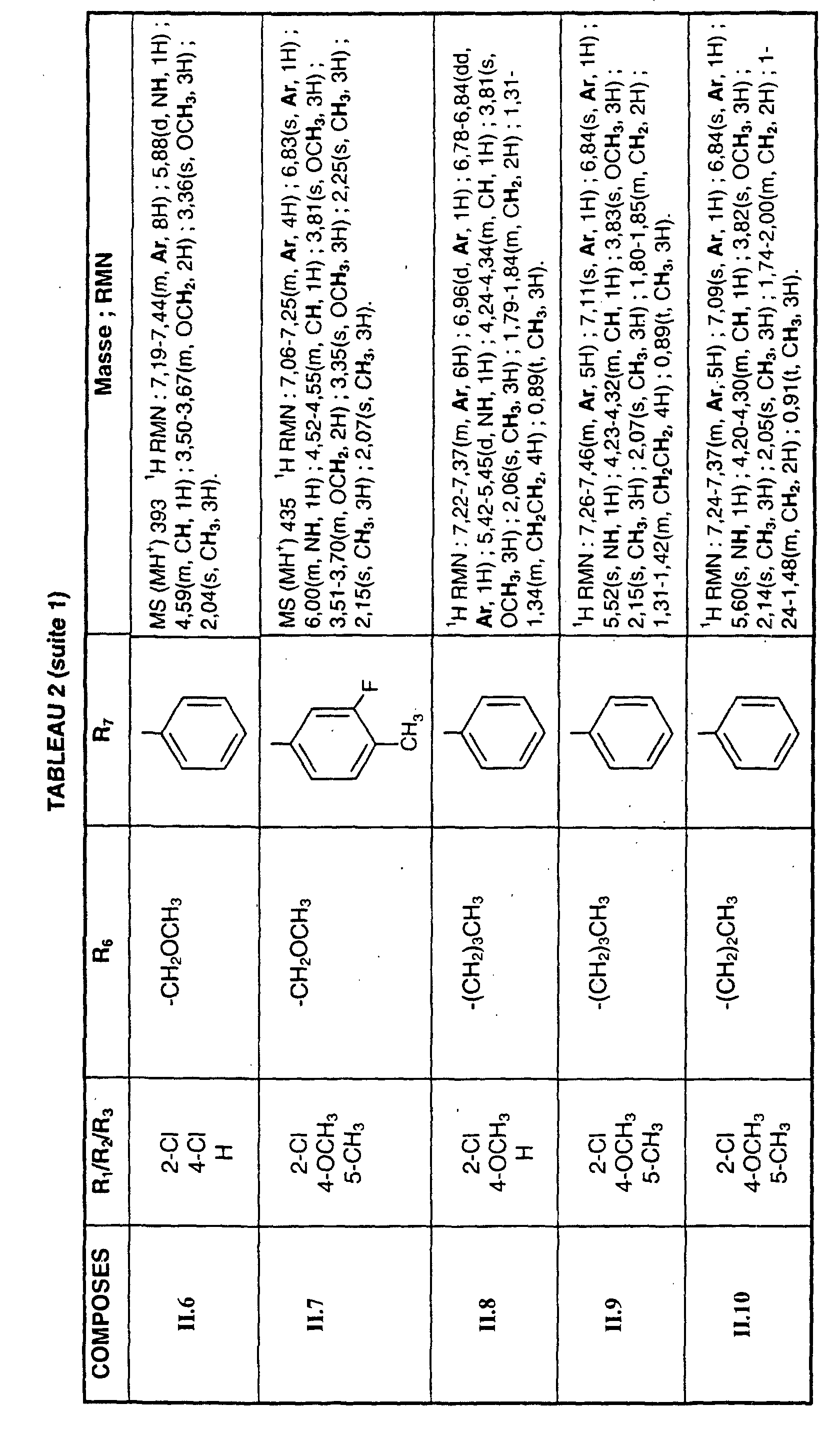

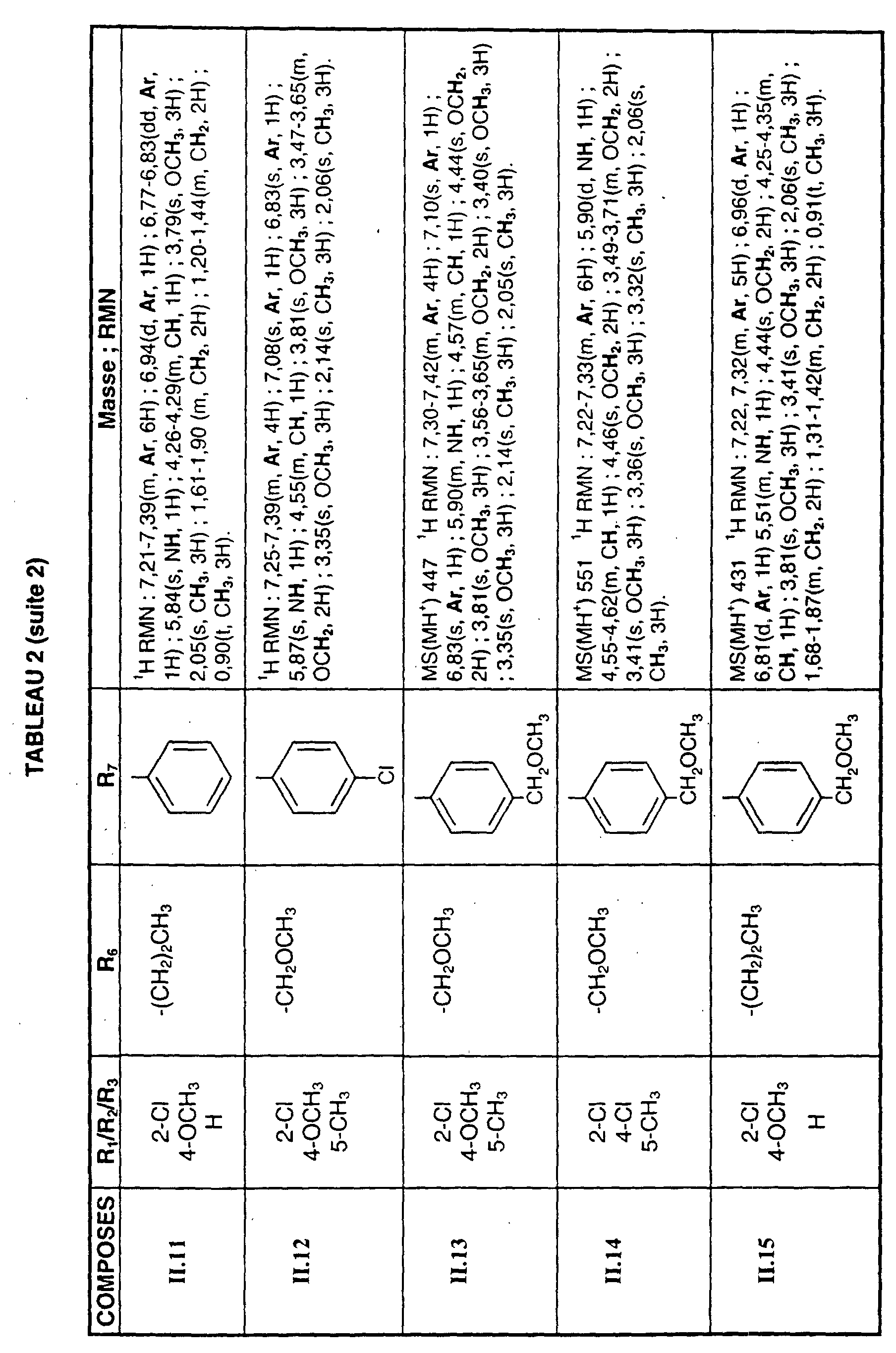

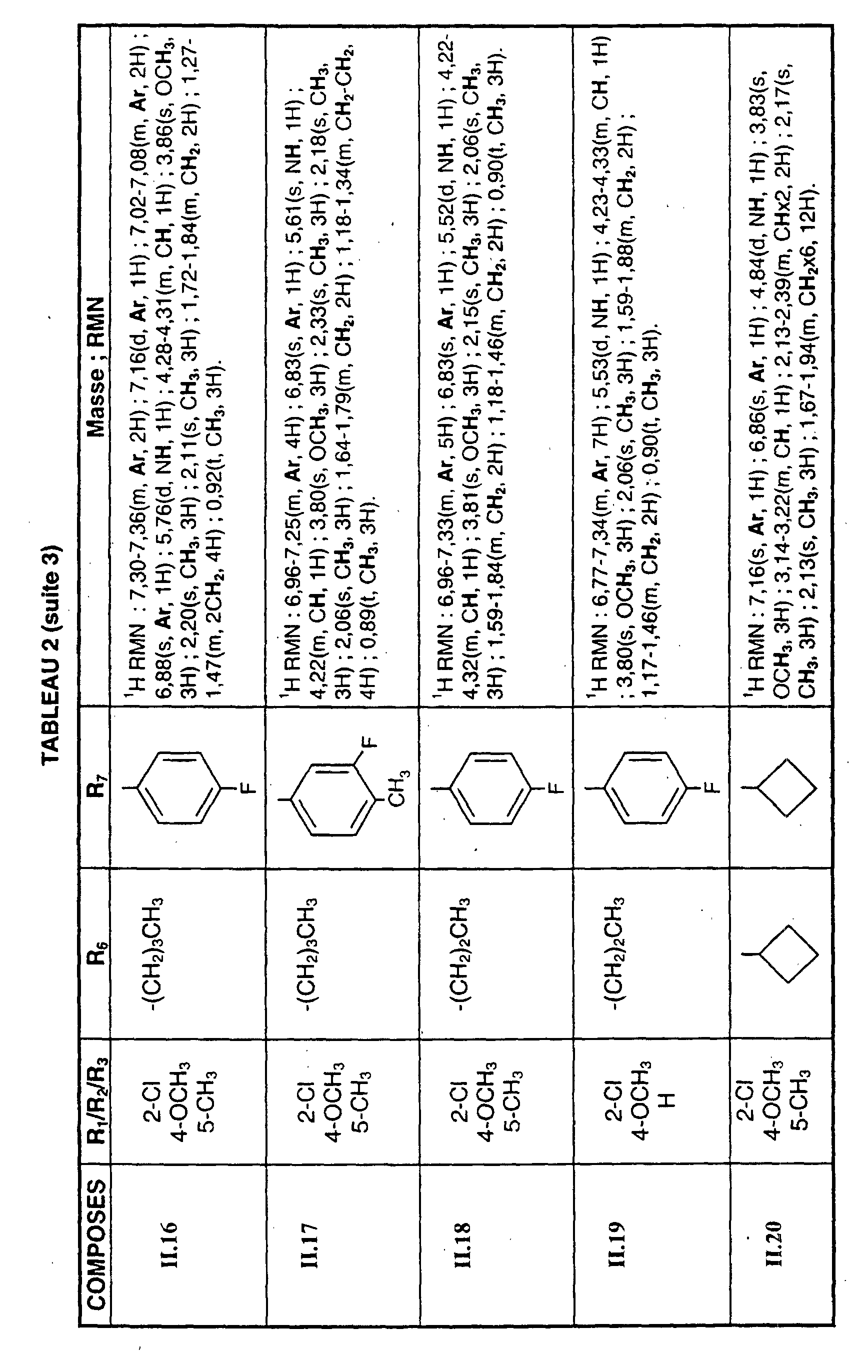

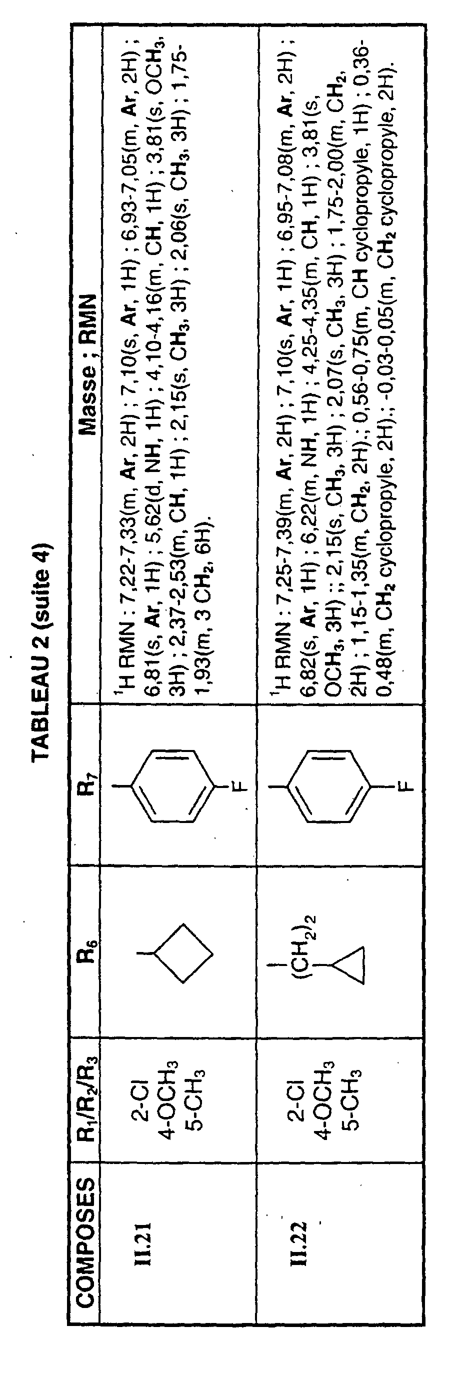

### PREPARATION DES THIAZOLES N SUBSTITUES Composés 1

### EXEMPLE 1

### [4-(2-chloro-4-méthoxy-5-méthylphényl)-5-méthylthiazol-2-yl]-[1-(4-fluorophényl)-2-méthoxyéthyl)prop-2-ynyl-amine Composé I.1

A 500 mg (1,2 mmol) du *Composé II.2*. dans 6 ml de diméthylformamide anhydre, sont ajoutés sous agitation et à 0°C, 50 mg d'hydrure de sodium à 60 % dans l'huile. Le mélange réactionnel est agité pendant vingt minutes à 0°C puis additionné de 0,22 ml (2 mmol) d'une solution de bromure de propargyle à 80% dans le toluène. Le mélange réactionnel est agité pendant une heure à 10°C puis est additionné 0,5 ml d'éthanol puis 10 ml d'eau. Le mélange est extrait par deux fois 50 ml d'acétate d'éthyle. La phase organique est lavée à l'eau, puis à l'eau saturée de chlorure de sodium, séchée sur sulfate de sodium anhydre puis évaporée à sec. Le résidu brut est chromatographié sur colonne de gel de silice [éluant cyclohexane/acétate d'éthyle 9/1 (v/v)]. On obtient 400 mg du composé attendu pur. R = 73 % ; Chlorhydrate hémihydraté : F = 94°C

De la même façon ont été préparés les produits suivants décrits dans le TABLEAU 3 :

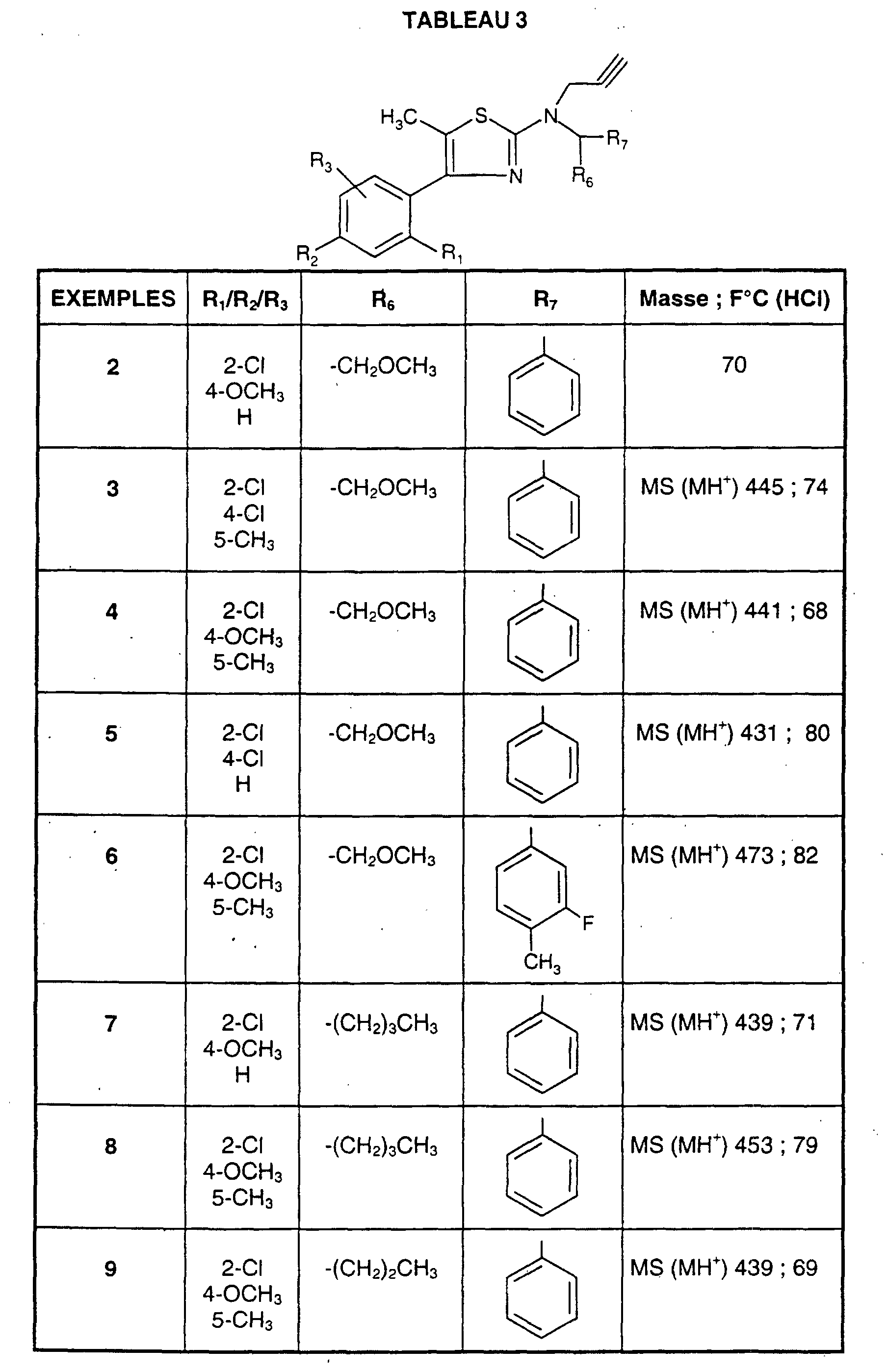

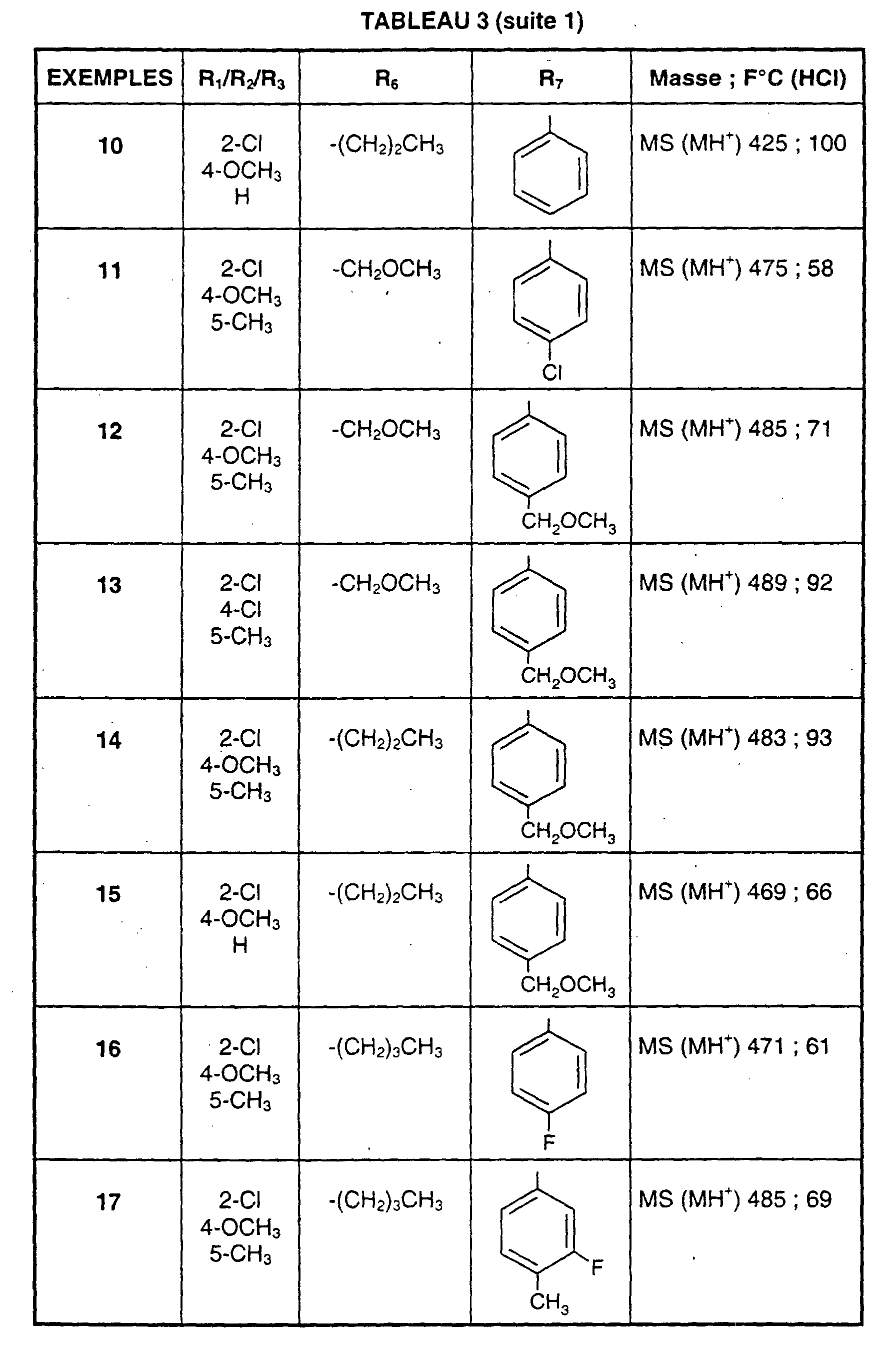

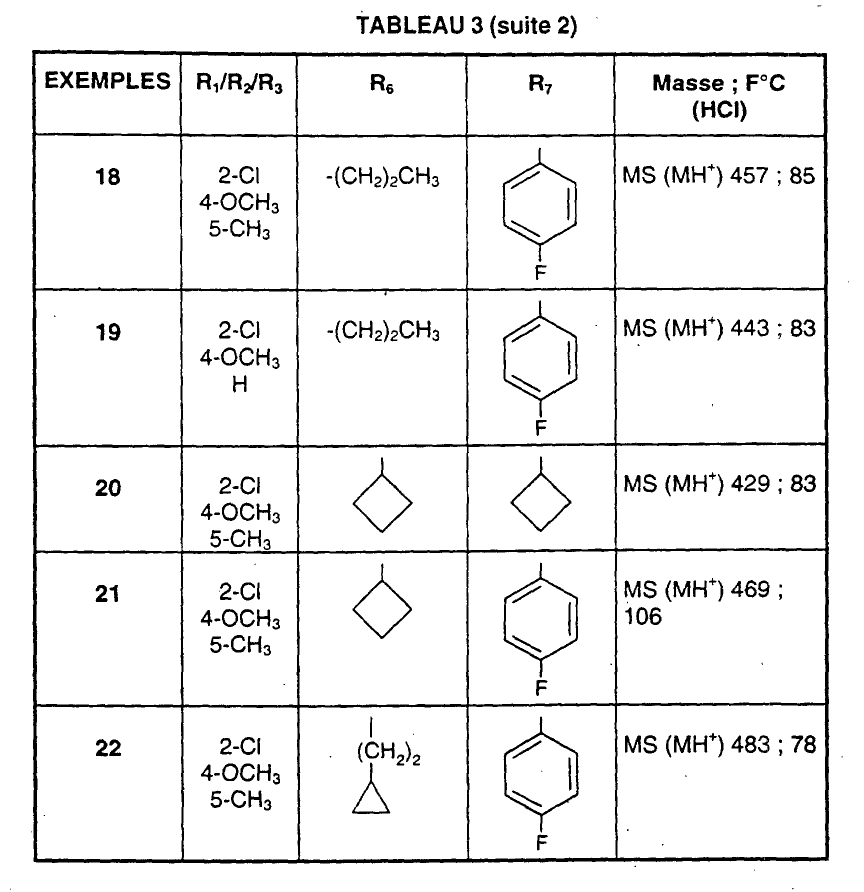

### PREPARATION DES AMINES SOUS FORME D'UN ENANTIOMERE Composé VI'

### Première Méthode

### a) (R)-2-amino-2-(4-fluorophényl)éthanol Composé 1'.1

240 ml (240 mmol) d'une solution 1 M d'hydrure d'aluminium-lithium dans le tétrahydrofurane sont agités à reflux puis additionnés par portions de 20 g (118 mmol) de (R)-(4-fluorophényl)glycine. Après agitation à reflux pendant six heures trente minutes, le mélange réactionnel est agité à 0°C puis additionné lentement de 9,5 ml d'eau, 9,5 ml d'une solution d'hydroxyde de sodium à 15 % puis 28,5 ml d'eau. La suspension obtenue est filtrée sur célite. Le filtrat est concentré et repris par 1 litre de dichlorométhane. La solution est lavée avec une solution saturée en chlorure de sodium, séchée sur du sulfate de sodium anhydre puis évaporée à sec. Une cristallisation dans l'éther isopropylique permet d'obtenir 13,22 g (85,2 mmol) de produit cristallisé, R = 72 %, F = 95°C ; MS (MH⁺) : 156.
¹H RMN (DMSO-D₆) : 7,30-7,41 (m, **Ar**, AH) ; 7,01-7,13(m, **Ar**, 2H) ; 4,73(s, **OH**, 1H) ; 3,84(m, **CH**, 1H) ; 3,35-3,45(m, C**H**₂O, 2H) ; 1,82(s, **NH**_{**2**}, 2H).

### b) (R)-1-(4-fluorophényl)-2-méthoxyéthylamine Composé VI'.1

3,64 g (91 mmol) d'hydrure de potassium obtenus par lavage au pentane de 8,1 g d'une suspension huileuse, sont mis en suspension dans 70 ml de tétrahydrofurane et agités à 10°C. Une solution de 13,22 g (85 mmol) de *composé 1'.1* dans 175 ml de tétrahydrofurane est additionnée lentement. Après agitation pendant seize heures à température ambiante, une solution de 5,2 ml (83,5 mmol) d'iodométhane dans 105 ml de tétrahydrofurane est additionnée en deux heures. Le mélange réactionnel est agité pendant trois heures à température ambiante puis est versé sur 1 litre d'eau glacée salée. Le mélange est extrait par 1 litre de *ter*-butyl-méthyloxyde. La phase organique est lavée à l'eau, puis avec une solution saturée en chlorure de sodium, séchée sur sulfate de sodium anhydre puis évaporée à sec. On obtient 11,87 g (70 mmol) d'amine huileuse. R = 82 %.
¹H RMN : 7,24-7,38(m, **Ar**, 2H) ; 6,93-7,05(m, **Ar**, 2H) ; 4,16(m, C**H**, 1H) ; 3,45(dd, C**H**_{**2**}, 1 H) ; 3,36(s, OC**H**_{**3**}, 3H) ; 3,29(d, C**H**_{**2**}, 1 H) 1,66(s, N**H**_{**2**}, 2H).

De la même façon, à partir de la (R)-phénylglycine, on obtient :
- **(R)-2-méthoxy-1-phényléthylamine *Composé VI'.2***

### Deuxième méthode

### a) (S)-2-amino-3-méthyl-1,1-diphénylbutan-1-ol Composé 2'.1

Une solution de 600 ml de bromure de phényl-magnésium 3,0 M (1790 mmol) dans l'éther diéthylique est agitée à 0°C, diluée par 300 ml de THF puis additionnée par portions 50 g (298 mmol) de chlorhydrate de l'ester méthylique de L-Valine en maintenant la température en dessous de 10°C. Après agitation pendant trois heures à température ambiante, le mélange réactionnel est versé lentement sur une solution glacée de chlorure d'ammonium. Le mélange additionné de 500 ml d'éther diéthylique et 500 ml d'acétate d'éthyle est agité pendant une nuit à température ambiante. Après décantation et séparation des phases, la phase aqueuse est réextraite par 1 L de TBME (ter-butyl-méthyl-éther). Les phases organiques réunies sont agitées à 0°C et acidifiées lentement par environ 40 ml d'acide chlorhydrique à 35% dans l'eau. Le précipité de chlorhydrate ainsi formé est filtré et rincé par du TMBE. Il est ensuite repris par 1 L de dichlorométhane et 1 L d'eau et le mélange est alcalinisé à 0°C par environ 50 ml de lessive de soude à 35 %. Après décantation et séparation des phases, la phase aqueuse est réextraite par 1 L de dichlorométhane. Les phases organiques réunies sont lavées à l'eau puis à l'eau salée, séchées sur sulfate de sodium et concentrées. Après cristallisation dans l'éther isopropylique on obtient 61 g du *Composé 2'.1.* (R = 87 %)
[ α_{D}]²⁰_{D} = - 127,8° (CHCl₃ c = 0,639)
¹H RMN : 7,00-7,60(**Ar**, m, 10H) ; 5,24(-O**H**, s, 1H) ; 3,66(-C**H**-N, d, J=1,5, 1H) ; 1,53(-C**H**-, hept d, J=1,5 et 7, 1H) ; 1,16(-N**H**_{**2**}, s, 2H) ; 0,81 (-C**H**_{**3**}, 2d, J = 7, 6H).

De la même façon, à partir du chlorhydrate de l'ester méthylique de la D-Valine, on obtient
- **(R)-2-amino-3-méthyl-1,1-diphénylbutan-1-ol *Composé 2'.2***

Ces composés sont utilisés comme auxillaire chiral lors de la réduction énantiosélective des O-benzyl-oximes 6'.

### b) Synthèse de phénylcétones substituées. Composés 3'

### Mode opératoire A

### • 2-Cyclopropyl-1-(3-fluoro-4-méthylphényl)éthan-1-one Composé 3'.1

10,2 g (418 mmol) de tournure de magnésium sont additionnés de 50ml d'éther diéthylique et d'un cristal d'iode et agités à température ambiante. Une solution de 75,35 g (398 mmol) de 4-bromo-2-fluoro-toluène dans 370 ml d'éther diéthylique est additionnée pendant trois heures de façon à maintenir un reflux modéré. Le mélange réactionnel est ensuite chauffé à reflux pendant une heure trente puis après refroidissement est filtré sur laine de verre. La solution obtenue est additionnée lentement sur une solution de 32,3 g (398 mmol) de cyclopropylacétonitrile dans 230 ml d'éther diéthylique agitée à 0°C. Le mélange réactionnel est agité pendant une nuit à température ambiante. Il est ensuite agitée à 0°C et additionnée lentement de 200 ml d'acide chlorhydrique 2N. Après séparation de la phase éthérée, la phase aqueuse acide est extaite par de l'acétate d'éthyle. Les phases organiques réunies sont lavées avec de l'eau puis avec une solution saturée en chlorure de sodium, séchées sur sulfate de sodium anhydre puis évaporées à sec. L'extrait brut est purifié par chromatographie sur gel de silice (solvant d'élution cyclohexane-acétate d'éthyle 20/1). On obtient 53,3 g de cétone 3'-1 (R = 70 %)
¹H RMN : 7,54-7,64(m, 2H, **Ar**) ; 7,22-7,30(m, 1 H, **Ar**) ; 2,82(d, J = 6,7 Hz, 2H, C**H**_{**2**}) ;2,31 (s, 3H, C**H**_{**3**}) ; 1,07-1,20(m, 1 H, C**H** cyclopropyle) ; 0,55-0,65(m, 2H, C**H**_{**2**} cyclopropyle) ; 0,15-0,21 (m, 2H, C**H**_{**2**} cyclopropyle) .

Par le même procédé les cétones suivantes ont été synthétisées :
- **1-(4-éthylphényl)-2-méthoxyéthan-1-one *Composé 3'.2***
- **2-cyclopropyl-1-(4-méthylphényl)éthan-1-one *Composé 3'.3***
- **2-cyclobutyl-1-(4-fluorophényl)éthan-1-one *Composé 3'.4***

### Mode opératoire B

Méthode décrite pour le *Composé 3.5* (réaction d'un phényl-lithien sur une amide de WEINREB) :
- **2-méthoxy-1-(3,4-méthylènedioxyphényl)éthan-1-one *Composé 3'.5***
- **1-(4-méthoxyméthylphényl)pentan-1-one *Composé 3'.6***
- **1-(3-fluoro-4-méthylphényl)butan-1-one *Composé 3'.7***
- **1-(3-fluoro-4-méthylphényl)pentan-1-one *Composé 3'.8***
- **1-(3-fluoro-4-méthoxyméthylphényl)butan-1-one *Composé 3'.9***
- **2-cyclopropyl-1-(3,4-méthylènedioxyphényl)éthan-1-one *Composé 3'.10***
- **1-(3,4-méthylènedioxyphényl)butan-1-one *Composé 3'.11***

### c) Synthèse des O-benzyl-oximes. Composés 6'

Les O-benzyl-oximes sont préparées par O-benzylation des oximes correspondantes selon le procédé suivant (les oximes de départ sont obtenus à partir des cétones par une des deux méthodes de synthèse décrites précédemment pour le *Composé 4.1*).

### • 1-(3-fluoro-4-méthylphényl)-2-méthoxyéthan-1-one O-benzyloxime isomère Z Composé 6 '.1

Une solution de 42,5 g (217 mmol) de 1-(3-fluoro-4-méthyl-phényl)-2-méthoxyéthan-1-one oxime Z (*Composé 4.1*) dans 100 ml de diméthylformamide est agitée à 0°C et additionnée par petites quantités de 15,6 g (325 mmol, 1,5 éq.) d'hydrure de sodium à 50 % dans l'huile. Le mélange réactionnel est agité pendant quinze minutes puis est additionné lentement d'une solution contenant 30 ml (280 mmol, 1,3 éq.) de bromure de benzyle dans 100 ml de diméthylformamide. Le mélange réactionnel est agité pendant deux heures à température ambiante, puis est refroidi à 0°C et additionné de 5 ml d'éthanol puis 50 ml d'eau. On extrait à l'acétate d'éthyle. La phase organique est lavée à l'eau puis avec une solution saturée de chlorure de sodium. Elle est ensuite sèchée sur sulfate de sodium et évaporée à sec. L'huile ainsi obtenue est purifiée par chromatographie sur gel de sicile (éluant cyclohexane/dichlorométhane 7/3 (v/v)). On obtient 39 g de *Composé 6'.1* (Z) ; R = 63 %.
¹H RMN : 7,10-7,50 (**Ar**, m 8H) ; 5,22(-O-**CH**_{**2**}-Ph, s, 2H) ; 4,58(-C**H**_{**2**}-O, s, 2H) ; 3,28(OC**H**_{**3**}, s, 3H) ; 2,26(C**H**_{**3**}-Ph, d, J = 1,8, 3H).

On prépare de la même façon les composés suivants :
- **1-(4-chloro-3-fluoro-phényl)-2-méthoxyéthan-1-one *O*-benzyloxime (Z) *Composé 6'.2***
- **1-(4-chlorophényl)-2-méthoxyéthan-1 -one *O*-benzyloxime (Z) *Composé 6'.3***
- **2-méthoxy-1-(3,4-méthylènedioxyphényl)éthan-1-one *O*-benzyloxime (Z)** *Composé* ***6'.4***
- **1-(4-éthylphényl)-2-méthoxyéthan-1-one *O*-benzyloxime (Z) *Composé 6'.5***
- **2-méthoxy-1-(4-méthoxyméthylphényl)éthan-1-one *O*-benzyloxime (Z) *Composé 6'.6***
- **1-phénylbutan-1-one *O*-benzyloxime (E) *Composé 6'.7***
- **1-(4-méthoxyméthylphényl)butan-1-one *O*-benzyloxime (E) *Composé 6'.8***
- **1-(4-méthoxyméthylphényl)pentan-1-one *O*-benzyloxime (E) *Composé 6'.9***
- **2-cyclopropyl-1-phényléthan-1-one *O*-benzyloxime (E) *Composé 6'.10***
- **2-cyclopropyl-1-(4-fluorophényl)éthan-1-one *O*-benzyloxime (E) *Composé 6'.11***
- **1-(4-fluorophényl)pentan-1-one *O*-benzyloxime (E) *Composé 6'.12***
- **cyclopropyl-phényl-cétone *O*-benzyloxime (E) *Composé 6'.13***
- **1-(3-fluoro-4-méthylphényl)butan-1-one *O*-benzyloxime (E) *Composé 6'.14***
- **1-(3-fluoro-4-méthylphényl)pentan-1-one *O*-benzyloxime (E) *Composé 6'.15***
- **2-cyclopropyl-1-(3-fluoro-4-méthylphényl)éthan-1-one *O*-benzyloxime (E) *Composé 6'.16***
- **1-(4-fluorophényl)butan-1-one *O*-benzyloxime (E) *Composé 6'.17***
- **1-(3-fluoro-4-méthoxyméthylphényl)butan-1-one *O*-benzyloxime (E) *Composé 6'.18***
- **2-cyclopropyl-1-(4-chlorophényl)éthan-1-one *O*-benzyloxime (E) *Composé 6'.19***
- **2-cyclopropyl-1-(4-méthylphényl)éthan-1-one *O*-benzyloxime (E) *Composé 6'.20***
- **2-cyclobutyl-1-(4-fluorophényl)éthan-1-one *O*-benzyloxime (E) *Composé 6'.21***
- **2-cyclopropyl-1-(4-bromophényl)éthan-1-one *O*-benzyloxime (E) *Composé 6'.22***
- **2-cyclopropyl-1-(3,4-méthylènedioxyphényl)éthan-1-one *O*-benzyloxime (E) *Composé 6'.23***
- **1-(3,4-méthylènedioxyphényl)butan-1-one *O*-benzyloxime (E) *Composé 6'.24***

### d) Synthèse des amines énantiomères

### •(R)-1-(3-fluoro-4-méthylphényl)-2-méthoxyéthylamine Composé VI'.3

Une solution de 86,5.g du *Composé 2'.1* (330 mmol..) dans 600 ml de tétrahydrofurane est agitée à une température inférieure à 30°C, puis est additionnée lentement de 670 ml d'une solution 1 M de borane-tétrahydrofurane (670 mmol.). On laisse la température remonter à l'ambiante pendant deux heures. Le milieu réactionnel est ensuite agité à 0°C et additionné de 39 g (132 mmol) de *Composé 6'.1* préalablement dissout dans 100 ml de tétrahydrofurane. Après agitation vingt heures à température ambiante, le mélange réactionnel est refroidi à 0°C et additionné de 1 litre d'acide chlorhydrique 2N. On laisse pendant seize heures sous agitation. Le mélange est alcalinisé à 0°C par addition d'hydroxyde de sodium à 35 % puis extrait à l'acétate d'éthyle. Cet extrait est lavé à l'eau, l'eau saturée en chlorure de sodium puis séché sur sulfate de sodium et évaporé à sec. Le résidu obtenu est chromatographié sur colonne de gel de silice (éluant dichlorométhane/méthanol, 95/5(v/v)). On obtient 17 g de *Composé VI'.3* ; R = 79 %.
¹H RMN : 6,90-7,20(m, **Ar**, 3H) ; 4,14(dd, J₁=4 Hz, J₂=8,5 Hz, CHN, 1 H) ; 3,47(dd, J₁=4 Hz, J₂=9 Hz, -C**H**_{**2**}-O, 1 H) ; 3,37(s, OC**H**_{**3**}, 3H) ; 3,32(dd, J₁=8,5 Hz, J₂=9 Hz, -C**H**_{**2**}-O, 1 H) ; 2,24(d, J=1,8Hz, C**H**_{**3**}-Ph, 3H) ; 1,68(s, -N**H**_{**2**}, 2H).
HPLC chirale : % Enantiomères : R = 99,5 % S = 0,5 % ee = 99,0 %

Remarque générale: Les excès énantiomériques (ee) sont évalués à partir des chromatogrammes (HPLC ou SFC chiral) de ces amines ou des thiourées IV' correspondantes.

De la même façon on obtient :
- **(R)-1-(4-chloro-3-fluorophényl)-2-méthoxyéthylamine *Composé VI'.4*** ee = 98,2 %
- **(R)-1-(4-chlorophényl)-2-méthoxyéthylamine *Composé VI'.5*** ee = 98,6 %
- **(R)-2-méthoxy-1-(3,4-méthylènedioxyphényl)éthylamine *ComposéVI'.6*** ee > 99%
- **(R)-1-(4-éthylphényl)-2-méthoxyéthylamine *Composé VI'.7*** ee > 99%
- **(R)-2-méthoxy-1-(4-méthoxyméthylphényl)éthylamine *Composé VI'.8*** ee> 99%
- **(S)-(1-phényl)butylamine *Composé VI'.9*** ee = 97,1 %
- **(S)-1-(4-méthoxyméthylphényl)butylamine *Composé VI'.10*** ee = 97,1 %
- **(S)-1-(4-méthoxyméthylphényl)pentylamine *Composé VI'.11*** ee = 96,8 %
- **(S)-2-cyclopropyl-1-phényléthylamine *Composé VI'.12*** ee = 95,8 %
- **(S)-2-cyclopropyl-1-(4-fluorophényl)éthylamine *Composé VI'.13*** ee = 95,4 %
- **(S)-1-(4-fluorophényl)pentylamine *Composé VI'.14***
- **(S)-cyclopropyl-phényl-méthylamine *Composé VI'.15*** ee = 90 %
- **(S)-1-(3-fluoro-4-méthylphényl)butylamine *Composé VI'.16*** ee > 99 %
- **(S)-1-(3-fluoro-4-méthylphényl)pentylamine *Composé VI'.17*** ee = 97 %
- **(S)-2-cyclopropyl-1-(3-fluoro-4-méthylphényl)éthylamine *Composé VI'.18*** ee > 99 %
- **(S)-1-(4-fluorophényl)butylamine *Composé VI'.19*** ee = 98,4 %
- **(S)-1-(3-fluoro-4-méthoxyméthylphényl)butylamine *Composé VI'.20*** ee = 90,5 %
- **(S)-2-cyclopropyl-1-(4-chlorophényl)éthylamine *Composé VI'.21*** ee > 99 %
- **(S)-2-cyclopropyl-1-(4-méthylphényl)éthylamine *Composé VI'.22*** ee =85,6 %
- **(S)-2-cyclobutyl-1-(4-fluorophényl)éthylamine *Composé VI'.23*** ee = 98,5 %
- **(S)-2-cyclopropyl-1-(4-bromophényl)éthylamine *Composé VI'.24*** ee = 98,3 %
- **(S)-2-cyclopropyl-1-(3,4-méthylènedioxyphényl)éthylamine *Composé VI'.25*** ee = 96,7%
- **(S)-1-(3,4-méthylènedioxyphényl)butylamine *Composé VI'.26*** ee = 84 %

### Troisième Méthode

Pour améliorer l'excès énantiomèrique, on peut traiter les amines ci-dessus par des acides organiques sous forme d'énantiomères purs (par exemple la N-acétyl-L-Leucine) et recristallisation :

### • (S)-(1-phényl)butylamine Composé VI'.9

Salification avec la N-acétyl-L-Leucine :

Une solution de 10,4 g (60 mmol) de N-acétyl-L-Leucine dans 70 ml de méthanol anhydre est agitée à 60°C, puis est additionnée goutte à goutte d'une solution de 9,0 g (60 mmol) de (S)-(1phényl)butylamine *Composé VI'.9* (ee 97,1 %) dans 30 ml de méthanol anhydre. A la fin de l'addition, la solution méthanolique est portée à ébullition (solubilisation totale) puis laissée au repos une nuit. Après filtration et rinçage avec 20 ml de méthanol anhydre froid, on récupère 7,7 g de cristaux qui sont dissous dans un minimum d'eau. Après alcalinisation par de l'hydroxyde de sodium 1 N et extraction au dichlorométhane, la phase organique est lavée avec une solution saturée en chlorure de sodium, séchée sur sulfate de sodium et évaporée sous vide. On obtient 3,4 g d'amine sous forme d'huile.
¹H RMN : 7,16-7,36(m, **Ar**, 5H) ; 3,87(m, -C**H**-N, 1H) ; 1,57-1,69(m, -CH-C**H**_{**2**}, 2H) ; 1,47(s, N**H**₂, 2H) ; 1,15-1,40(m, -C**H**₂CH₃, 2H) ; 0,88(t, -CH₂C**H**_{**3**}, 3H).
HPLC chirale : % Enantiomères : S = 100 % R = 0 % ee = 100 %
[α]_{D}²⁵ = - 22,0° (c = 1,05, CHCl₃)

### PREPARATION DES THIOUREES SOUS FORME D'UN ENANTIOMERE

### Composé IV'

### Première méthode

### • N[(R)-1-(4-fluorophényl)-2-méthoxyéthyl]thiourée Composé IV'.1

A une solution de 2,83 g (37,2 mmol) d'isothiocyanate d'ammonium dans 75 ml d'acétone agitée à 0°C sont additionnés 4,23 ml (36,6 mmol) de chlorure de benzoyle. Après trente minutes, 6 g (35,5 mmol) de *Composé VI'1* en solution dans 75 ml d'acétone sont ajoutés lentement. Le mélange réactionnel est agité pendant deux heures à température ambiante puis concentré sous pression réduite. La suspension est reprise par 200 ml de *ter*-butylméthyloxyde et 200 ml d'eau. La phase organique est lavée à l'eau, puis avec une solution saturée en chlorure de sodium, séchée sur sulfate de sodium anhydre puis évaporée à sec. Le résidu d'évaporation est dissout dans 180 ml d'éthanol et la solution obtenue est additionnée de 3,75 ml (75 mmol) d'hydrazine mono-hydratée. Après agitation pendant vingt-quatre heures à température ambiante, le mélange réactionnel est évaporé. Le résidu d'évaporation est dissout par 200 ml d'acétate d'éthyle et la phase organique est lavée à l'eau puis avec une solution saturée en chlorure de sodium, séchée sur sulfate de sodium anhydre et évaporée à sec. Le résidu d'évaporation est chromatographié sur une colonne de gel de silice : éluant : cyclohexane/acétate d'éthyle 1/1, (v/v). On obtient 5 g (23 mmol) de produit solide blanc ; R = 63 % ; F = 119°C.
¹H RMN (DMSO D₆) : 8,10(d, NH, 1H) ; 7,28-7,32(m, **Ar**, 2H) ; 7,08-7,17(m, **Ar** et N**H**_{**2**}, 4H) ; 5,45(m, C**H**-N, 1 H) ; 3,54-3,62(m, CH-C**H**_{**2**}, 2H) ; 3,24(s, OC**H**_{**3**}, 3H).
[α]¹⁹D = + 32,0° (c = 0,87 CH₂Cl₂).

Chromatographie chirale supercritique ee = 100 %

De la même façon, on obtient les produits suivants :
- **N-[(R)-2-méthoxy-1-phényléthyl]thiourée *Composé IV'.2***
   F = 140°C [ α ]¹⁹_{D} = + 4,6°(c= 1,0 CH₂Cl₂).
- ***N*-[(R)-1-(4-chlorophényl)-2-méthoxyéthyl]thiourée *Composé IV'.3***
   F = 133°C [ α ]¹⁹_{D} = + 25,7°(c= 1,04 CH₂Cl₂).
- ***N*-[(R)-2-méthoxy-1-(3,4-méthylènedioxyphényl)éthyl]thiourée *Composé IV'.4***
   F = 160°C [ α ]¹⁹_{D} = + 19,4°(c= 0,68 CH₂Cl₂).
- ***N*-[(R)-1-(4-éthylphényl)-2-méthoxyéthyl]thiourée *Composé IV'.5***
   F =116°C [ α ]¹⁹_{D} = + 20,0°(c= 0,93 CH₂Cl₂).
- ***N*-[(S)-1-phénylbutyl]thiourée *Composé IV'.6***
   F = 140°C [ α ]¹⁹_{D} = + 48,7°(c= 0,82 CH₂Cl₂).
- **N-[(R)-2-méthoxy-1-(4-méthoxyméthylphényl)éthyl]thiourée *Composé IV'.7***
   ¹H RMN : 7,25-7,36(m, **Ar**, 4H) ; 6,85(m, N**H**, 1H) ; 5,93(m, N**H**_{**2**}, 2H) ; 4,78(m, C**H**-N, 1 H) ; 4,43(s, O-C**H**_{**2**}, 2H) ; 3,58-3,65(m, O-C**H**_{**2**}, 2H) ; 3,38(s, OC**H**_{**3**}, 3H) ; 3,35(s, O-C**H**_{**3**}, 3H).
   [ α ]¹⁹_{D} = + 20,5° (c = 0,95 CH₂Cl₂).
- **N-[(S)-1-(4-méthoxyméthylphényl)pentyl]thiourée *Composé IV'.8***
   ¹H RMN :7,22-7,35(m, **Ar**, 4H) ; 6,71 (m, N**H**, 1 H) ; 5,63(m, N**H**_{**2**}, 2H) ; 4,42(s, O-C**H**_{**2**}, 2H) ; 4,40(m, C**H**, 1H) ; 3,39(s, OC**H**_{**3**}, 3H) ; 1,68-1,79(m, C**H**_{**2**}, 2H) ; 1,14-1,30(m, C**H**_{**2**}-C**H**_{**2**}, 4H) ; 0,81-0,87(m, C**H**_{**3**}, 3H).
   [ α ]¹⁹_{D} = + 49,8° (c = 1,04 CH₂Cl₂).
- **N-[(S)-1-(4-méthoxyméthylphényl)butyl]thiourée *Composé IV'.9***
   ¹H RMN : 7,20-7,40(m, **Ar**, 4H) ; 6,69(m, NH, 1H) ; 5,63(m, N**H**_{**2**}, 2H) ; 4,41 (s, O-C**H**_{**2**}, 2H) ; 4,40(m, C**H**, 1 H) ; 3,39(s, OC**H**_{**3**}, 3H) ; 1,59-1,88(m, CH-C**H**_{**2**}-CH₂, 2H) ; 1,15-1,44(m, CH₂-C**H**_{**2**}-CH₃, 2H) ; 0,85-0,92(m, CH₂-C**H**_{**3**}, 3H).
   [ α ]¹⁹_{D} = + 43,9° (c = 1,17 CH₂Cl₂).
- **N-[(S)-2-cyclopropyl-1-phényléthyl]thiourée *Composé IV'.10***
   F = 80°C [ α ]¹⁹_{D} = + 55,0° (c = 0,97 CH₂Cl₂) ; ee = 95,8%
- **N-[(R)-1-(3-fluoro-4-méthylphényl)-2-méthoxyéthyl]thiourée *Composé IV'.11***
   F = 149°C [ α ]²⁰_{D} = + 30,3° (c = 0,97 CH₂Cl₂).
- **N-[(R)-1-(3-fluoro-4-chlorophényl)-2-méthoxyéthyl]thiourée *Composé IV'.12***
   F = 110°C [α]²⁰_{D} = + 29,1° (c = 1,04 CH₂Cl₂).
- **N-[(S)-1-(4-fluorophényl)pentyl]thiourée *Composé IV'.13***
   F = 118°C [ α ]²⁰_{D} = -19,2° (c = 0,78 méthanol)
- **N-[(S)-cyclopropyl-phényl-méthyl]thiourée *Composé IV'.14***
   ¹H RMN : 7,25-7,41 (m, **Ar**, 5H) ; 6,92(m, NH, 1 H) ; 5,58(m, N**H**_{**2**}, 2H) ; 3,92(m, C**H**, 1 H) ; 1,08-1,25(m, C**H** cyclopropyle, 1 H) ; 0,35-0,69(m, 2C**H**_{**2**} cyclopropyle, 4H).
   [ α ]²⁰_{D} = + 33,5° (c = 0,48 méthanol); ee = 90%
- **N-[(S)-1-(3-fluoro-4-méthylphényl)butyl]thiourée *Composé IV'.15***
   F = 129°C [ α ]²⁰_{D} = 44,4° (c = 0,81 CH₂Cl₂) ; ee = 99%
- **N-[(S)-1-(3-fluoro-4-méthylphényl)pentyl]thiourée *Composé IV'.16***
   F = 124°C [ α ]²⁰_{D} = + 4,6° (c = 1,4 CH₂Cl₂) ; ee = 97%
- **N-[(S)-2-cyclopropyl-1-(3-fluoro-4-méthylphényl)éthyl]thiourée *Composé IV'.17***
   F = 91 °C [ α ]²²_{D} = + 55,4° (c = 0,9 CH₂Cl₂) ; ee = 99%
- **N-[(S)-1-(4-fluorophényl)butyl]thlourée *Composé IV'.18***
   ¹H RMN : 7,21-7,28(m, **Ar**, 2H) ; 6,99-7,09(m, **Ar**, 2H) ; 6,75(s, NH, 1H) ; 5,71 (s, N**H**_{**2**}, 2H) ; 4,35-4,60(m, C**H**, 1H) ; 1,65-1,85(m, C**H**_{**2**}, 2H) ; 1,18-1,45(m, C**H**_{**2**}, 2H) 0,86-0,93(m, C**H**_{**3**}, 3H).
   [ α ]²²_{D} = + 49° (c = 0,95 CH₂Cl₂) ; ee = 98,4%.
- **N-[(S)-2-cyclopropyl-1-(4-chlorophényl)éthyl]thiourée *Composé IV'.19***
   F = 93,7°C [ α ]²³_{D} = + 53° (c = 0,5 CH₂Cl₂) ; ee = 99,1 %.
- **N-[(S)-2-cyclobutyl-1-(4-fluorophényl)éthyl]thiourée *Composé IV'.20***
   F = 104°C [ α ]²⁰_{D} = -21 ° (c = 1 méthanol) ; ee = 98,5%
- **N-[(S)-2-cyclopropyl-1-(4-bromophényl)éthyl]thiourée *Composé IV'.21***
   F = 130°C [ α ]²⁰_{D} = + 57° (c = 0,67 CH₂Cl₂) ; ee = 98,3%.
- **N-[(S)-2-cyclopropyl-1-(3,4-méthylènedioxyphényl)éthyl]thiourée *Composé IV'.22***
   F = 125°C [ α ]¹⁹_{D} = + 63° (c = 0,75 CH₂Cl₂); ee = 96,7%.

### Deuxième Méthode

a) Obtention par chromatographie de thiourées sous forme d'énantiomère (ee > 99%) à partir de thiourées enrichies en un énantiomère :
   - **N-[(S)-2-cyclopropyl-1-phényléthyl]thiourée *Composé IV'.10***
      A partir d'un mélange majoritaire en énantiomère S (ee 95,8%) , on obtient après séparation par chromatographie sur phase CHIRACEL OJ éluant isohexane/éthanol 97/3, l'énantiomère S pur (ee 100 %)
      F = 84° [ α ]¹⁹_{D} = + 59,3° (c = 1,06 CH₂Cl₂).
   - **N-[(S)-2-cyclopropyl-1-(4-fluorophényl)éthyl]thiourée *Composé IV'.23***
      F = 105° [ α ]²²_{D} = + 61,0° (c = 0,53 CH₂Cl₂) ; ee = 100%.
   - **N-[(S)-1-(3-fluoro-4-méthoxyméthylphényl)butyl]thiourée *Composé IV'.24***
      ¹H RMN : 7,39-7,46(m, **Ar**, 1 H) ; 6,93-7,07(m, **Ar**, 2H et NH, 1H) ; 5,85(m, N**H**_{**2**}, 2H) ; 4,45(s, O-C**H**_{**2**}, 2H) ; 4,35(m, C**H**, 1H) ; 3,38(s, OC**H**_{**3**}, 3H) ; 1,59-1,88(m, CH-C**H**_{**2**}-CH₂, 2H) ; 1,18-1,40(m, CH₂-C**H**_{**2**}-CH₃, 2H) ; 0,85-0,92(m, CH₂-C**H**_{**3**}, 3H).
      [ α ]¹⁹_{D} = + 30,5° (c = 0,77 CH₂Cl₂) ; ee = 100%.
   - **N-[(S)-2-cyclopropyl-1-(4-méthylphényl)éthyl]thiourée *Composé IV'.25***
      ¹H RMN : 7,10-7,20(m, **Ar**, 4H) ; 6,93(m, NH, 1H) ; 5,75(m, N**H**_{**2**}, 2H) ; 4,43(m, C**H**, 1H) ;2,30(s, C**H**_{**3**}, 3H) ; 1,62-1,73(m, C**H**_{**2**}, 2H) ; 0,40-0,59(m, CH et C**H**_{**2**}, cyclopropyle, 3H) ; 0,04-0,13(m, C**H**_{**2**}, cyclopropyle, 2H).
      [ α ]¹⁹_{D} = + 75,5° (c = 0,42 CH₂Cl₂) ; ee = 100%.
   - **N-[(S)-1-(3,4-méthylènedioxyphényl)butyl]thiourée *Composé IV'.26***
      F = 140°C [ α ]²⁰_{D} = + 40,3 (c = 1,18 CH₂Cl₂) ; ee = 100%.
b) Obtention par chromatographie de thiourées optiquement actives (ee > 99%) à partir de thiourées racémiques.
   - **N-[(S)-1-phénylpentyl]thiourée *Composé IV'.27***
      A partir de la N-(1-phénylpentyl)thiourée racémique, on obtient après séparation par chromatographie sur phase CHIRACEL OJ éluant isohexane/éthanol 95/5, l'énantiomère S dont la pureté énantiomérique est 99,8%.
      F = 147°C [ α ]¹⁹_{D} = + 46,0° (c = 1,00 CH₂Cl₂).

### PREPARATION DES AMINOTHIAZOLES NH SOUS FORME D'UN ENANTIOMERE

### Composé II'

### • [4-(2-chloro-4-méthoxy-5-méthylphényl)-5-méthylthiazol-2-yl]-[(1R)-1-(4-fluorophényl)-2-méthoxyéthyl]amine Composé II'.1

A 3,28 g (14,3 mmol) de thiourée (*Composé IV'1*) en solution dans 70 ml d'éthanol sont ajoutés 4,23 g (14,5 mmol) de 2-bromo-1-(2'-chloro-4'-méthoxy-5'-méthylphényl)propan-1-one (*Composé III.1*) et 4,2 ml (30 mmol) de triéthylamine. Le mélange réactionnel est agité à 90°C pendant 3 heures puis est concentré sous pression réduite. Le résidu est repris par 200 ml de dichlorométhane et 100 ml d'eau. La phase organique est lavée avec une solution saturée en chlorure de sodium, séchée sur sulfate de sodium anhydre et évaporée à sec. L'extrait brut est purifié par chromatographie sur colonne de gel de silice : éluant cyclohexane/acétate d'éthyle 4/1 (v/v). On obtient 5,27 g (12,5 mmol) du *Composé II'.1* ; R = 87 % ; MS (MH⁺) 421.
¹H RMN : 7,34-7,44(m, **Ar**, 2H) ; 7,0-7,09(m, **Ar**, 3H) ; 6,83(s, **Ar**, 1H) ; 5,87(d, **NH**, 1H) ; 4,57(m, C**H**, 1H) ; 3,81 (s, OC**H**_{**3**}, 3H) ; 3,46-3,62(m, OC**H**_{**2**}, 2H) ; 3,35(s, OC**H**_{**3**}, 3H) ; 2,14(s, C**H**_{**3**}, 3H) ; 2,04(s, C**H**_{**3**}, 3H).

De la même façon ont été obtenus les composés intermédiaires suivants :

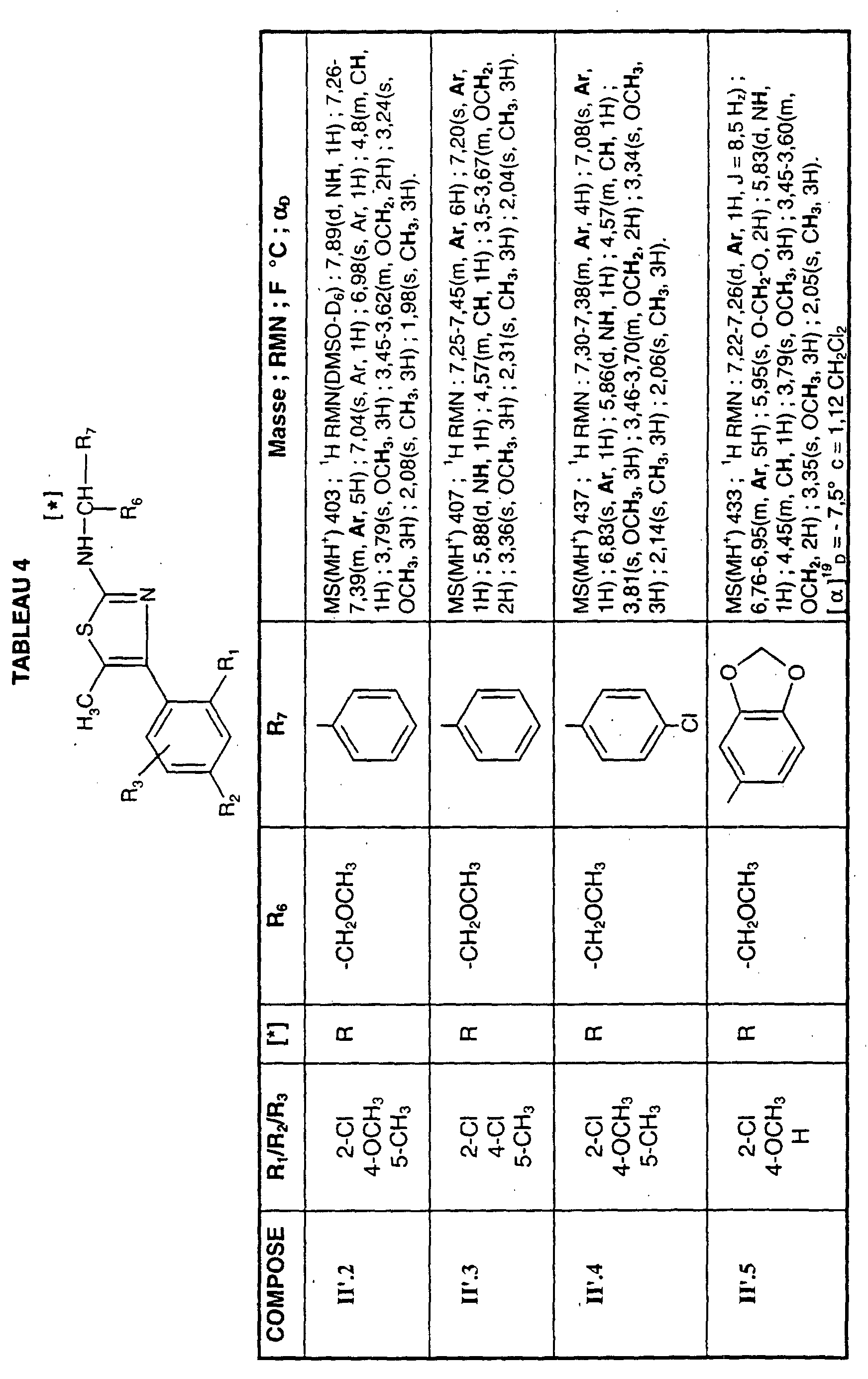

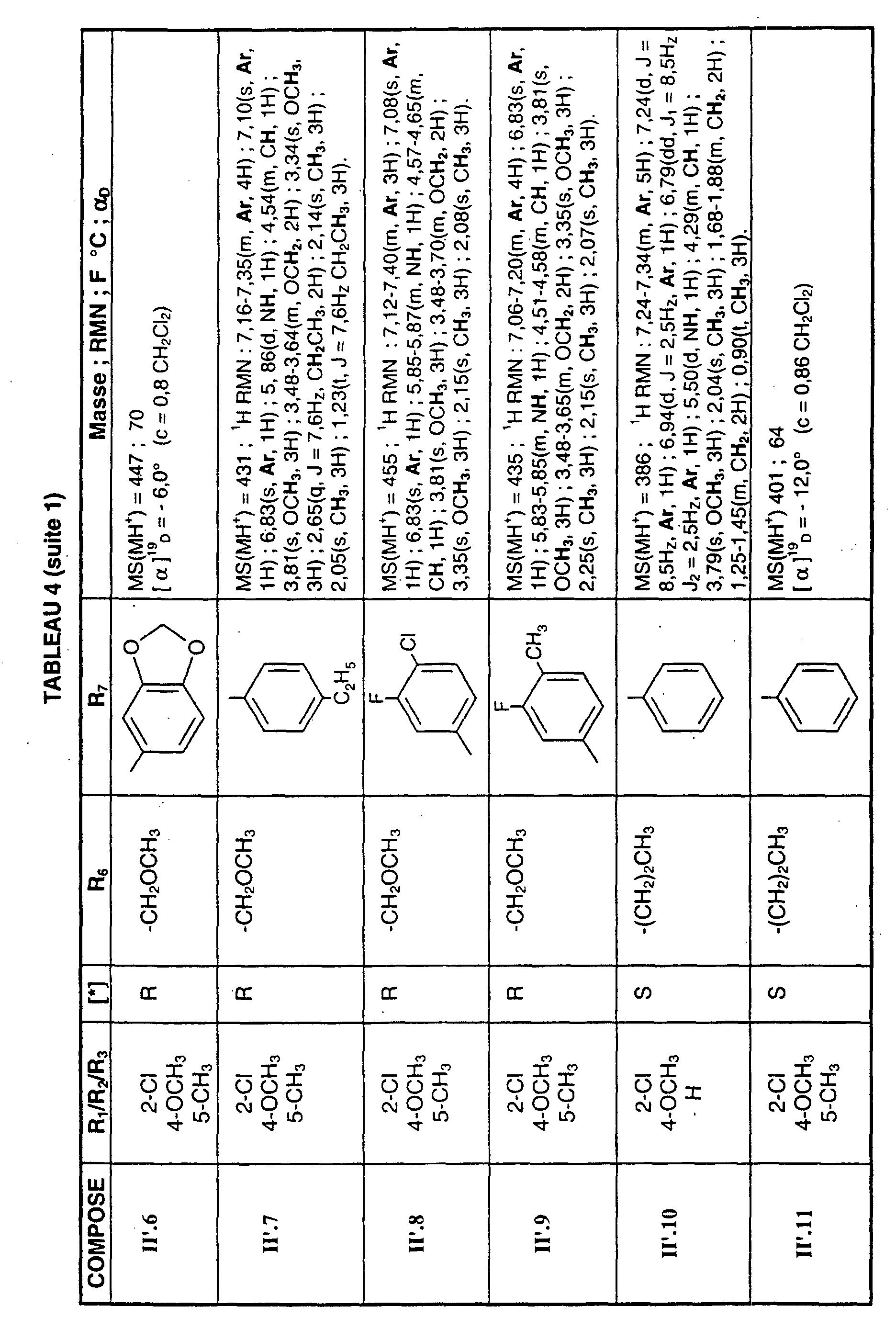

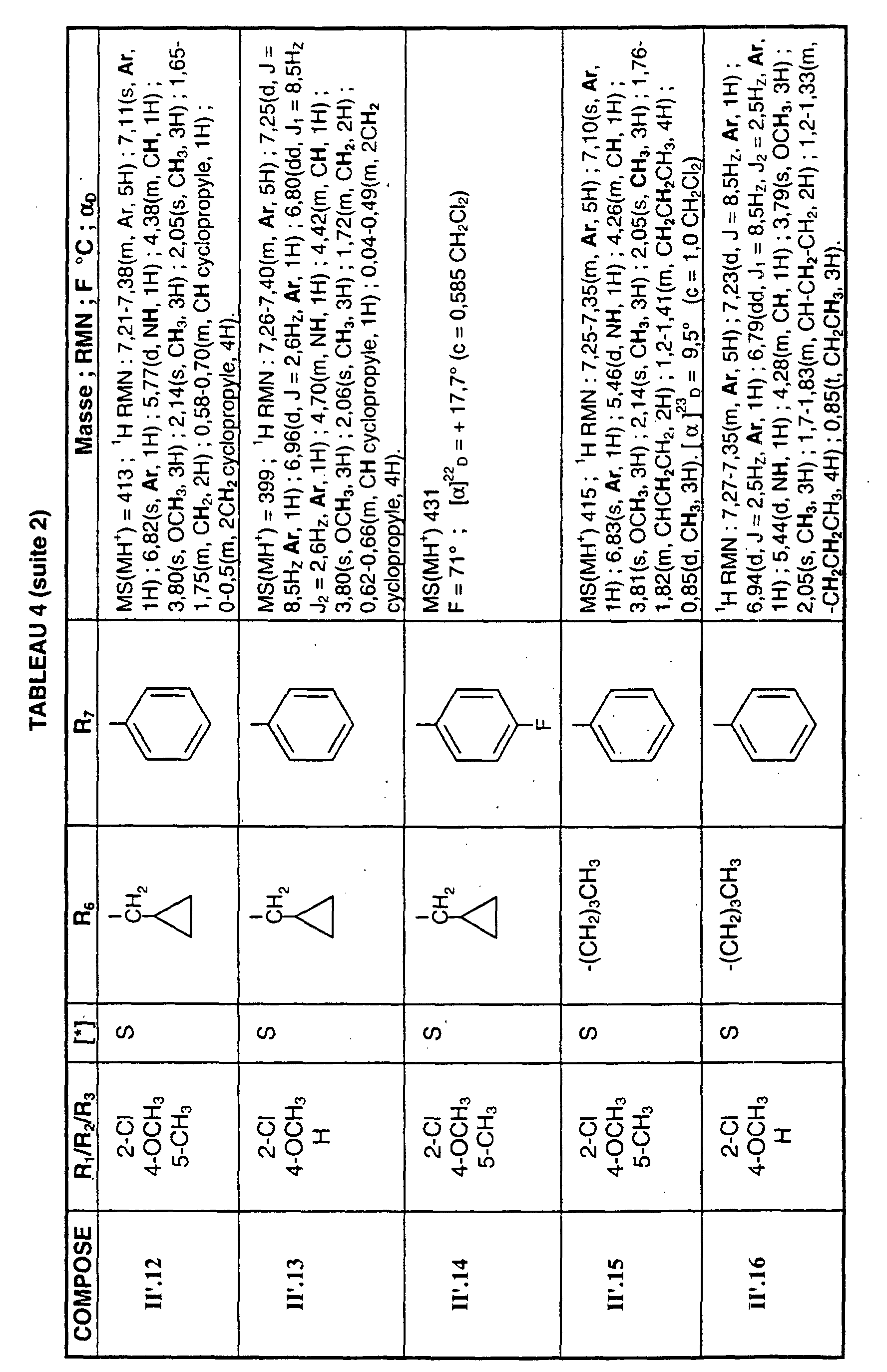

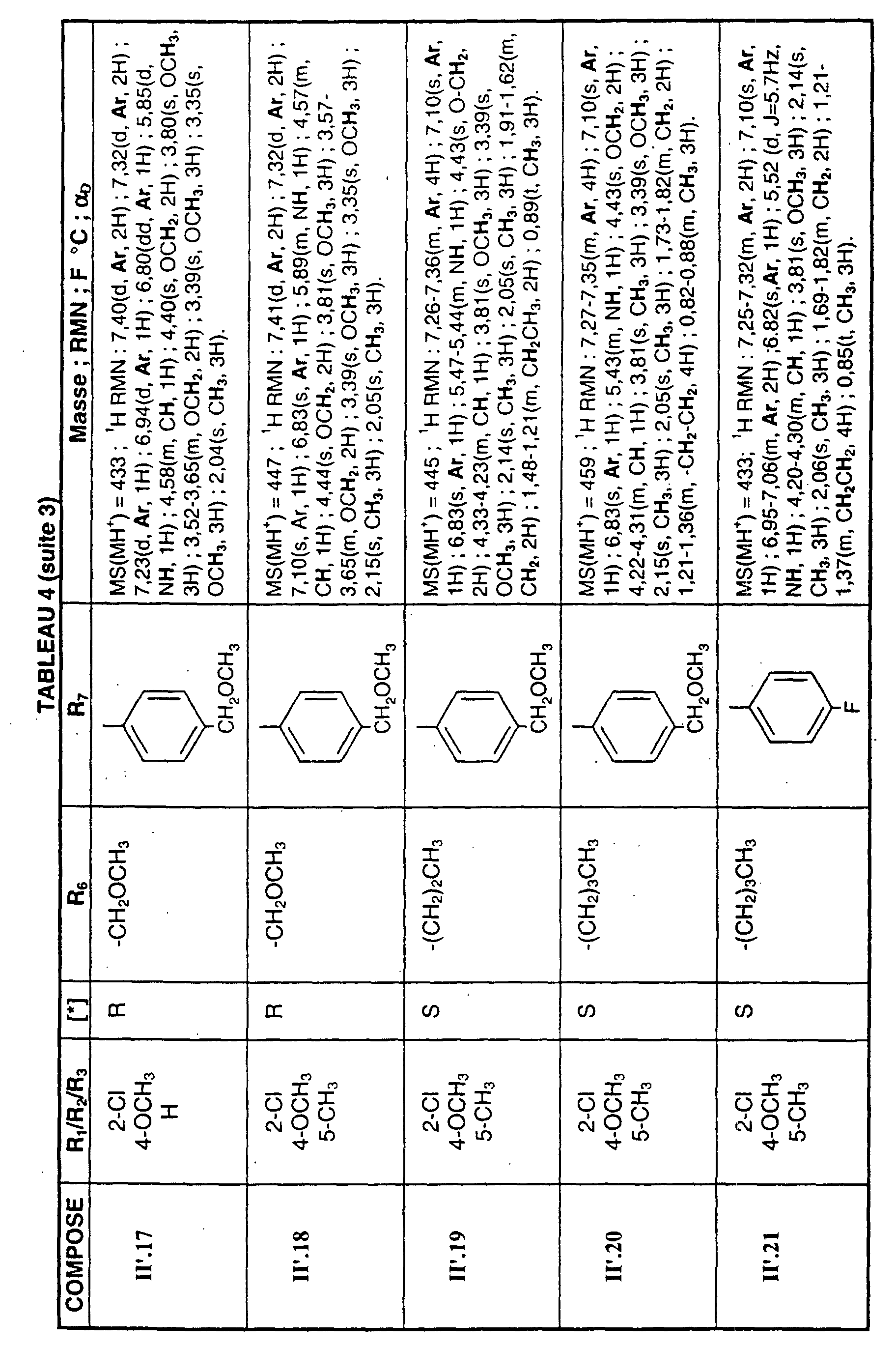

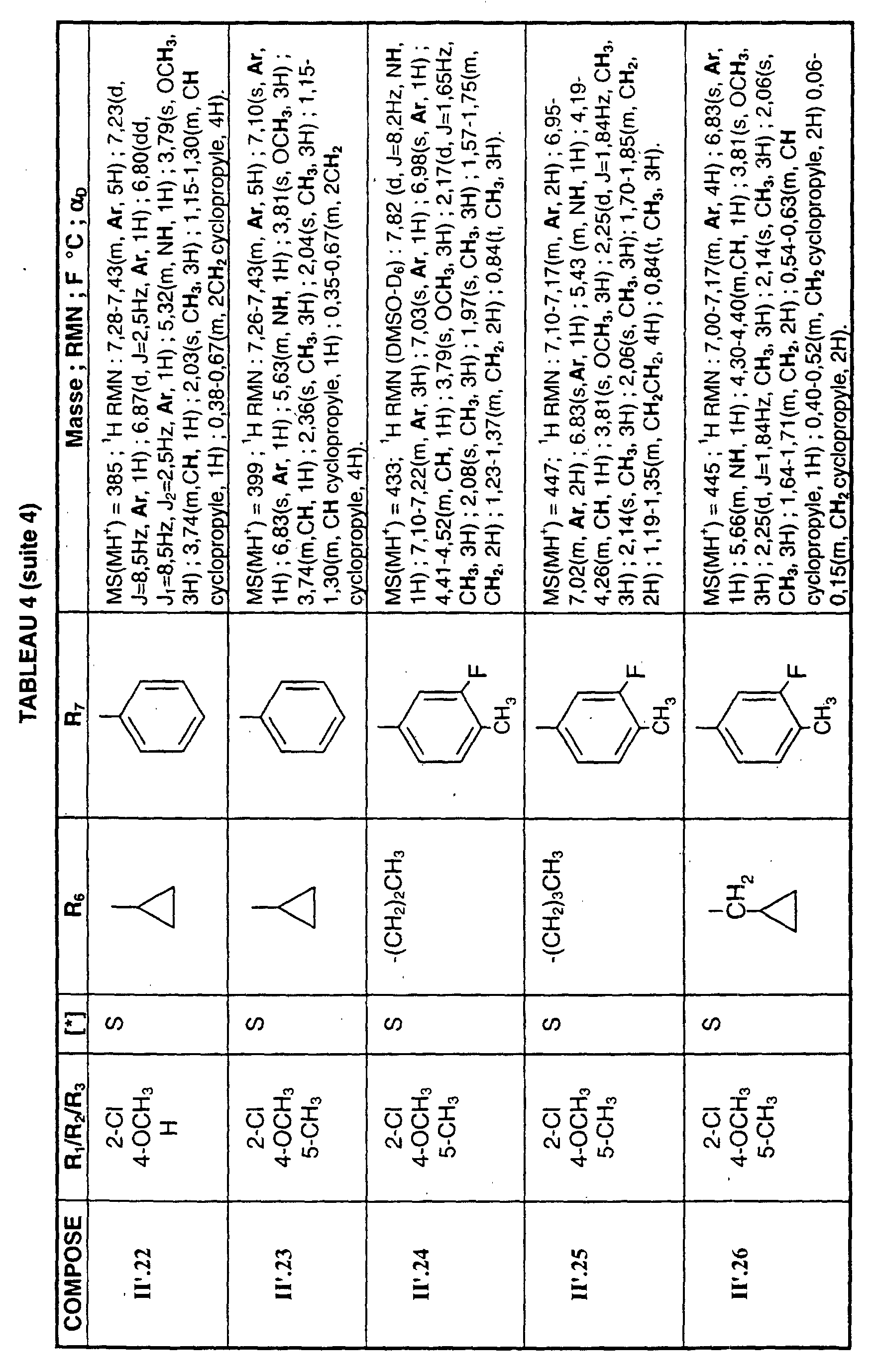

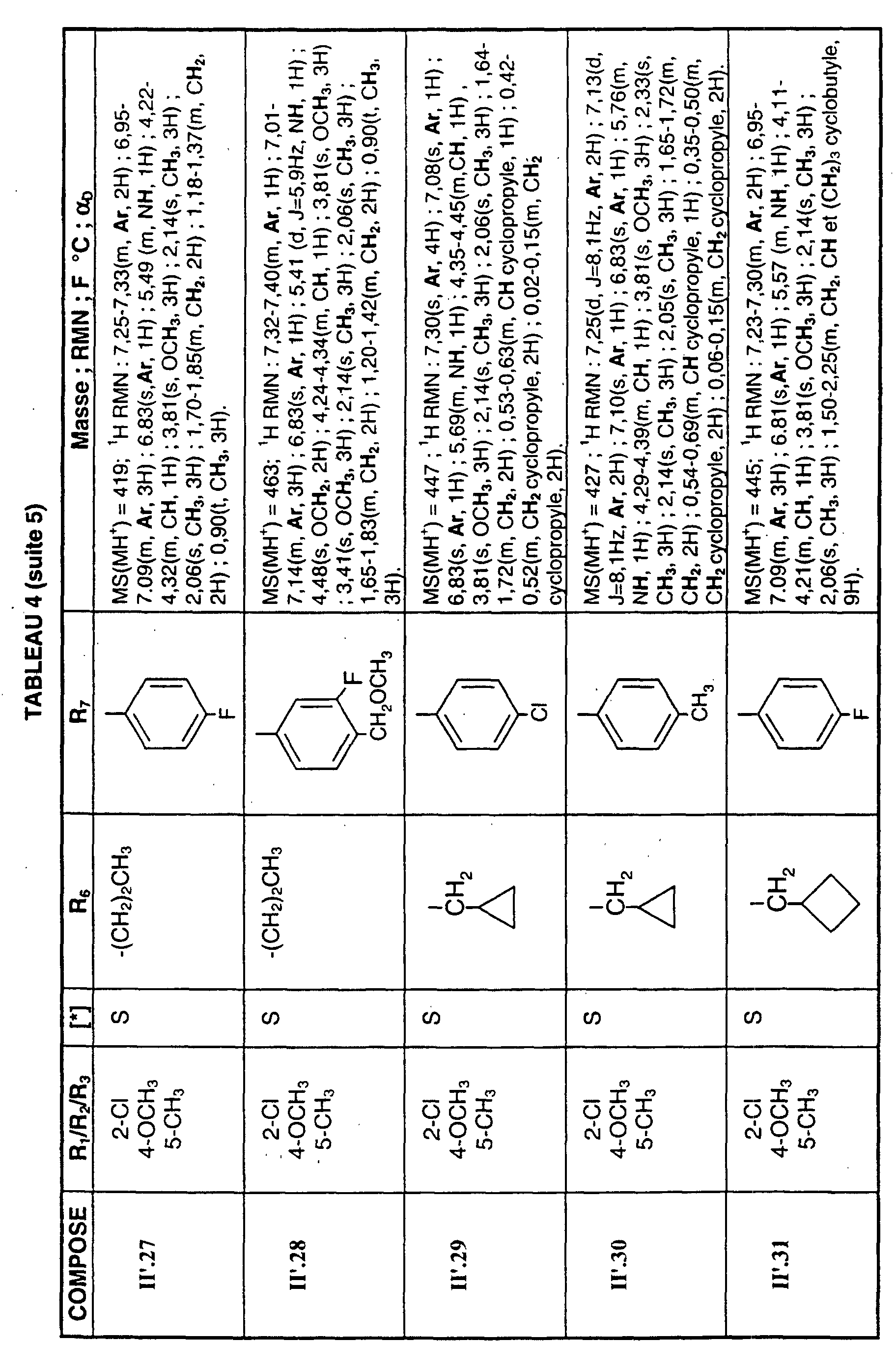

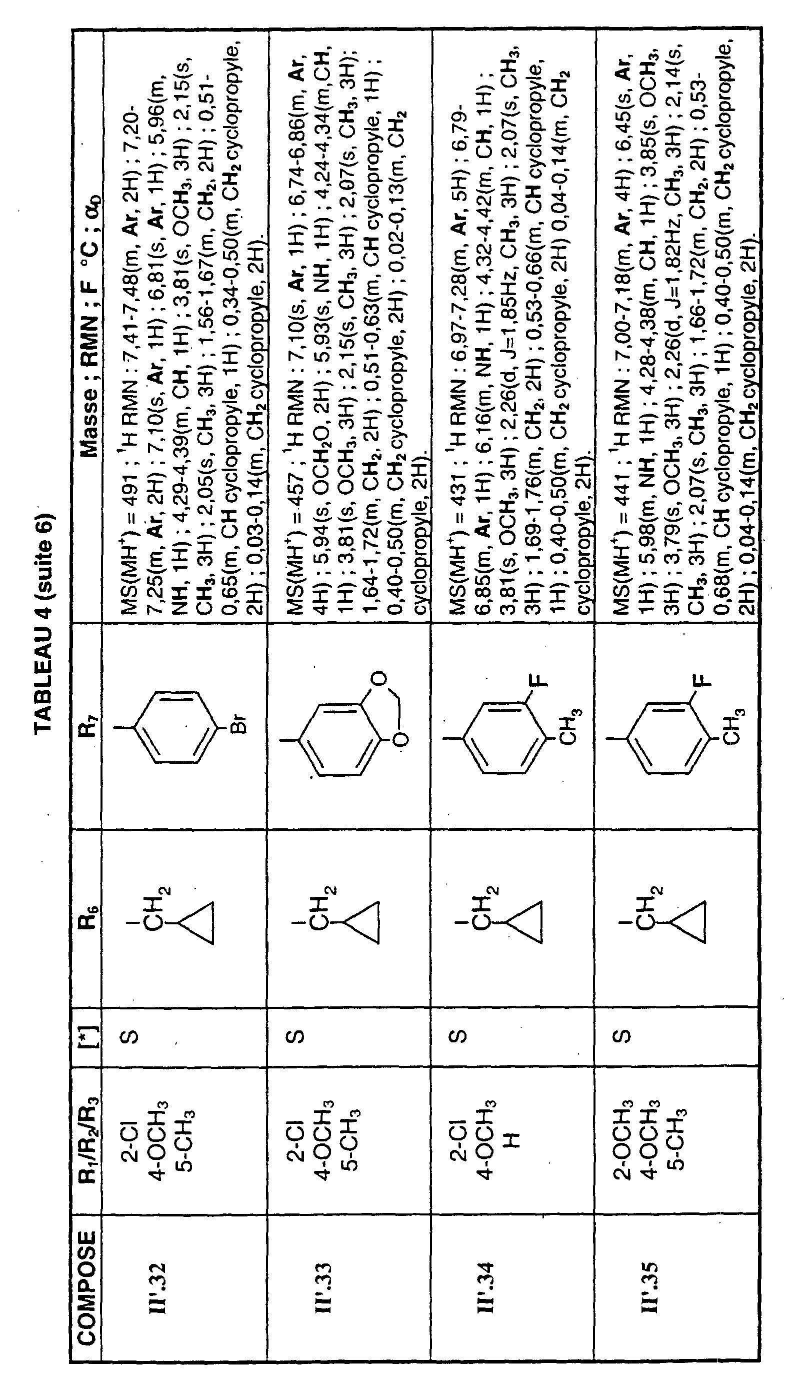

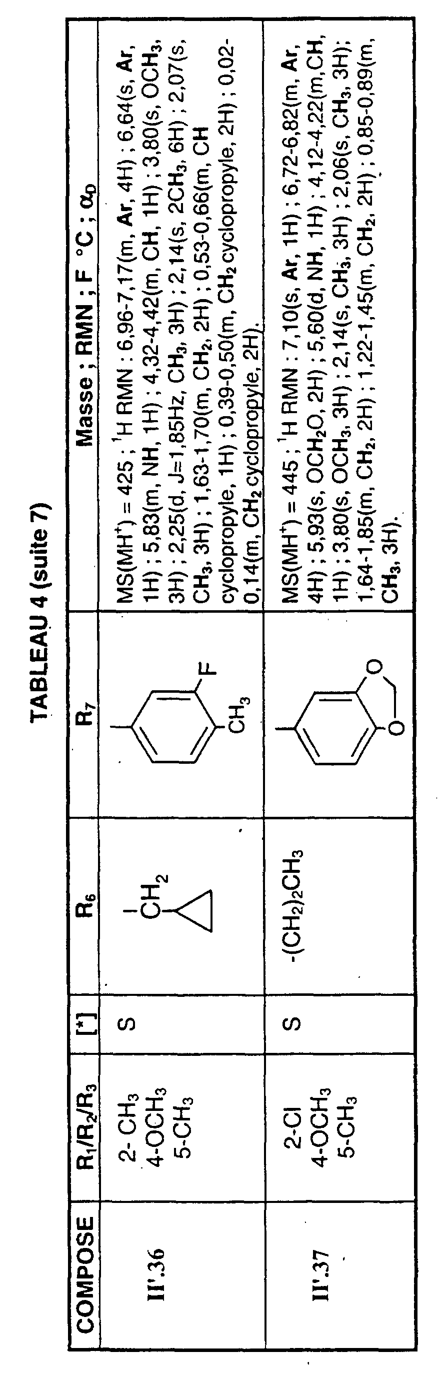

### PREPARATION DES AMINOTHIAZOLES N SUBSTITUÉS SOUS FORME D'UN ENANTIOMERE

### EXEMPLE 25

### [4-(2-chloro-4-méthoxy-5-méthylphényl)-5-méthylthiazol-2-yl]-[(1R)-1-(4-fluorophényl)-2-méthoxyéthyl]-prop-2-ynyl-amine

Une solution de 2,5 g (5,9 mmol) du *Composé II'.1* dans 30 ml de diméthylformamide est agitée à 0°C et additionnée de 260 mg (6,5 mmol) d'hydrure de sodium à 60 % dans l'huile. Le mélange réactionnel est agité pendant 20 minutes à 0°C puis additionné de 0,83 ml (7,5 mmol) d'une solution de bromure de propargyle à 80% dans le toluène. Le mélange réactionnel est agité pendant une heure à 10°C puis additionné à 0°C de 2 ml d'éthanol puis 50 ml d'eau. Le mélange est extrait par 200 ml d'acétate d'éthyle. La phase organique est lavée à l'eau puis avec une solution saturée en chlorure de sodium, séchée sur sulfate de sodium anhydre puis évaporée à sec. Le résidu brut est chromatographié sur colonne de gel de silice : éluant cyclohexane/acétate d'éthyle 9/1 (v/v). On obtient 2,14 g de produit pur gommeux ; R = 80 % ; MS (MH⁺) 459
¹H RMN : 7,37-7,46(m, **Ar**, 2H) ; 7,14(s, **Ar**, 1H) ; 6,95-7,07(m, **Ar**, 2H) ; 6,81(s, **Ar**, 1H) ; 5,54(m, C**H**, 1H) ; 4,15(dd, J₁ = 18H_{z}, J₂ = 2,4H_{z}, C**H**_{**2**}-N, 1H) ; 4,00-4,08(m, OC**H**_{**2**}, 2H) ; 3,98(dd, J₁ = 18H_{z}, J₂ = 2,4H_{z}, C**H**_{**2**}-N, 1 H) ; 3,82(s, OC**H**_{**3**}, 3H) ; 3,40(s, OC**H**_{**3**}, 3H) ; 2,18(t, J = 2,4H_{z}, C**H** propargyle, 1H) ; 2,17(s, C**H**_{**3**}, 3H) ; 2,16(s, C**H**_{**3**}, 3H).
[α]¹⁹_{D} = - 127° (c = 0,99 CH₂Cl₂)
HPLC chirale supercritique : ee = 99,4 %

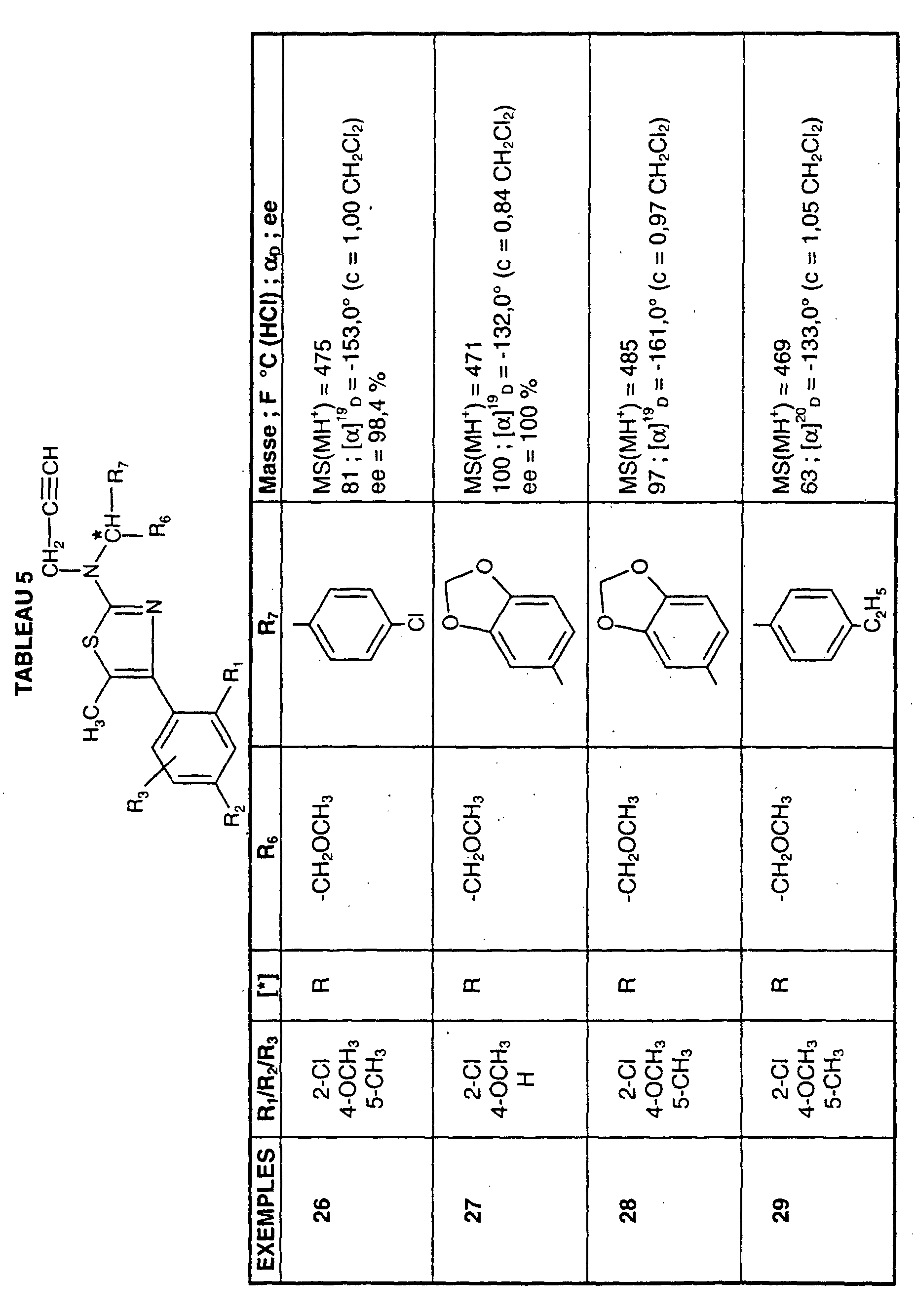

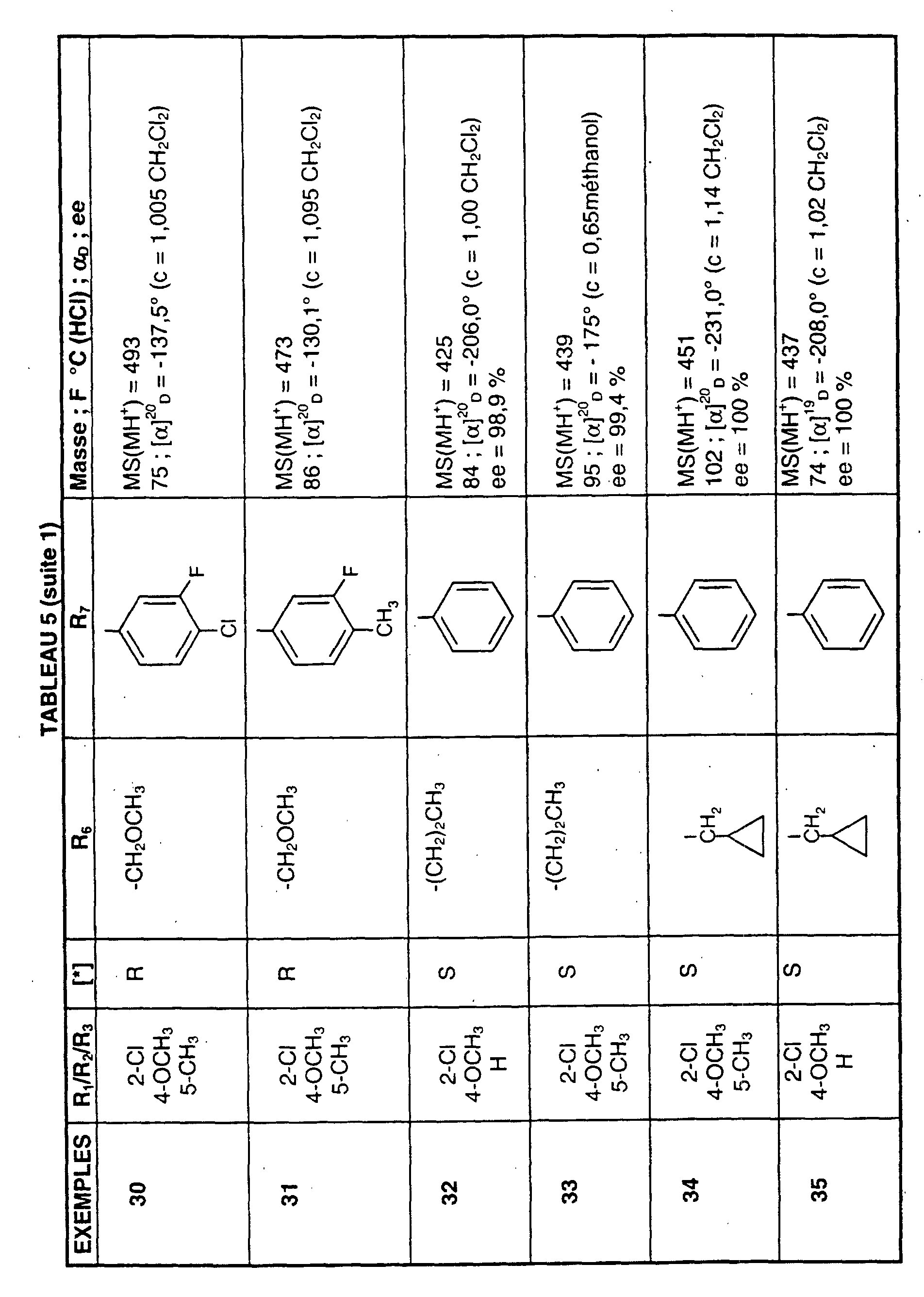

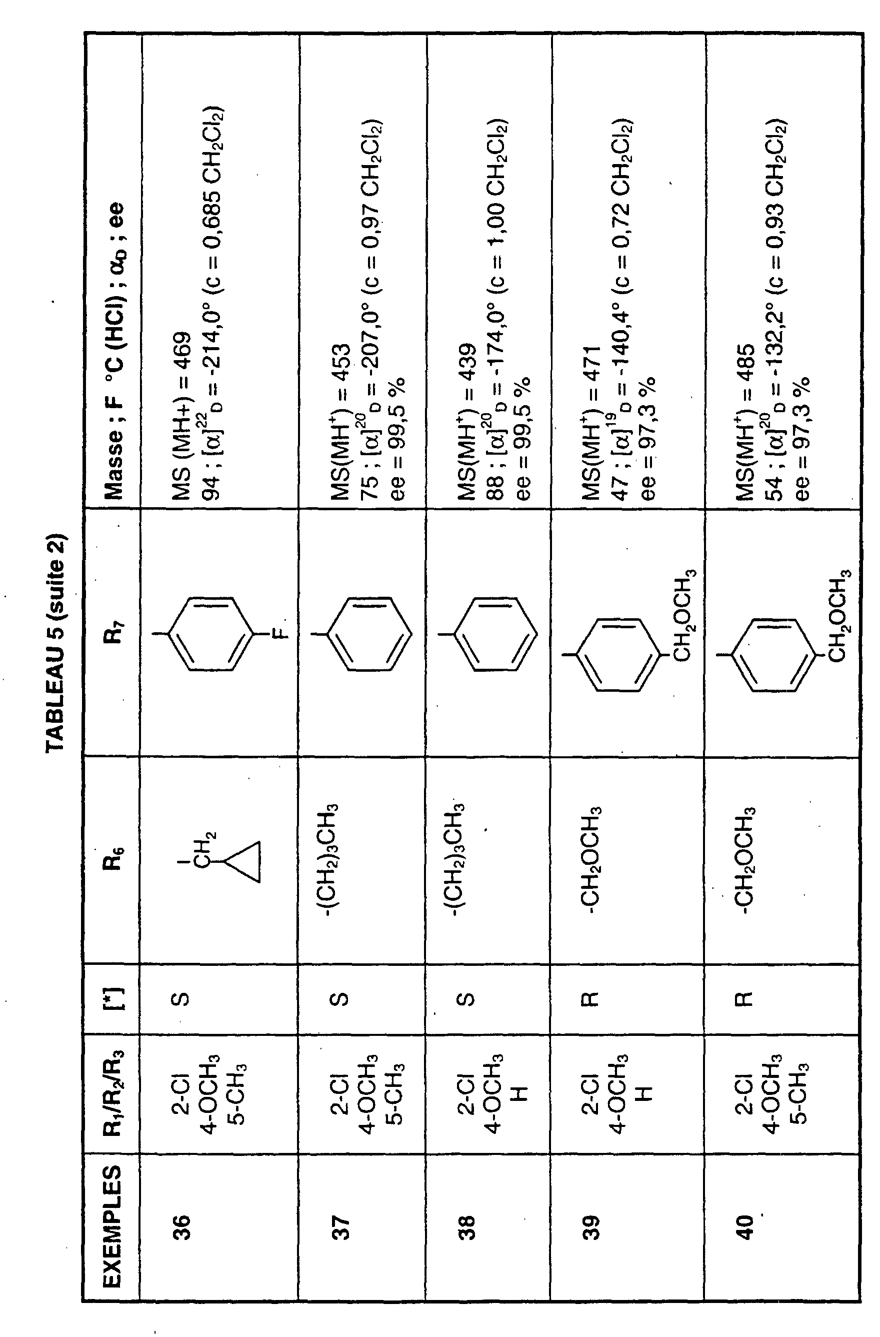

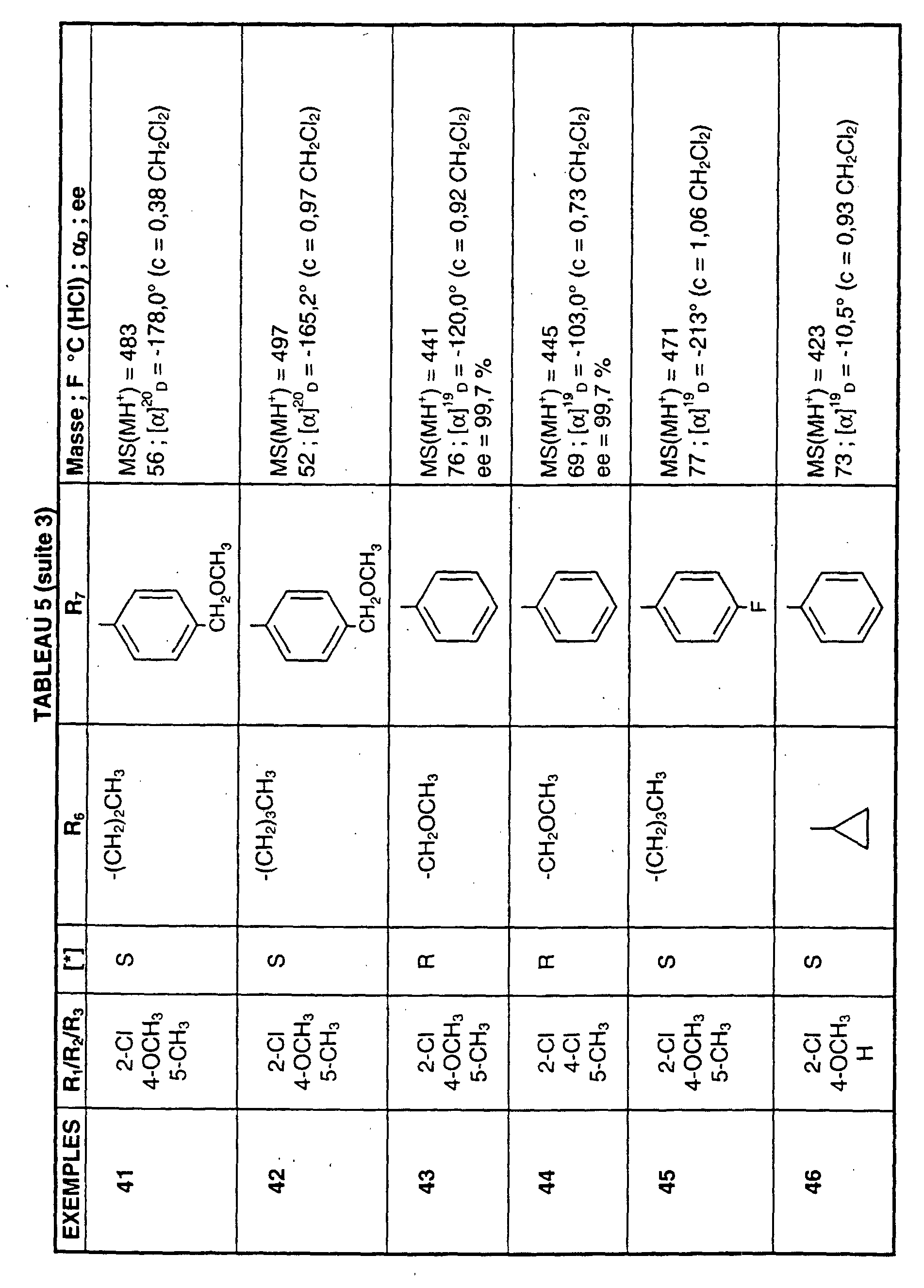

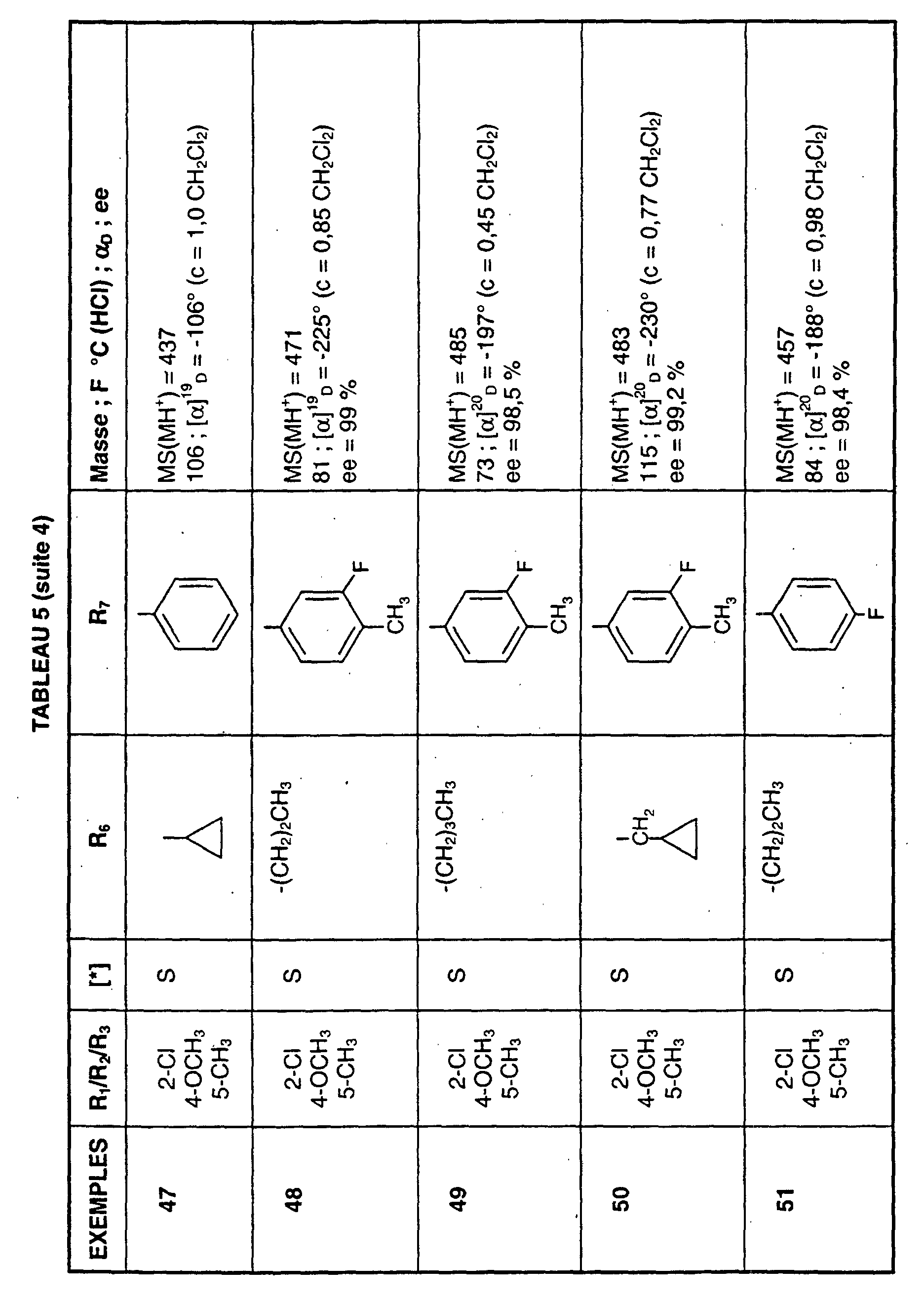

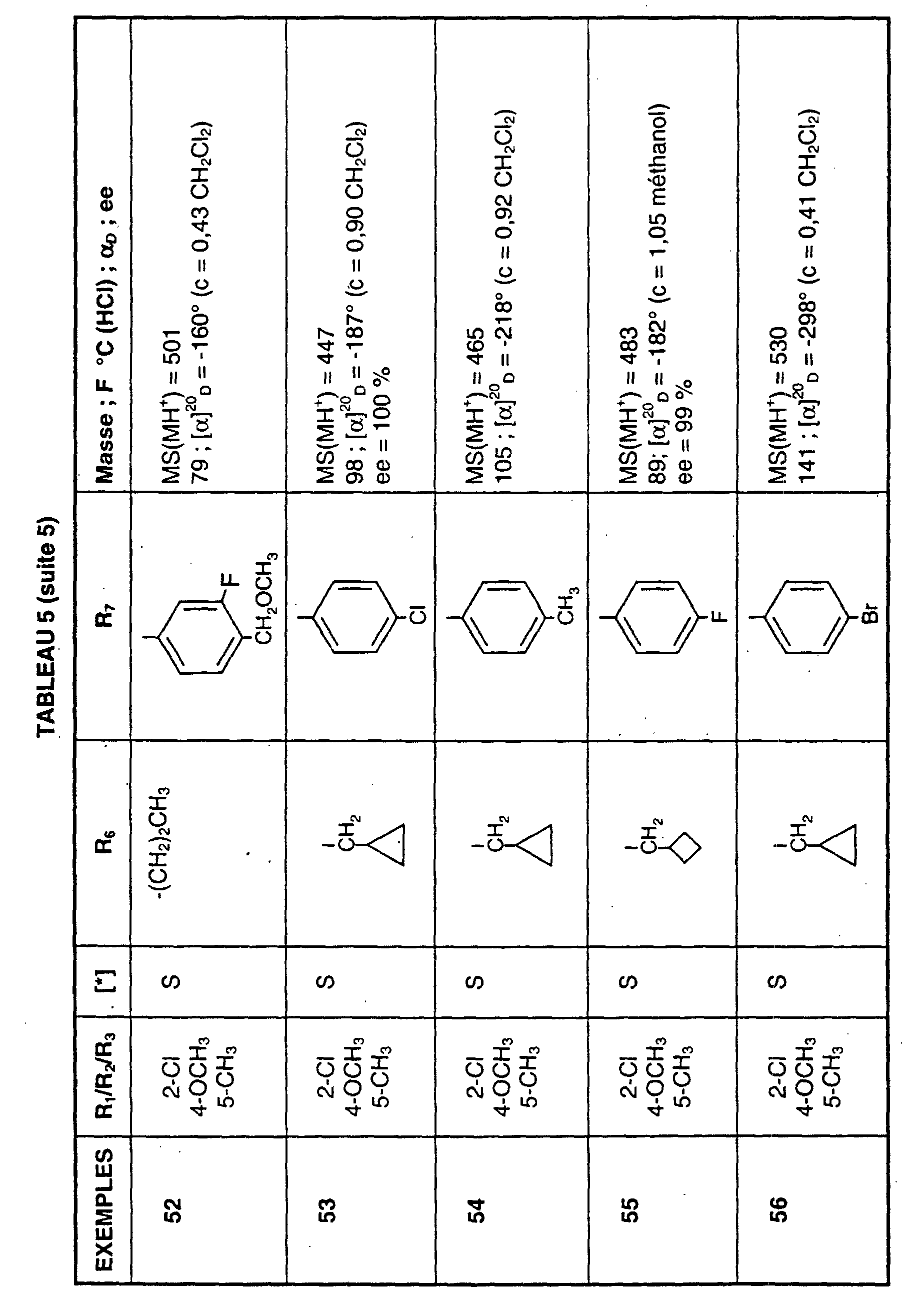

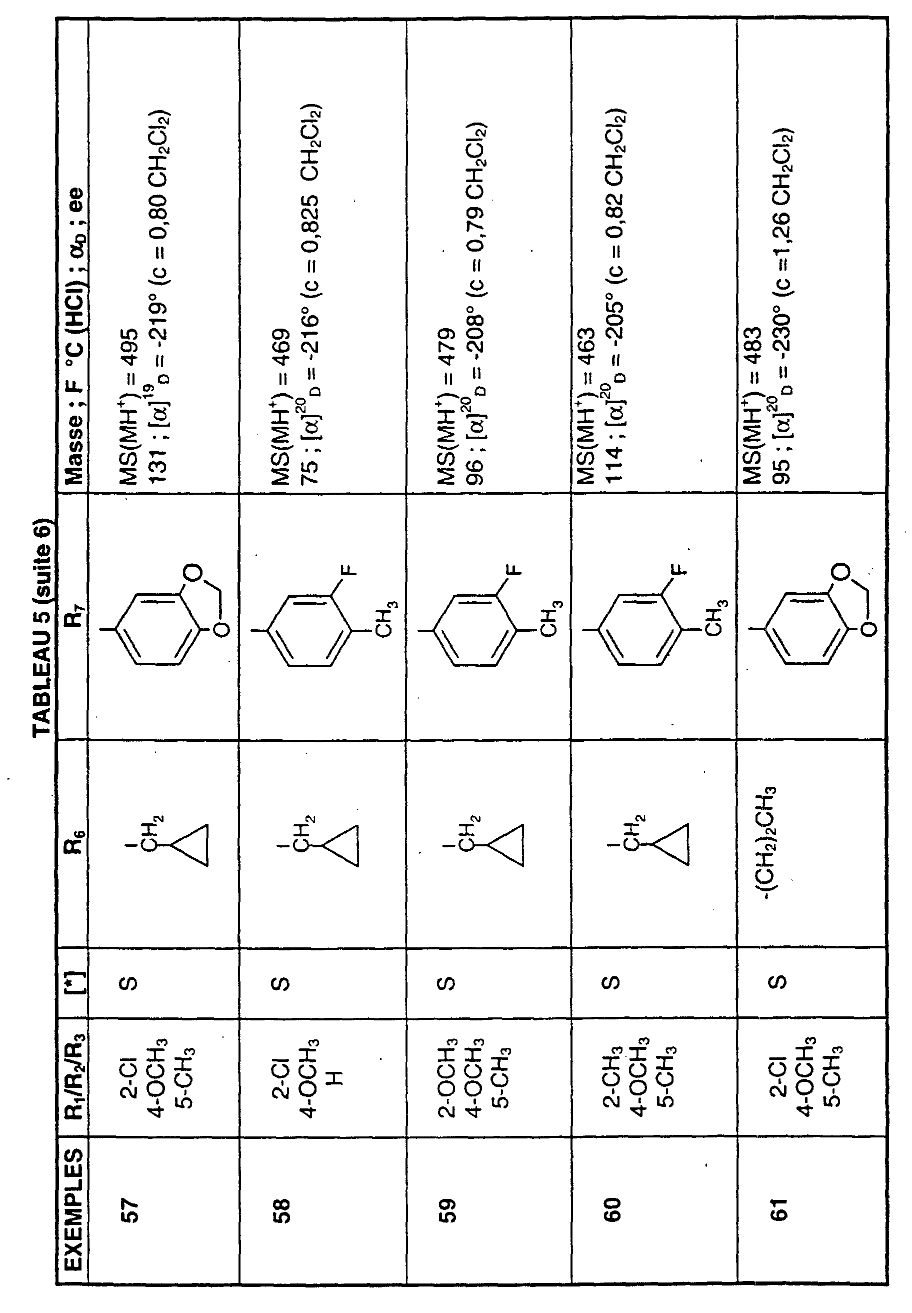

## Revendications

1. Composés, sous forme racémique ou d'énantiomère pur, de formule : 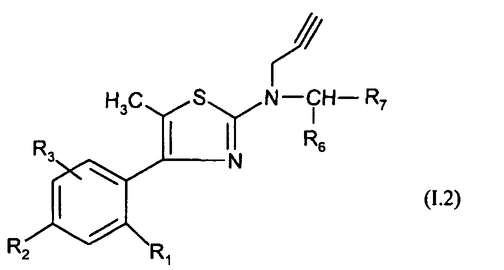 dans laquelle
- R₁ et R₂ identiques ou différents représentent chacun indépendamment un atome d'halogène ; un hydroxy(C₁-C₅)alkyle ; un (C₁-C₅)alkyle ; un aralkyle dans lequel la partie aryle est en (C₆-C₈) et la partie alkyle en (C₁-C₄) ; un (C₁-C₅)alcoxy ; un groupe trifluorométhyle ; un groupe nitro ; un groupe nitrile ; un groupe -SR dans lequel R représente l'hydrogène, un (C₁-C₅)alkyle ou un aralkyle dans lequel la partie aryle est en (C₆-C₈) et la partie alkyle en (C₁-C₄) ; un groupe -S-CO-R dans lequel R représente un radical (C₁-C₅)alkyle ou un aralkyle dans lequel la partie aryle est en (C₆-C₈) et la partie alkyle est en (C₁-C₄) ; un groupe -COORa dans lequel Ra représente l'hydrogène ou un (C₁-C₅)alkyle ; un groupe -CONRaRb avec Ra et Rb tels que définis ci-dessus pour Ra ; un groupe -NRaRb avec Ra et Rb tels que définis précédemment pour Ra ; un groupe -CONRcRd ou -NRcRd dans lesquels Rc et Rd constituent avec l'atome d'azote auquel ils sont liés un hétérocycle de 5 à 7 chaînons ; ou un groupe -NHCO-NRaRb avec Ra et Rb tels que définis ci-dessus pour Ra ;
- R₃ représente l'hydrogène ou est tel que défini ci-dessus pour R₁ et R₂ ;
- ou bien R₂ constitue avec R₃ lorsque celui-ci substitue le phényle en position 5 un groupe -X-CH₂-X- dans lequel X représente indépendamment un CH₂, un atome d'oxygène ou de soufre ;
- R₆ représente un (C₁-C₆)alkyle ; un (C₁-C₆)alcoxy(C₁-C₃)alkyle ; un (C₃-C₅)cycloalkyle ; un (C₃-C₆)cycloalkyl(C₁-C₆)alkyle ; un (C₁-C₆)alkylthio(C₁-C₃)alkyle ; un (C₁-C₆)alkylsulfoxy(C₁-C₃)alkyle ; un (C₁-C₆)alkylsulfodioxy(C₁-C₃)alkyle ;
- R₇ représente un phényle non substitué, mono, di ou trisubstitué en position 3, 4 ou 5 par un halogène, par un (C₁-C₅)alkyle, par un groupe -O-CH₂-O- sur deux atomes de carbone voisins du phényle, par un -CF₃, -NO₂, -CN, par un groupe -COOR₈,
- CONR₈R₉ ou par un groupe -CH₂OR₈ dans lesquels R₈ et R₉ représentent un (C₁-C₃)alkyle, OR₁₀ dans lequel R₁₀ représente un (C₁-C₅)alkyle ; ou bien R₇ représente un groupe pyridyle, thiophène, pyrazolyle, imidazolyle, (C₃-C₅)cycloalkyle ou (C₃-C₆)cycloalkyl(C₁-C₆)alkyle ;
leurs sels d'addition, leurs hydrates et/ou leurs solvats.

2. Composés selon la revendication 1 **caractérisés en ce que** :
- R₁ et R₂ identiques ou différents représentent chacun indépendamment un atome d'halogène ; un (C₁-C₅)alkyle ; un (C₁-C₅)alcoxy ;
- R₃ représente l'hydrogène ou est tel que défini ci-dessus pour R₁ et R₂ ;
- R₆ représente un (C₁-C₆)alkyle ; un (C₁-C₆)alcoxy(C₁-C₃)alkyle ; un (C₃-C₅)cycloalkyle ; un (C₃-C₆)cycloalkyl(C₁-C₆)alkyle ;
- R₇ représente un phényle non substitué, mono ou disubstitué en position 3 ou 4 par un halogène, un groupe (C₁-C₅)alkyle, un groupe -CH₂OR₈ dans lequel R₈ représente un (C₁-C₃)alkyle ou par un groupe -O-CH₂-O- en position 3,4 ; ou bien R₇ représente un groupe (C₃-C₅)cycloalkyle.

3. Composés selon l'une des revendications 1 ou 2, **caractérisés en ce que** R₃ est en position 5 du phényle.

4. Composés selon la revendication 1, choisis parmi :
- Chlorhydrate de la [4-(2-chloro-4-méthoxy-5-méthylphényl)-5-méthylthiazol-2-yl]-[(1R)-(1-(3-fluoro-4-méthylphényl)-2-méthoxyéthyl)]prop-2-ynylamine (EXEMPLE 31)
- Chlorhydrate de la [4-(2-chloro-4-méthoxy-5-méthylphényl)-5-méthylthiazol-2-yl]-[(1S)-(1-phénylbutyl)]prop-2-ynylamine (EXEMPLE 33)
- Chlorhydrate de la [4-(2-chloro-4-méthoxy-5-méthylphényl)-5-méthylthiazol-2-yl]-[(1S)-(2-cyclopropyl-1-phényléthyl)]prop-2-ynylamine (EXEMPLE 34)
- Chlorhydrate de la [4-(2-chloro-4-méthoxyphényl)-5-méthylthiazol-2-yl]-[(1S)-(2-cyclopropyl-1-phényléthyl)]prop-2-ynylamine (EXEMPLE 35)
- Chlorhydrate de la [4-(2-chloro-4-méthoxy-5-méthylphényl)-5-méthylthiazol-2-yl]-[(1S)-(2-cyclopropyl-1-(4-fluorophényl)éthyl)]prop-2-ynylamine (EXEMPLE 36)
- Chlorhydrate de la [4-(2-chloro-4-méthoxy-5-méthylphényl)-5-méthylthiazol-2-yl]-[(1S)-(1-phénylpentyl)]prop-2-ynylamine (EXEMPLE 37)
- Chlorhydrate de la [4-(2-chloro-4-méthoxy-5-méthylphényl)-5-méthylthiazol-2-yl]-[(1 R)-(2-méthoxy-1-(4-méthoxyméthylphényl)éthyl)]prop-2-ynylamine (EXEMPLE 40)
- Chlorhydrate de la [4-(2-chloro-4-méthoxy-5-méthylphényl)-5-méthylthiazol-2-yl]-[(1 S)-(1-(4-méthoxyméthylphényl)pentyl)]prop-2-ynylamine (EXEMPLE 42)
- Chlorhydrate de la [4-(2-chloro-4-méthoxy-5-méthylphényl)-5-méthylthiazol-2-yl]-[(1S)-(1-(4-fluorophényl)pentyl)]prop-2-ynylamine (EXEMPLE 45)
- Chlorhydrate de la [4-(2-chloro-4-méthoxy-5-méthylphényl)-5-méthylthiazol-2-yl]-[(1S)-(cyclopropyl-phényl-méthyl)]prop-2-ynylamine (EXEMPLE 47)
- Chlorhydrate de la [4-(2-chloro-4-méthoxy-5-méthylphényl)-5-méthylthiazol-2-yl]-[(1S)-(1-(3-fluoro-4-méthylphényl)pentyl)]prop-2-ynylamine (EXEMPLE 49)
- Chlorhydrate de la [4-(2-chloro-4-méthoxy-5-méthylphényl)-5-méthylthiazol-2-yl]-[(1S)-(2-cyclopropyl-1-(3-fluoro-4-méthylphényl)éthyl)]prop-2-ynylamine (EXEMPLE 50)
- Chlorhydrate de la [4-(2-chloro-4-méthoxy-5-méthylphényl)-5-méthylthiazol-2-yl]-[(1S)-(1-(4-fluorophényl)butyl)]prop-2-ynylamine (EXEMPLE 51)
- Chlorhydrate de la [4-(2-chloro-4-méthoxy-5-méthylphényl)-5-méthylthiazol-2-yl]-[(1S)-(1-(3-fluoro-4-méthoxyméthylphényl)butyl)]prop-2-ynylamine (EXEMPLE 52)
- Chlorhydrate de la [4-(2-chloro-4-méthoxy-5-méthylphényl)-5-méthylthiazol-2-yl]-[(1S)-(2-cyclopropyl-1-(4-chlorophényl)éthyl)]prop-2-ynylamine (EXEMPLE 53)
- Chlorhydrate de la [4-(2-chloro-4-méthoxy-5-méthylphényl)-5-méthylthiazol-2-yl]-[(1S)-(2-cyclopropyl-1-(4-méthylphényl)éthyl)]prop-2-ynylamine (EXEMPLE 54)
- Chlorhydrate de la [4-(2-chloro-4-méthoxy-5-méthylphényl)-5-méthylthiazol-2-yl]-[(1S)-(2-cyclobutyl-1-(4-fluorophényl)éthyl)]prop-2-ynylamine (EXEMPLE 55)
- Chlorhydrate de la [4-(2-chloro-4-méthoxy-5-méthylphényl)-5-méthylthiazol-2-yl]-[(1S)-(2-cyclopropyl-1-(4-bromophényl)éthyl)]prop-2-ynylamine (EXEMPLE 56)
- Chlorhydrate de la [4-(2-chloro-4-méthoxy-5-méthylphényl)-5-méthylthiazol-2-yl]-[(1S)-(2-cyclopropyl-1-(3,4-méthylènedioxyphényl)éthyl)]prop-2-ynylamine (EXEMPLE 57)
- Chlorhydrate de la [4-(2-chloro-4-méthoxyphényl)-5-méthylthiazol-2-yl]-[(1S)-(2-cyclopropyl-1-(3-fluoro-4-méthylphényl)éthyl)]prop-2-ynylamine (EXEMPLE 58)
- Chlorhydrate de la [4-(2,4-diméthoxy-5-méthylphényl)-5-méthylthiazol-2-yl]-[(1S)-(2-cyclopropyl-1-(3-fluoro-4-méthylphényl)éthyl)]prop-2-ynylamine (EXEMPLE 59)
- Chlorhydrate de la [4-(4-méthoxy-2,5-diméthylphényl)-5-méthylthiazol-2-yl]-[(1S)-(2-cyclopropyl-1-(3-fluoro-4-méthylphényl)éthyl)]prop-2-ynylamine (EXEMPLE 60)
- Chlorhydrate de la [4-(2-chloro-4-méthoxy-5-méthylphényl)-5-méthylthiazol-2-yl]-[(1 S)-(1-(3,4-méthylènedioxyphényl)butyl)]prop-2-ynylamine (EXEMPLE 61)
ainsi que les bases correspondantes, les autres sels d'addition, leurs solvats et/ou hydrates.

5. Procédé de préparation des composés de formule (I.2) selon la revendication 1 **caractérisé en ce que** ces composés sont obtenus par alkylation des composés de formule : 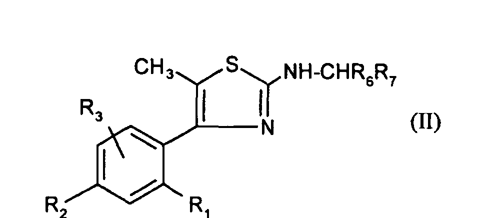 dans laquelle R₁, R₂, R₃, R₆ et R₇ sont tels que définis pour (I.2).

6. Composition pharmaceutique **caractérisée en ce qu'**elle contient en tant que principe actif un composé selon l'une quelconque des revendications 1 à 4.

7. Utilisation d'un composé selon l'une quelconque des revendications 1 à 4 pour la préparation de médicaments destinés à prévenir et/ou traiter les maladies CRF dépendantes.

8. Utilisation d'un composé selon l'une quelconque des revendications 1 à 4 pour la préparation d'un médicament destiné à traiter à titre préventif ou curatif, des maladies liées au stress et plus généralement dans le traitement de toutes les pathologies impliquant le CRF, choisies parmi la maladie de Cushing, les désordres neuropsychiatriques comme la dépression, l'anxiété, la panique, les désordres compulsifs obsessionnels, les troubles de l'humeur, le stress post-traumatique, les troubles du comportement, l'agressivité, l'anorexie, la boulimie, l'hyperglycémie, le travail prématuré, les grossesses à risques, la croissance retardée, les troubles du sommeil, l'épilepsie, et les dépressions de tout types ; la maladie d'Alzheimer, de Parkinson ; la chorée de Huntington ; la sclérose latérale amyotrophique ; les troubles vasculaires, cardiaques et cérébraux ; les troubles de l'activité sexuelle et de la fertilité; l'immunodépression, l'immunosuppression, les processus inflammatoires, les infections multiples, l'arthrite rhumatoïde, l'ostéo-arthrite, les uvéites, le psoriasis ainsi que le diabète ; les cancers ; les troubles fonctionnels gastro-intestinaux et les inflammations qui en découlent comme le colon irritable et inflammatoire, les diarrhées ; les troubles de perception de la douleur, les fibromyalgies liées ou non aux troubles du sommeil, la fatigue, la migraine ; les symptômes liés à la dépendance alcoolique et au sevrage de drogues.

## Patentansprüche

1. Verbindungen in racemischer oder enantiomerenreiner Form der Formel: 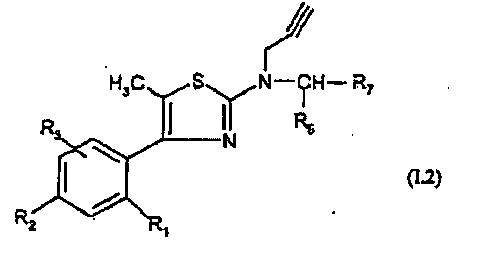 in der
- R₁ und R₂, die gleichartig oder verschieden sind, jeweils unabhängig voneinander ein Halogenatom; eine Hydroxy-(C₁-C₅)-alkylgruppe; eine (C₁-C₅)-Alkylgruppe; eine Aralkylgruppe, in der der Arylteil (C₆-C₈) und der Alkylteil (C₁-C₄) ist; eine (C₁-C₅)-Alkoxygruppe; eine Trifluormethylgruppe; eine Nitrogruppe; eine Nitrilgruppe; eine Gruppe -SR, in der R Wasserstoff, eine (C₁-C₅)-Alkylgruppe oder eine Aralkylgruppe, in der der Arylteil (C₆-C₈) und der Alkylteil (C₁-C₄) ist, bedeutet; eine Gruppe -S-CO-R, in der R eine (C₁-C₅)-Alkylgruppe oder eine Aralkylgruppe, in der der Arylteil (C₆-C₈) und der Alkylteil (C₁-C₄) ist, bedeutet; eine Gruppe -COORa, worin Ra Wasserstoff oder eine (C₁-C₅)-Alkylgruppe darstellt; eine Gruppe -CONRaRb, worin Ra und Rb die oben für Ra angegebenen Bedeutungen besitzen; eine Gruppe -NRaRb, worin Ra und Rb die oben für Ra angegebenen Bedeutungen besitzen; eine Gruppe -CONRcRd oder -NRcRd, worin Rc und Rd zusammen mit dem Stickstoffatom, an das sie gebunden sind, einen Heterocyclus mit 5 bis 7 Kettengliedern bilden; oder eine Gruppe -NHCO-NRaRb, worin Ra und Rb die oben für Ra angegebenen Bedeutungen besitzen; bedeuten;
- R₃ Wasserstoff bedeutet oder die oben für R₁ und R₂ angegebenen Bedeutungen besitzt;
- oder R₂ mit R₃, wenn dieser Rest den Phenylrest in der 5-Stellung substituiert, eine Gruppe -X-CH₂-X- bildet, worin X unabhängig voneinander eine CH₂-Gruppe, ein Sauerstoffatom oder ein Schwefelatom bedeutet;
- R₆ eine (C₁-C₆)-Alkylgruppe; eine (C₁-C₆)-Alkoxy-(C₁-C₃)-alkylgruppe; eine (C₃-C₅)-Cycloalkylgruppe; eine (C₃-C₆)-Cycloalkyl-(C₁-C₆)-alkylgruppe; eine (C₁-C₆)-Alkylthio-((C₁-C₃)-alkylgruppe; eine (C₁-C₆)-Alkylsulfoxy-(C₁-C₃)-alkylgruppe; oder eine (C₁-C₆)-Alkylsulfodioxy-(C₁-C₃)-alkylgruppe bedeutet;
- R₇ eine nichtsubstituierte Phenylgruppe bedeutet oder Phenylgruppe, die einfach, zweifach oder dreifach in den Stellungen 3, 4 oder 5 substituiert ist durch ein Halogen, eine (C₁-C₅)-Alkylgruppe, eine Gruppe -O-CH₂-O- an zwei benachbarten Kohlenstoffatomen des Phenylrests, durch eine Gruppe -CF₃,
- NO₂, -CN, durch eine Gruppe -COOR₈, -CONR₈R₉ oder durch eine Gruppe
- CH₂OR₈, worin R₈ und R₉ eine (C₁-C₃)-Alkylgruppe bedeuten, OR₁₀, worin R₁₀ eine (C₁-C₅)-Alkylgruppe darstellt; oder R₇ eine Pyridyl-, Thiophen-, Pyrazolyl-, Imidazolyl-, (C₃-C₅)-Cycloalkyl- oder (C₃-C₆)-Cycloalkyl-(C₁-C₆)-alkylgruppe bedeutet;
deren Additionssalze, deren Hydrate und/oder deren Solvate.

2. Verbindungen nach Anspruch 1, **dadurch gekennzeichnet, daß**:
- R₁ und R₂, die gleichartig oder verschieden sind, jeweils unabhängig voneinander ein Halogenatom; eine (C₁-C₅)-Alkylgruppe; oder eine (C₁-C₅)-Alkoxygruppe bedeuten;
- R₃ Wasserstoff bedeutet oder die oben für R₁ und R₂ angegebenen Bedeutungen besitzt;
- R₆ eine (C₁-C₆)-Alkylgruppe; eine (C₁-C₆)-Alkoxy-(C₁-C₃)-alkylgruppe; eine (C₃-C₅)-Cycloalkylgruppe; oder eine (C₃-C₆)-Cycloalkyl-(C₁-C₆)-alkylgruppe bedeutet;
- R₇ eine nichtsubstituierte Phenylgruppe oder eine Phenylgruppe, die monooder disubstituiert ist in der 3- oder 4-Stellung durch ein Halogen, eine (C₁-C₅)-Alkylgruppe, eine Gruppe -CH₂OR₈, in der R₈ eine (C₁-C₃)-Alkylgruppe bedeutet, oder durch eine Gruppe -O-CH₂-O- in der Stellung 3, 4; oder R₇ eine (C₃-C₅)-Cycloalkylgruppe bedeutet.

3. Verbindungen nach einem der Ansprüche 1 oder 2, **dadurch gekennzeichnet, daß** R₃ in der 5-Stellung des Phenylrests steht.

4. Verbindungen nach Anspruch 1, ausgewählt aus:
- [4-(2-Chlor-4-methoxy-5-methylphenyl)-5-methylthiazol-2-yl]-[(1R)-(1-(3-fluor-4-methylphenyl)-2-methoxyethyl)]-prop-2-inylamin-Hydrochlorid (BEISPIEL 31)
- [4-(2-Chlor-4-methoxy-5-methylphenyl)-5-methylthiazol-2-yl]-[(1S)-(1-phenylbutyl)-prop-2-inylamin-Hydrochlorid (BEISPIEL 33)
- [4-(2-Chlor-4-methoxy-5-methylphenyl)-5-methylthiazol-2-yl]-[(1S)-(2-cyclopropyl-1-phenylethyl)]-prop-2-inylamin-Hydrochlorid (BEISPIEL 34)
- [4-(2-Chlor-4-methoxy-5-methylphenyl)-5-methylthiazol-2-yl]-[(1S)-(2-cyclopropyl-1-phenylethyl)]-prop-2-inylamin-Hydrochlorid (BEISPIEL 35)
- [4-(2-Chlor-4-methoxy-5-methylphenyl)-5-methylthiazol-2-yl]-[(1S)-(2-cyclopropyl-1-(4-fluorphenyl)-ethyl)]-prop-2-inylamin-Hydrochlorid (BEISPIEL 36)
- [4-(2-Chlor-4-methoxy-5-methylphenyl)-5-methylthiazol-2-yl]-[(1S)-(1-phenylpentyl)]-prop-2-inylamin-Hydrochlorid (BEISPIEL 37)
- [4-(2-Chlor-4-methoxy-5-methylphenyl)-5-methylthiazol-2-yl]-[(1R)-(2-methoxy-1-(4-methoxymethylphenyl)-ethyl]-prop-2-inylamin-Hydrochlorid (BEISPIEL 40)
- [4-(2-Chlor-4-methoxy-5-methylphenyl)-5-methylthiazol-2-yl]-[(1S)-(1-(4-methoxymethylphenyl)-pentyl]-prop-2-inylamin-Hydrochlorid (BEISPIEL 42)
- [4-(2-Chlor-4-methoxy-5-methylphenyl)-5-methylthiazol-2-yl]-[(1S)-(1-(4-fluorphenyl)-pentyl]-prop-2-inylamin-Hydrochlorid (BEISPIEL 45)
- [4-(2-Chlor-4-methoxy-5-methylphenyl)-5-methylthiazol-2-yl]-[(1S)-(cyclopropylphenyl-methyl)]-prop-2-inylamin-Hydrochlorid (BEISPIEL 47)
- [4-(2-Chlor-4-methoxy-5-methylphenyl)-5-methylthiazol-2-yl]-[(1S)-(1-(3-fluor-4-methylphenyl)-pentyl]-prop-2-inylamin-Hydrochlorid (BEISPIEL 49)
- [4-(2-Chlor-4-methoxy-5-methylphenyl)-5-methylthiazol-2-yl]-[(1S)-(2-cyclopropyl-1-(3-fluor-4-methylphenyl)-ethyl]-prop-2-inylamin-Hydrochlorid (BEISPIEL 50)
- [4-(2-Chlor-4-methoxy-5-methylphenyl)-5-methylthiazol-2-yl]-[(1S)-(1-(4-fluorphenyl)-butyl]-prop-2-inylamin-Hydrochlorid (BEISPIEL 51)
- [4-(2-Chlor-4-methoxy-5-methylphenyl)-5-methylthiazol-2-yl]-[(1S)-(1-(3-fluor-4-methoxymethylphenyl)-butyl)]-prop-2-inylamin-Hydrochlorid (BEISPIEL 52)
- [4-(2-Chlor-4-methoxy-5-methylphenyl)-5-methylthiazol-2-yl]-[(1S)-(2-cyclopropyl-1-(4-chlorphenyl)-ethyl)]-prop-2-inylamin-Hydrochlorid (BEISPIEL 53)
- [4-(2-Chlor-4-methoxy-5-methylphenyl)-5-methylthiazol-2-yl]-[(1S)-(2-cyclopropyl-1-(4-methylphenyl)-ethyl)]-prop-2-inylamin-Hydrochlorid (BEISPIEL 54)
- [4-(2-Chlor-4-methoxy-5-methylphenyl)-5-methylthiazol-2-yl]-[(1S)-(2-cyclobutyl-1-(4-fluorphenyl)-ethyl)]-prop-2-inylamin-Hydrochlorid (BEISPIEL 55)
- [4-(2-Chlor-4-methoxy-5-methylphenyl)-5-methylthiazol-2-yl]-[(1S)-(2-cyclopropyl-1-(4-bromphenyl)-ethyl)]-prop-2-inylamin-Hydrochlorid (BEISPIEL 56)
- [4-[2-Chlor-4-methoxy-5-methylphenyl)-5-methylthiazol-2-yl]-[(1S)-(2-cyclopropyl-1-(3,4-methylendioxyphenyl)-ethyl)]-prop-2-inylamin-Hydrochlorid (BEISPIEL 57)
- [4-(2-Chlor-4-methoxyphenyl)-5-methylthiazol-2-yl]-[(1S)-(2-cyclopropyl-1-(3-fluor-4-methylphenyl)-ethyl)]-prop-2-inylamin-Hydrochlorid (BEISPIEL 58)
- [4-(2,4-Dimethoxy-5-methylphenyl)-5-methylthiazol-2-yl]-[(1S)-(2-cyclopropyl-1-(3-fluor-4-methylphenyl)-ethyl)]-prop-2-inylamin-Hydrochlorid (BEISPIEL 59)
- [4-(4-Methoxy-2,5-dimethylphenyl)-5-methylthiazol-2-yl]-[(1S)-(2-cyclopropyl-1-(3-fluor-4-methylphenyl)-ethyl)]-prop-2-inylamin-Hydrochlorid (BEISPIEL 60)
- [4-(2-Chlor-4-methoxy-5-methylphenyl)-5-methylthiazol-2-yl]-[(1S)-(1-(3,4-methylendioxyphenyl)-butyl)]-prop-2-inylamin-Hydrochlorid (BEISPIEL 61)
sowie die entsprechenden Basen, die weiteren Additionssalze, ihre Solvate und/ oder Hydrate.

5. Verfahren zur Herstellung der Verbindungen der Formel (1.2) nach Anspruch 1, **dadurch gekennzeichnet, daß** man sie durch Alkylierung von Verbindungen der Formel: 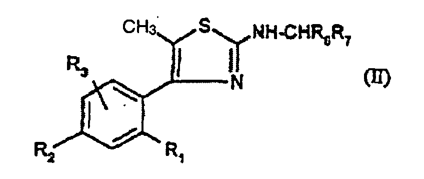 in der R₁, R₂, R₃, R₆ und R₇ die für (I.2) angegebenen Bedeutungen besitzen, erhält.

6. Pharmazeutische Zubereitung, **dadurch gekennzeichnet, daß** sie als Wirkstoff eine Verbindung nach einem der Ansprüche 1 bis 4 enthält.

7. Verwendung einer Verbindung nach einem der Ansprüche 1 bis 4 zur Herstellung von Arzneimitteln, die für die Vorbeugung und/oder Behandlung von CRF-abhängigen Erkrankungen bestimmt sind.

8. Verwendung einer Verbindung nach einem der Ansprüche 1 bis 4 für die Herstellung eines Arzneimittels zur vorbeugenden oder heilenden Behandlung von Erkrankungen, die mit dem Stress verbunden sind, und insbesondere für die Behandlung sämtlicher pathologischer Zustände, bei denen CRF beteiligt ist, ausgewählt aus der Cushing-Krankheit, neuropsychiatrischen Störungen, wie der Depression, der Angst, der Panik, krampfartigen Besessenheitsstörungen, Stimmungsstörungen, posttraumatischen Stress, Verhaltensstörungen, Aggressivität, Anorexie, Bulimie, Hyperglykämie, Frühgeburten, Risikoschwangerschaften, Wachstumsverzögerungen, Schlafstörungen, Epilepsie, Depressionen jeglicher Art; der Alzheimerschen Krankheit, der Parkinsonschen Krankheit; der Huntington Chorea; der amyotrophen Lateralsklerose; Gefäßstörungen, Herzstörungen und Gehirnstörungen; Störungen der sexuellen Aktivität und der Fertilität; der Immunodepression, der Immunosuppression, von Entzündungsprozessen, multipleten Infektionen, der rheumatoiden Arthritis, der Osteoarthritis, der Uveitis, Psoriasis sowie Diabetes; Krebs; funktionellen Magen-Darm-Störungen und die dadurch ausgelösten Entzündungszustände, wie Reizdarm und Darmentzündung, Diarrhoen; Störungen der Schmerzwahrnehmung; Fibromyalgien, die mit Schlafstörungen, Müdigkeit oder Migräne verknüpft sind oder auch nicht; und Symptome, die mit der Alkoholabhängigkeit und dem Drogenentzug verknüpft sind.

## Claims

1. Compounds, in racemic form or in the form of a pure enantiomer, of formula: 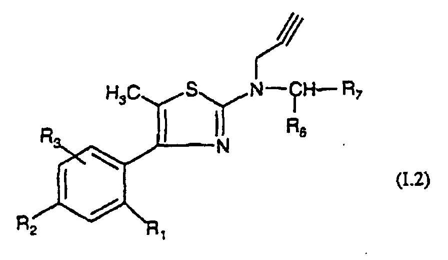 in which
- R₁ and R₂, which may be identical or different, each independently represent a halogen atom; a hydroxy (C₁-C₅)alkyl; a (C₁-C₅)alkyl; an aralkyl in which the aryl portion is (C₆-C₈) and the alkyl portion is (C₁-C₄); a (C₁-C₅)alkoxy; a trifluoromethyl group; a nitro group; a nitrile group; a group -SR in which R represents hydrogen, a (C₁-C₅)alkyl or an aralkyl in which the aryl portion is (C₆-C₈) and the alkyl portion is (C₁-C₄); a group -S-CO-R in which R represents a (C₁-C₅)alkyl or an aralkyl radical in which the aryl portion is (C₆-C₈) and the alkyl portion is (C₁-C₄); a group -COORa in which Ra represents hydrogen or a (C₁-C₅)alkyl; a group -CONRaRb with Ra and Rb as defined above for Ra; a group -NRaRb with Ra and Rb as defined above for Ra; a group -CONRcRd or -NRcRd in which Rc and Rd constitute, with the nitrogen atom to which they are attached, a 5- to 7-membered heterocycle; or a group -NHCO-NRaRb with Ra and Rb as defined above for Ra;
- R₃ represents hydrogen or is as defined above for R₁ and R₂;
- or alternatively R₂ constitutes with R₃, when the latter substitutes the phenyl in position 5, a group -X-CH₂-X- in which X independently represents a CH₂ or an oxygen or sulphur atom;
- R₆ represents a (C₁-C₆)alkyl; a (C₁-C₆)alkoxy(C₁-C₃)alkyl; a (C₃-C₅)cycloalkyl; a (C₃-C₆)cycloalkyl(C₁-C₆)alkyl; a (C₁-C₆)alkylthio(C₁-C₃)alkyl; a (C₁-C₆)alkylsulphoxy(C₁-C₃)alkyl; a (C₁-C₆)alkylsulphodioxy(C₁-C₃)alkyl;
- R₇ represents a phenyl which is unsubstituted, mono-, di- or trisubstituted in position 3, 4 or 5 with a halogen, with a (C₁-C₅)alkyl, with an -O-CH₂-O- group on two neighbouring carbon atoms of the phenyl, with a -CF₃, -NO₂ or -CN, with a group -COOR₈ or -CONR₈R₉ or with a group -CH₂OR₈ in which R₈ and R₉ represent a (C₁-C₃)alkyl, OR₁₀ in which R₁₀ represents a (C₁-C₅)alkyl; or alternatively R₇ represents a pyridyl, thiophene, pyrazolyl, imidazolyl, (C₃-C₅)cycloalkyl or (C₃-C₆)cycloalkyl(C₁-C₆)alkyl group;
the addition salts thereof, the hydrates thereof and/or the solvates thereof.

2. Compounds according to Claim 1, **characterized in that**:
- R₁ and R₂, which may be identical or different, each independently represent a halogen atom; a (C₁-C₅)alkyl; a (C₁-C₅)alkoxy;
- R₃ represents hydrogen or is as defined above for R₁ and R₂;
- R₆ represents a (C₁-C₆)alkyl; a (C₁-C₆)alkoxy(C₁-C₃)alkyl; a (C₃-C₅)cycloalkyl; a (C₃-C₆)cycloalkyl (C₁-C₆)alkyl;
- R₇ represents a phenyl which is unsubstituted or mono- or disubstituted in position 3 or 4 with a halogen, a (C₁-C₅)alkyl group, a group -CH₂OR₈ in which R₈ represents a (C₁-C₃)alkyl or with an -O-CH₂-O- group in position 3, 4; or alternatively R₇ represents a (C₃-C₅)cycloalkyl group.

3. Compounds according to either of Claims 1 and 2, **characterized in that** R₃ is in position 5 of the phenyl.

4. Compounds according to Claim 1, chosen from:
- [4-(2-chloro-4-methoxy-5-methylphenyl)-5-methylthiazol-2-yl][(1R)-(1-(3-fluoro-4-methylphenyl)-2-methoxyethyl)]prop-2-ynylamine hydrochloride (Example 31)
- [4-(2-chloro-4-methoxy-5-methylphenyl)-5-methylthiazol-2-yl][(1S)-(1-phenylbutyl)]prop-2-ynylamine hydrochloride (Example 33)
- [4-(2-chloro-4-methoxy-5-methylphenyl)-5-methylthiazol-2-yl][(1S)-(2-cyclopropyl-1-phenylethyl)]prop-2-ynylamine hydrochloride (Example 34)
- [4-(2-chloro-4-methoxyphenyl)-5-methylthiazol-2-yl][(1S)-(2-cyclopropyl-1-phenylethyl)]prop-2-ynylamine hydrochloride (Example 35)
- [4-(2-chloro-4-methoxy-5-methylphenyl)-5-methylthiazol-2-yl][(1S)-(2-cyclopropyl-1-(4-fluorophenyl)ethyl)]prop-2-ynylamine hydrochloride (Example 36)
- [4-(2-chloro-4-methoxy-5-methylphenyl)-5-methylthiazol-2-yl][(1S)-(1-phenylpentyl)]prop-2-ynylamine hydrochloride (Example 37)
- [4-(2-chloro-4-methoxy-5-methylphenyl)-5-methylthiazol-2-yl][(1R)-(2-methoxy-1-(4-methoxymethylphenyl)ethyl)]prop-2-ynylamine hydrochloride (Example 40)
- [4-(2-chloro-4-methoxy-5-methylphenyl)-5-methylthiazol-2-yl][(1S)-(1-(4-methoxymethylphenyl)pentyl)]prop-2-ynylamine hydrochloride (Example 42)
- [4-(2-chloro-4-methoxy-5-methylphenyl)-5-methylthiazol-2-yl][(1S)-(1-(4-fluorophenyl)pentyl)]prop-2-ynylamine hydrochloride (Example 45)
- [4-(2-chloro-4-methoxy-5-methylphenyl)-5-methylthiazol-2-yl][(1S)-(cyclopropylphenylmethyl)]prop-2-ynylamine hydrochloride (Example 47)
- [4-(2-chloro-4-methoxy-5-methylphenyl)-5-methylthiazol-2-yl][(1S)-(1-(3-fluoro-4-methylphenyl)pentyl)]prop-2-ynylamine hydrochloride (Example 49)
- [4-(2-chloro-4-methoxy-5-methylphenyl)-5-methylthiazol-2-yl][(1S)-(2-cyclopropyl-1-(3-fluoro-4-methylphenyl)ethyl)]prop-2-ynylamine hydrochloride (Example 50)
- [4-(2-chloro-4-methoxy-5-methylphenyl)-5-methylthiazol-2-yl][(1S)-(1-(4-fluorophenyl)butyl)]prop-2-ynylamine hydrochloride (Example 51)
- [4-(2-chloro-4-methoxy-5-methylphenyl)-5-methylthiazol-2-yl][(1S)-(1-(3-fluoro-4-methoxymethylphenyl)butyl)]prop-2-ynylamine hydrochloride (Example 52)
- [4-(2-chloro-4-methoxy-5-methylphenyl)-5-methylthiazol-2-yl][(1S)-(2-cyclopropyl-1-(4-chlorophenyl)ethyl)]prop-2-ynylamine hydrochloride (Example 53)
- [4-(2-chloro-4-methoxy-5-methylphenyl)-5-methylthiazol-2-yl][(1S)-(2-cyclopropyl-1-(4-methylphenyl)ethyl)]prop-2-ynylamine hydrochloride (Example 54)
- [4-(2-chloro-4-methoxy-5-methylphenyl)-5-methylthiazol-2-yl][(1S)-(2-cyclobutyl-1-(4-fluorophenyl)ethyl)]prop-2-ynylamine hydrochloride (Example 55)
- [4-(2-chloro-4-methoxy-5-methylphenyl)-5-methylthiazol-2-yl][(1S)-(2-cyclopropyl-1-(4-bromophenyl)ethyl)]prop-2-ynylamine hydrochloride (Example 56)
- [4-(2-chloro-4-methoxy-5-methylphenyl)-5-methylthiazol-2-yl][(1S)-(2-cyclopropyl-1-(3,4-methylenedioxyphenyl)ethyl)]prop-2-ynylamine hydrochloride (Example 57)
- [4-(2-chloro-4-methoxyphenyl)-5-methylthiazol-2-yl][(1S)-(2-cyclopropyl-1-(3-fluoro-4-methylphenyl)ethyl)]prop-2-ynylamine hydrochloride (Example 58)
- [4-(2,4-dimethoxy-5-methylphenyl)-5-methylthiazol-2-yl][(1S)-(2-cyclopropyl-1-(3-fluoro-4-methylphenyl)ethyl)]prop-2-ynylamine hydrochloride (Example 59)
- [4-(4-methoxy-2,5-dimethylphenyl)-5-methylthiazol-2-yl][(1S)-(2-cyclopropyl-1-(3-fluoro-4-methylphenyl)ethyl)]prop-2-ynylamine hydrochloride (Example 60)
- [4-(2-chloro-4-methoxy-5-methylphenyl)-5-methylthiazol-2-yl][(1S)-(1-(3,4-methylenedioxyphenyl)butyl)]prop-2-ynylamine hydrochloride (Example 61)
as well as the corresponding bases, the other addition salts and the solvates and/or hydrates thereof.

5. Process for preparing the compounds of formula (I.2) according to Claim 1, **characterized in that** these compounds are obtained by alkylation of the compounds of formula: 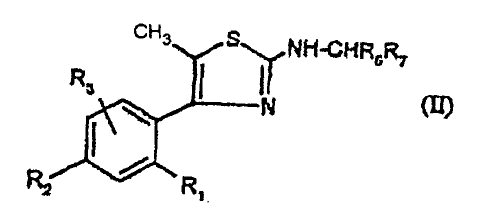 in which R₁, R₂, R₃, R₆ and R₇ are as defined for (I.2).

6. Pharmaceutical composition, **characterized in that** it contains a compound according to any one of Claims 1 to 4 as active principle.

7. Use of a compound according to any one of Claims 1 to 4 for the preparation of medicinal products intended for preventing and/or treating CRF-dependent conditions.

8. Use of a compound according to any one of Claims 1 to 4 for the preparation of a medicinal product intended for preventively or curatively treating stress-related conditions, and more generally in the treatment of any pathology involving CRF, chosen from Cushing's disease, neuropsychiatric disorders such as depression, anxiety, panic, obsessive compulsive disorders, mood disorders, post-traumatic stress, behavioural disorders, aggressiveness, anorexia, bulimia, hyperglycaemia, premature labour, at-risk pregnancy, retarded growth, sleeping disorders, epilepsy, and all types of depression; Alzheimer's disease, Parkinson's disease; Huntington's chorea; amyotrophic lateral sclerosis; vascular, cardiac and cerebral disorders; sexual activity disorders and fertility disorders; immunodepression, immunosuppression, inflammatory processes, multiple infections, rheumatoid arthritis, osteoarthritis, uveitis, psoriasis and diabetes; cancers; gastrointestinal functional disorders and inflammations arising therefrom, for instance irritable and inflammatory bowel, diarrhoea; pain-perception disorders, fibromyalgias which may or may not be associated with sleeping disorders, fatigue or migraine; symptoms associated with alcohol dependency and withdrawal from drugs.
